# EUROPEAN PATENT APPLICATION

(11) **EP 4 316 514 A1**
(43) Date of publication of application: **07.02.2024**
(21) Application number: 22382754.4
(22) Date of filing: 03.08.2022
(51) Int. Cl.: A61K 39/12, A61P 31/12, C07K 14/005

(54) **MVA-BASED VECTORS AND THEIR USE AS VACCINE AGAINST SARS-COV-2**

(71) Applicant: Consejo Superior de Investigaciones Científicas (CSIC), 28006 Madrid (ES)
(72) Inventor: BLASCO LOZANO, Rafael, 28040 Madrid (ES); LORENZO GILSANZ, María del Mar, 28040 Madrid (ES); SÁNCHEZ-PUIG EYRE, Juana María, 28040 Madrid (ES); MATÍA ESTEPAR, Alejandro, 28040 Madrid (ES); ORTEGO ALONSO, Francisco Javier, 28130 Madrid (ES); NOGALES GONZÁLEZ, Aitor, 28130 Madrid (ES); MORENO FERNÁNDEZ, Sandra, 28040 Madrid (ES)
(74) Representative: Hoffmann Eitle

(57) **Abstract**

The present invention is directed to recombinant modified vaccinia virus Ankara (MVA) vectors containing a Vaccinia virus codon optimized gene sequence encoding the Spike protein or fragment thereof of at least a SARS-CoV-2 virus. The present invention is further directed to a composition containing said recombinant MVAs as well as to a vaccine for eliciting T cell and humoral immune responses in a mammal against COVID19.

## Description

### TECHNICAL FIELD OF THE INVENTION

The present invention is directed to a modified vaccinia virus Ankara (MVA) vector comprising a nucleic acid sequence coding for an prefusion stabilized spike (S) protein, wherein the nucleic acid is codon optimized for vaccinia virus and/or is inserted in between the genes F12L and F13L. The present invention is also related to vaccine compositions and combinations as well as to a method for generating an immune response against at least a SARS-CoV-2 variant and a method for codon optimizing a nucleotide sequence.

### BACKGROUND OF THE INVENTION

The emergence of coronavirus SARS-CoV-2 (severe acute respiratory syndrome coronavirus 2) and its high impact on global health have made imperative the development of safe and effective vaccines for this virus, the causal agent of COVID-19 (coronavirus disease 2019). Covid-19 now adds to the list of coronavirus diseases that have threatened global health, along with the SARS (severe acute respiratory syndrome) and MERS (Middle East respiratory syndrome) coronaviruses that emerged in 2002/2003 and 2012, respectively.

Coronavirus virions are decorated with a spike (S) glycoprotein that binds to host cell receptors and mediates cell entry via fusion of the host and viral membranes. The receptor binding domain (RBD) comprised in the S protein is a key target for virus neutralizing antibodies, with an 'up' conformation, in which more neutralizing epitopes are exposed, and a 'down' conformation in which many epitopes are buried. As is the case in many viral fusion proteins, triggering of the fusion activity is preceded by a conformational change of the protein that exposes hydrophobic segments of the protein ("fusion peptide sequences") that mediate membrane fusion. Obviously, the functional S protein present in virus particles is in the prefusion conformation, but only amounts to a fraction of the total S protein. Prefusion stabilization is an strategy to present the S protein in its original conformation to the immune system and can be accompanied by an increase in recombinant expression of viral fusion glycoproteins. Producing an S antigen stabilized in the prefusion conformation represents also a superior immunogen to their wild-type counterparts, since the prefusion stabilized S protein is in the functional state that is present in the infective virus.

On the other hand, codon optimization techniques allow to increase the efficiency of translation of the antigenic proteins. Translation and protein synthesis rely on transfer RNAs (tRNAs). tRNAs bind to and deliver amino acids to the ribosome where these amino acids are incorporated into the growing polypeptide chain. Considering the three non-sense, or "stop" codons, there are 61 possible tRNAs out of the possible 64 triplet nucleotide combinations. Cells however, may not express all 61 of these tRNAs and those tRNAs that are expressed may be found at very different levels. Primarily due to this variation in tRNA expression, the codons present the sequences that encode a protein in a specific organism may affect the rate of translation and thus protein expression. Because of synonymous codons, different gene sequences can be used for expression of a protein without altering the amino acid sequence, and these different versions can have different expression efficiencies. Although these differences can derive from codon usage, also mRNA stability, and conforming to the AT/GC bias of the expressing organism can influence expression of the gene of interest. Most commonly, scientists codon optimize their nucleic acid sequences to the organisms where it will be expressed, but the testing is largely an empirical process. However, it is also known that codon optimization may disrupt the fine-tuned timing of translation and ultimately protein function. Different sequence optimization tools are available and choosing the right one for a given system may not be straight-forward.

In the present invention, we provide an effective MVA-based comprising a Vaccinia virus codon optimized nucleotide sequence that is able to produce a Spike (S) protein from at least a SARS-CoV-2 virus.

### BRIEF DESCRIPTION OF THE FIGURES

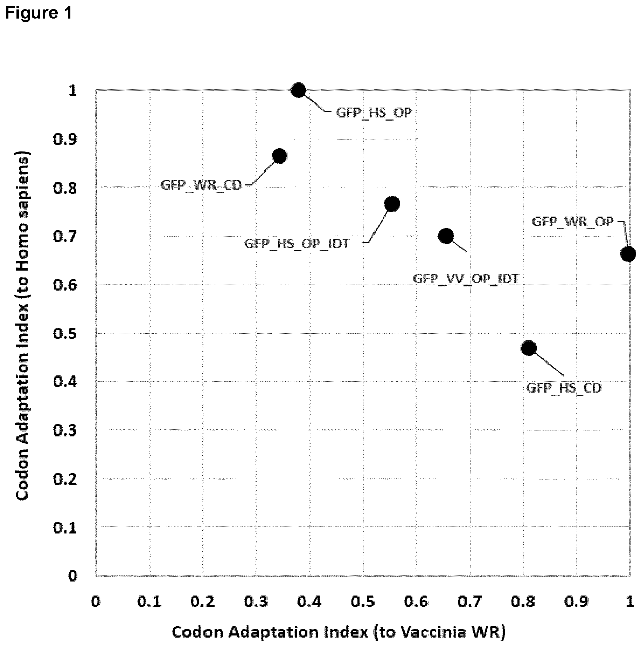

**Figure 1****. Codon adaptation index for different gene versions.** Coding gene sequences were optimized or deoptimized with respect to the Human codon usage table (termed HS) or to the Vaccinia WR codon usage table (termed WR). Codon optimized versions (OP) and codon-deoptimized versions (CD) were designed. In addition a codon-optimized version designed with an online tool by the IDT company web page (https://eu.idtdna.com/pages/tools/codon-optimization-tool) was designed for both the vaccinia and the human codon usage (termed GreenLantern_VV_OP_IDT and GreenLantern_HS_OP_IDT) respectively. The Codon adaptation index of each of the versions to each of the codon usage tables was calculated using an online program "CAI calculation from DNA or RNE sequences: http://genomes.urv.es/CAIcal/). The representation of the values obtained with each codon usage table for each gene version are shown.

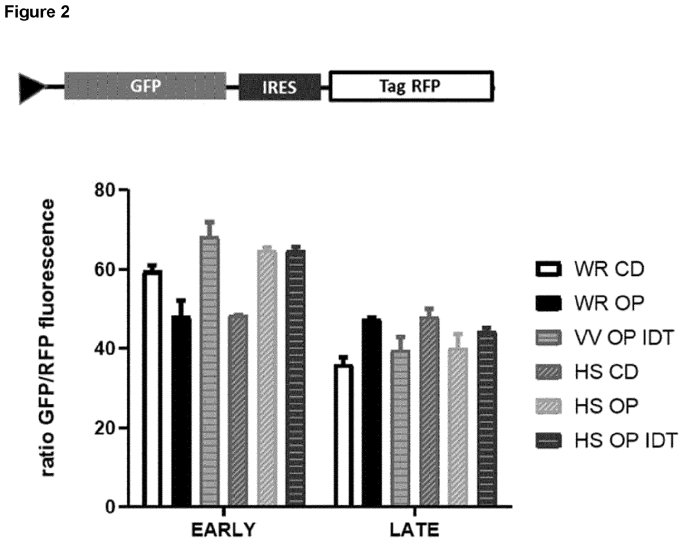

**Figure 2****. Effect of gene codon composition on VV-directed expression.** The upper image shows schematically the bicistronic construct which is placed under the control of a VV synthetic early/late promoter (black triangle). The GFP gene was located downstream of the promoter and the TagRFP gene was placed downstream of an IRES sequence. Different versions of GFP were inserted in the upstream position. GFP and RFP fluorescence were measured in cells infected with the corresponding viruses. In the lower panel, the ratio GFP/RFP fluorescence for each construct is shown. Early conditions correspond to moi (multiplicity of infection) 0.5 infections maintained for 24 hours in the presence of cytosine arabinoside to prevent DNA replication. Late conditions correspond to cells infected at an moi of 3 and maintained for 24 hours.

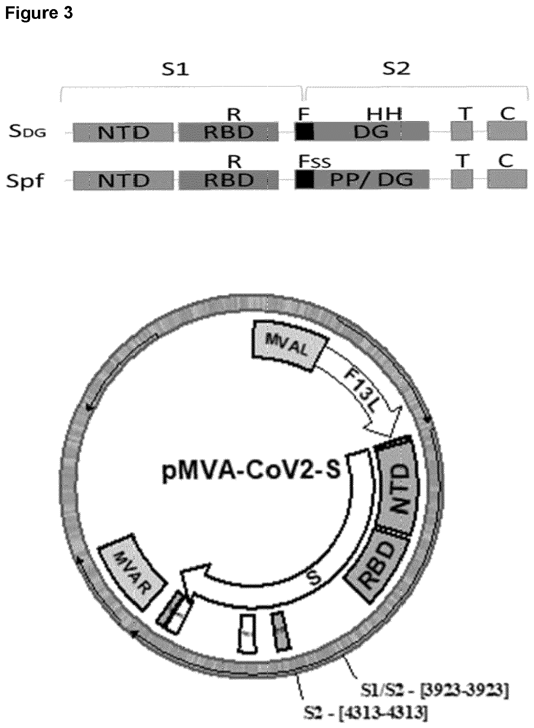

**Figure 3****. Schematic representation of the SARS-Cov-2 Spike glycoproteins tested.** The positions of the two portions of the mature S protein (S1 and S2) after proteolitic cleavage are indicated. Domains are the N-terminal domain (NTD), Receptor binding domain (RBD), the fusion peptide (F) heptad repeat domain (HH), Transmembrane domain (T) and cytoplasmic domain (C). The approximate position of the D614G mutation (DG) and the K986P V987P (PP) mutations are shown. On the left, the name of the different versions is shown. Bottom: Schematic representation of the transfer plasmid used to insert the gene into the VACV genome by homologous recombination. MVA-L and MVA-R correspond to the recombination flanks surrounding viral gene F13L. The position of the S gene and the F13L gene are also shown. E/L promoter is placed between the F13L gene and the S coding sequence. S1/S2 and S2 indicate the proteolytic cleavage positions.

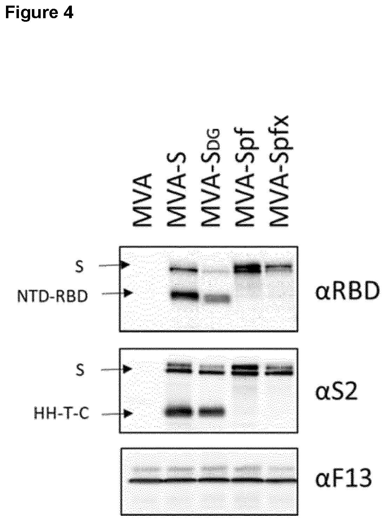

**Figure 4****. Immunoblot of S protein expressed from MVA recombinants.** Cells were infected with the indicated viruses for 24h, after which cell extracts were prepared and analyzed by Western Blot using antibodies to the RBD domain or to the S2 portion of the S protein. Antibody to the vaccinia F13 protein was used as a loading control.

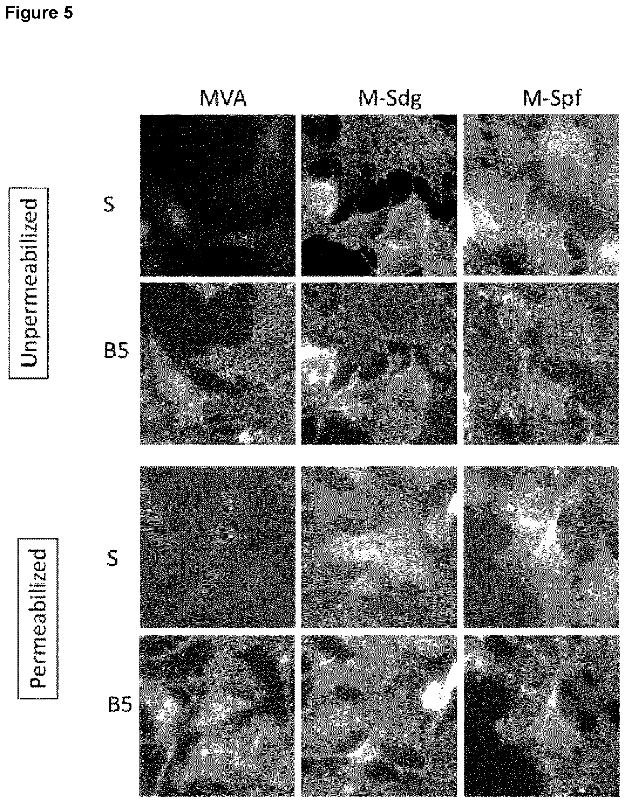

**Figure 5****. Subcellular location of S protein. BHK cells were infected for 18h and** incubated with specific antibodies under permeabilizing or non-permeabilizing conditions. Antibody to the RBD domain was used to label the extracellular domain of S (panels "S"). As a control we used antibody to the B5 protein of the vaccinia virus, which is a glycosylated transmembrane protein located both on the cell surface and in the Golgi complex.

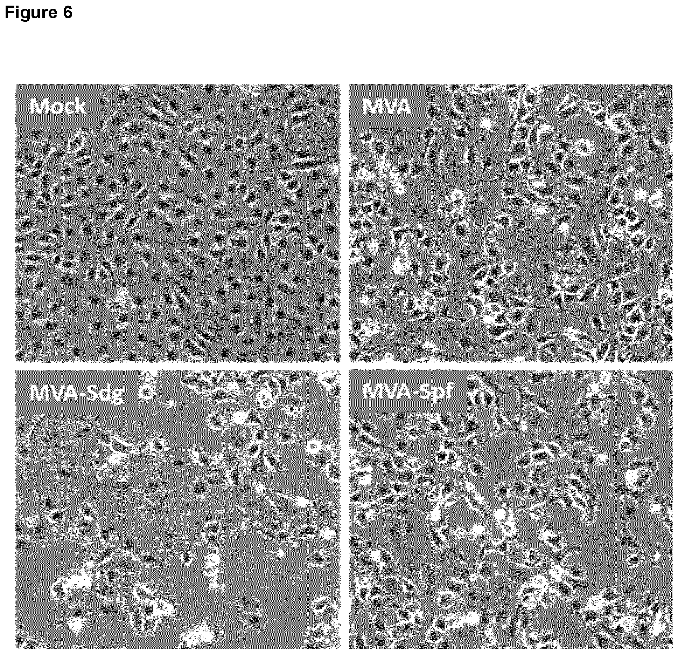

**Figure 6****. Fusion phenotype in BSC-1 cells infected with MVA virus recombinants.** Note the large syncytia caused by infection with MVA-Sdg and their absence in cultures infected with MVA-Spf expressing the pre-fusion stabilized form of the Spike glycoprotein.

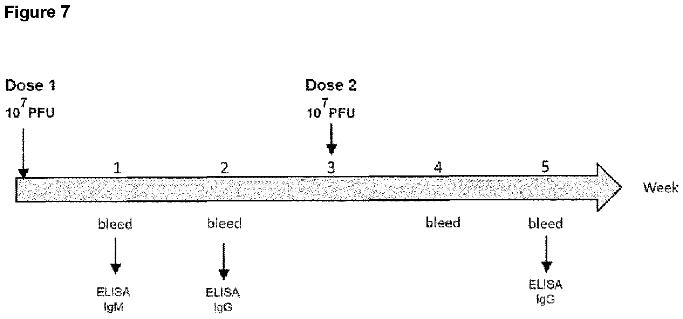

**Figure 7****. Immunogenicity in Balb/c mice: Immunization schedule. A two-dose** immunization schedule separated by 21 days was used. Time of blood collection for antibody testing is indicated.

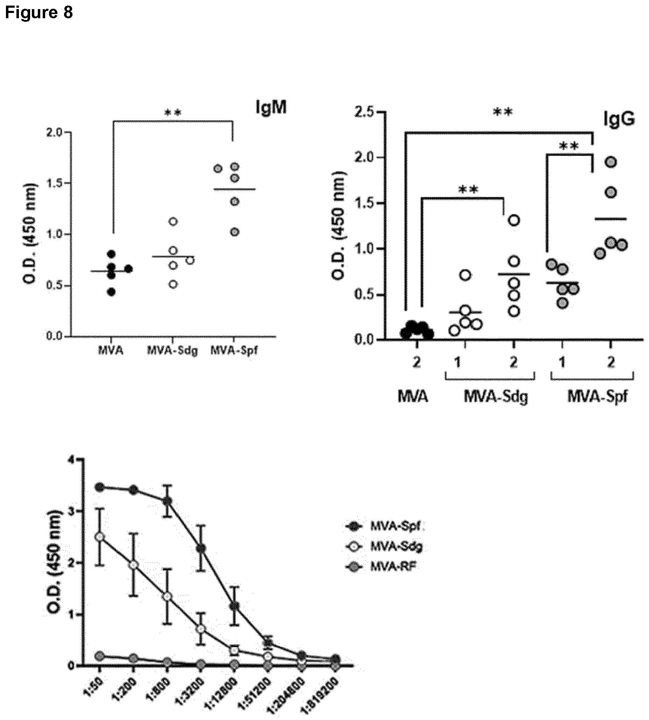

**Figure 8****. Immunogenicity in Balb/c mice: antibody response. Results of the ELISA** test with the sera of the vaccinated animals are shown. Top panels show the IgM and IgG levels specific to the RBD domain of the S glycoprotein. IgM levels correspond to day 7, whereas IgG levels are shown for day 14 (1) and day 35 (2) after the first inoculation of the vaccine candidates. Bottom panel shows the ELISA result of sera obtained 7 days after the second inoculation of the recombinants Spf and Sdg. Note the better response obtained with the Spf version. Response to a control virus (MVA-RF) expressing an incomplete version of the S protein is also shown.

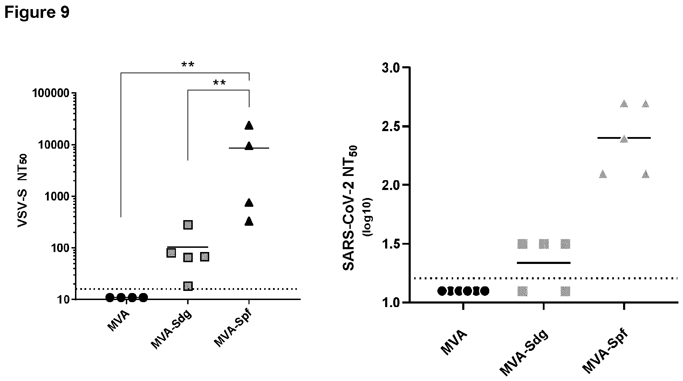

**Figure 9****. Immunogenicity in Balb/c mice: Neutralization titters. Neutralization** assays were carried out using VSV pseudotyped particles (left panel) or complete infectious SARS-Cov-2 virus (right panel), using the sera obtained 7 days after the last inoculation. Neutralization titer 50 (ND50) are represented on a logarithmic scale.

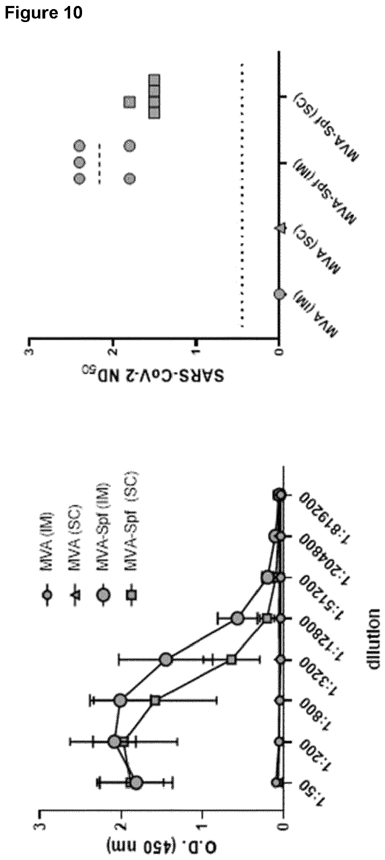

**Figure 10****. Immunogenicity in Balb/c mice: Inoculation route. Control MVA and the** vaccine candidate MVA-Spf were used to immunize mice by the intramuscular (IM) or the subcutaneous (SC) route. Left panel shows the level of RBD-specific antibodies in an ELISA assay. Right panel show the level of neutralizing antibodies in neutralization tests using infectious SARS-CoV-2. Note the superior response obtained after inoculation by the IM route.

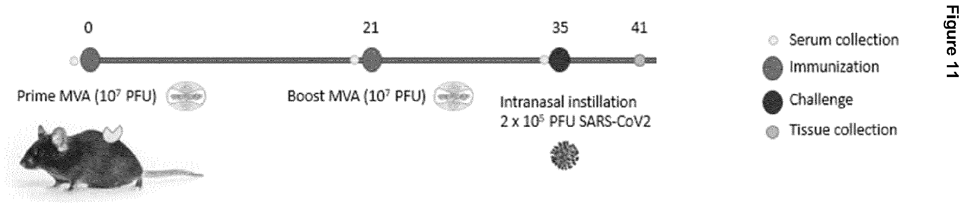

**Figure 11****. Vaccination of K18-hACE2 transgenic mice: Immunization schedule.** Mice were immunized with MVA vaccine candidates by the intramuscular route. Two inoculations on days 0 and 21. Challenge with 2 × 10⁵ PFUs of SARS-CoV-2 intranasally.

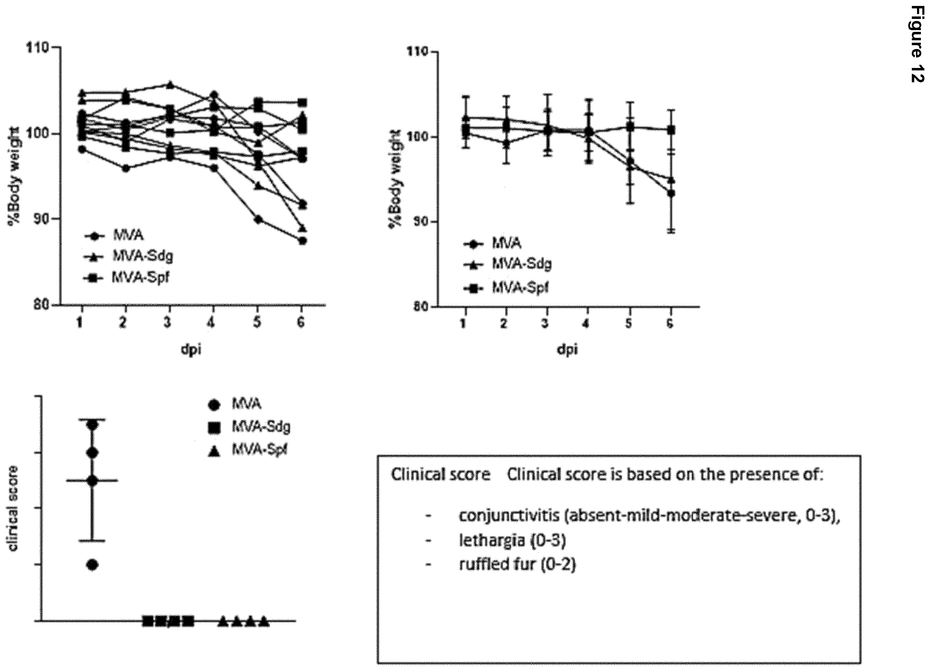

**Figure 12****. Vaccination of K18-hACE2 transgenic mice: pathology after challenge.** In the top panels the body weight recorded for individual animals (left) and the mean values in each group (right) are shown. Lower panel shows the clinical scores based on the criteria specified in the box.

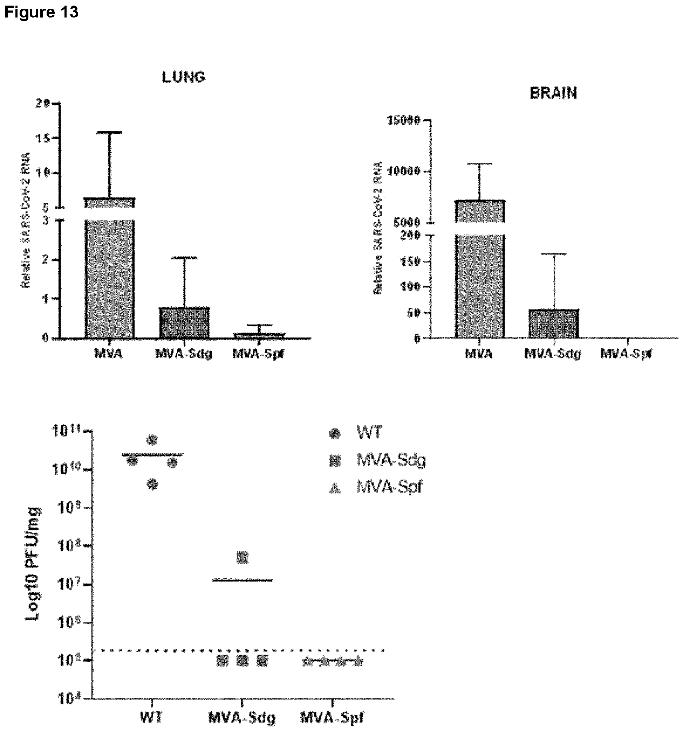

**Figure 13****. Vaccination of K18-hACE2 transgenic mice: Viral replication.** Upper panels: The level of SARS-CoV-2 viral RNA in lung and brain at 6 days post-challenge was measured by qRT-PCR at endpoint. Lower panel: SARS-CoV-2 viral titers in the brain after challenge. Virus present in the brain of mice was titrated. Dashed line indicates the limit of detection in our titration assay (points below the line indicate that no virus plaques were detected in a 10⁻⁵ dilution).

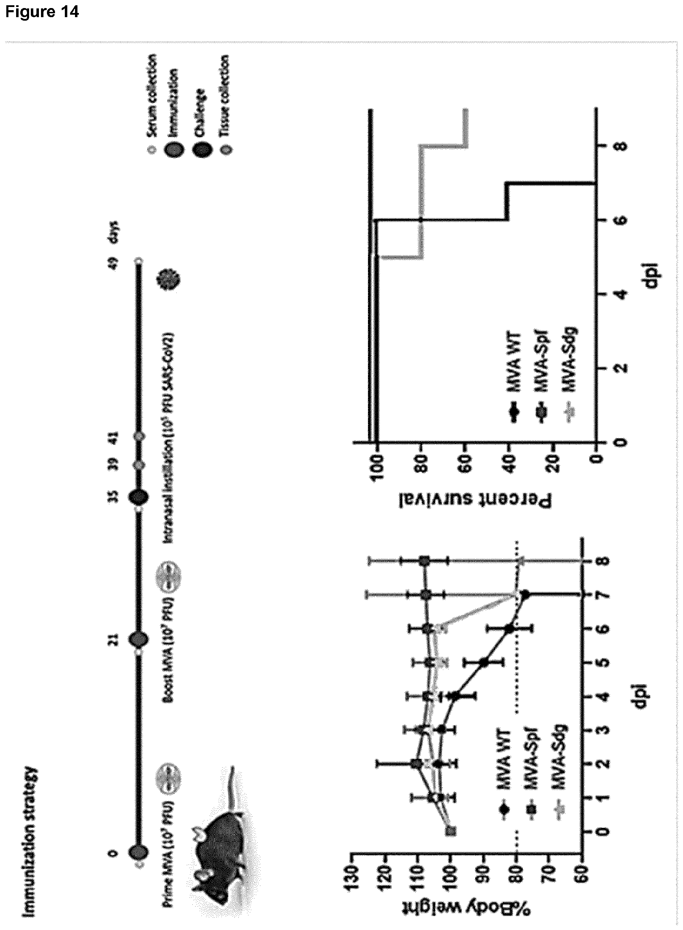

**Figure 14****. Vaccination of K18-hACE2 transgenic mice: protection and survival.** Upper panel shows the timeline for this experiment. Groups of hACE2 transgenic mice (8-10weeks old) were inoculated twice with 107 PFU of the MVA recombinants by the intramuscular route. Challenge was carried out by intranasal instillation of SARS-CoV-2 strain NR-52281 strain USA-WA1/2020, (MT020880.1) infective clone expressing nanoluciferase. Lower panels show the weight change of the animals (left), which was recorded daily after challenge and survival of the animals and the percent survival in each group (right). Note complete protection from body weight loss and from death in animals vaccinated with the MVA-Spf vaccine candidate.

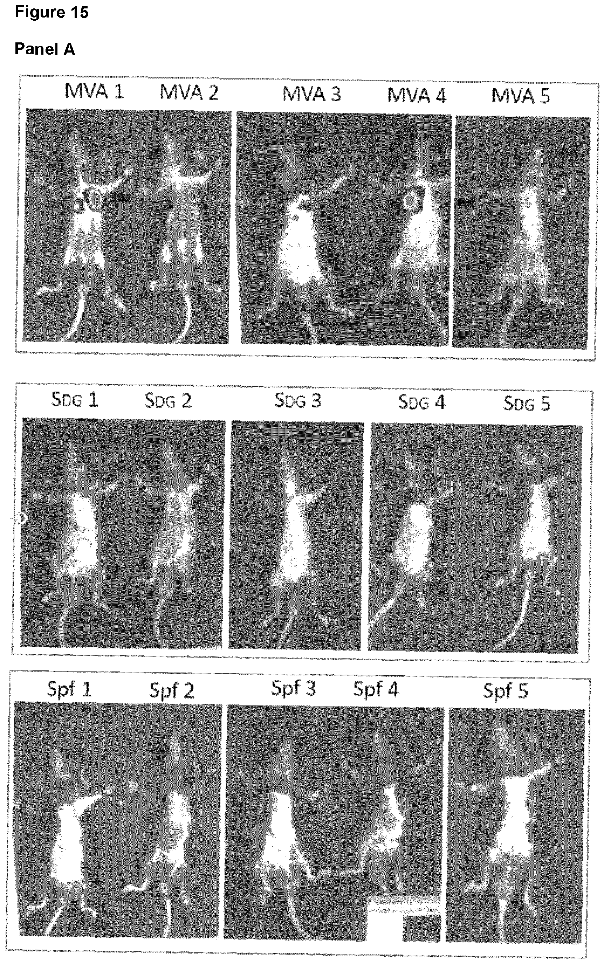

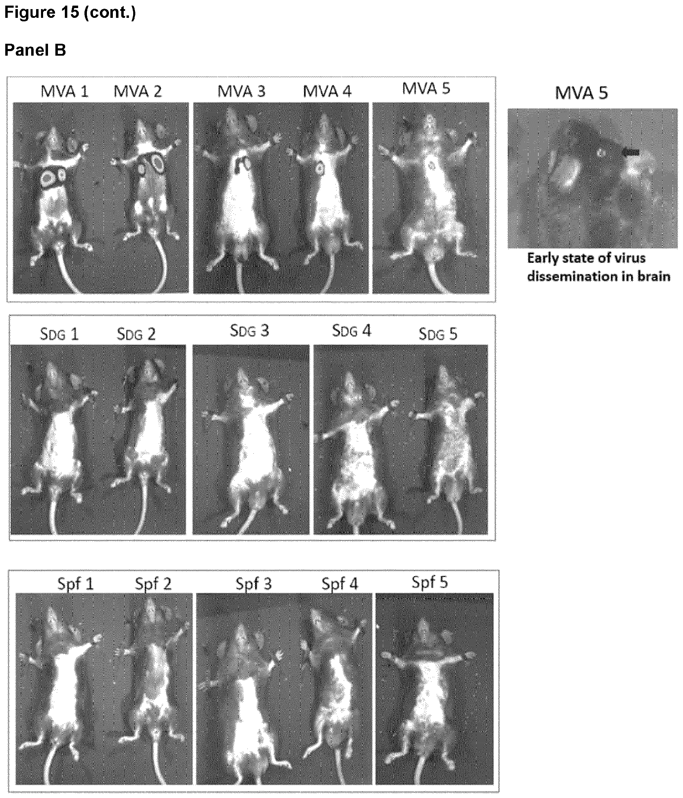

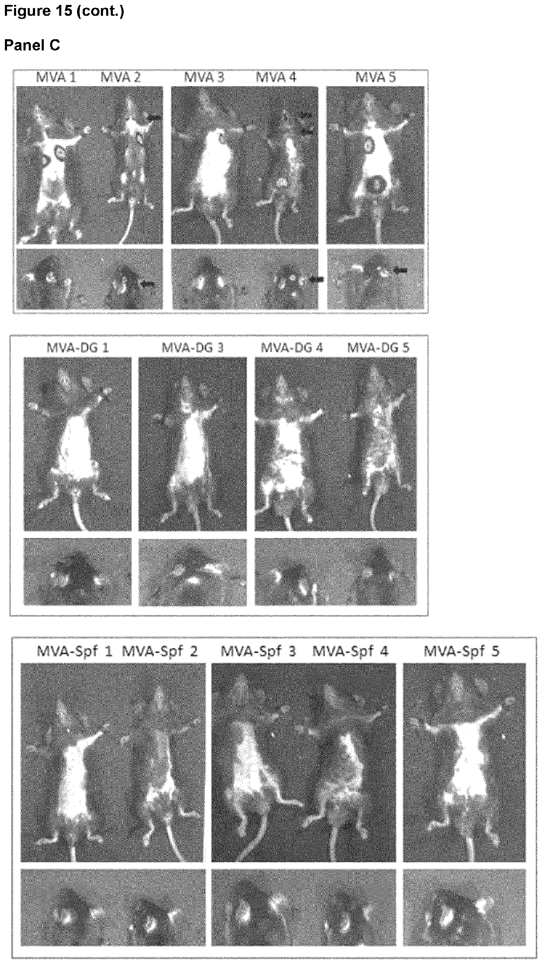

**Figure 15****. Vaccination of K18-hACE2 transgenic mice: virus dissemination.** Animals were infected with a SARS-CoV-2 clone expressing nanoluc luciferase. Mice were imaged for luminiscence at 2, 4 and 5 days after challenge. Note complete protection from virus dissemination in animals vaccinated with MVA-Sdg or MVA-Spf vaccine candidate. Panel (A): Virus dissemination at day 2 post-infection as seen by luciferase assay. Panel (B): Virus dissemination at day 4 post-infection as seen by luciferase assay. Panel (C): Virus dissemination at day 5 post-infection as seen by luciferase assay.

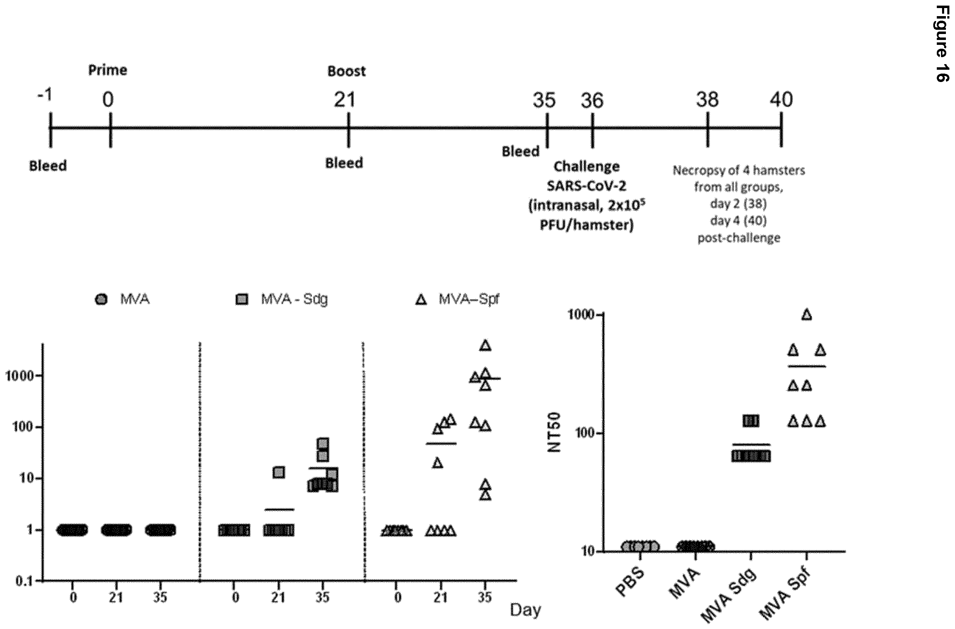

**Figure 16****. Protection of Syrian hamsters from SARS-CoV-2 infection. Upper panel** shows the timeline for this experiment. Groups Golden Syrian Hamsters were inoculated twice with 107 PFUs of the MVA recombinants by the intramuscular route. Challenge was carried out by intranasal inoculation of 2x 10⁵ PFUs of SARS-CoV-2. Lower panel: Neutralization titers in sera from hamsters. Neutralization titers were obtained using a VSV pseudotypes-based assay (left) or infectious SARS-CoV-2 virus (right). Titers in the left panel correspond to days 21 (one inoculation) and 35 (two inoculations) as indicated in the lower axis and those in the right panel correspond to sera from day 35, immediately before challenge.

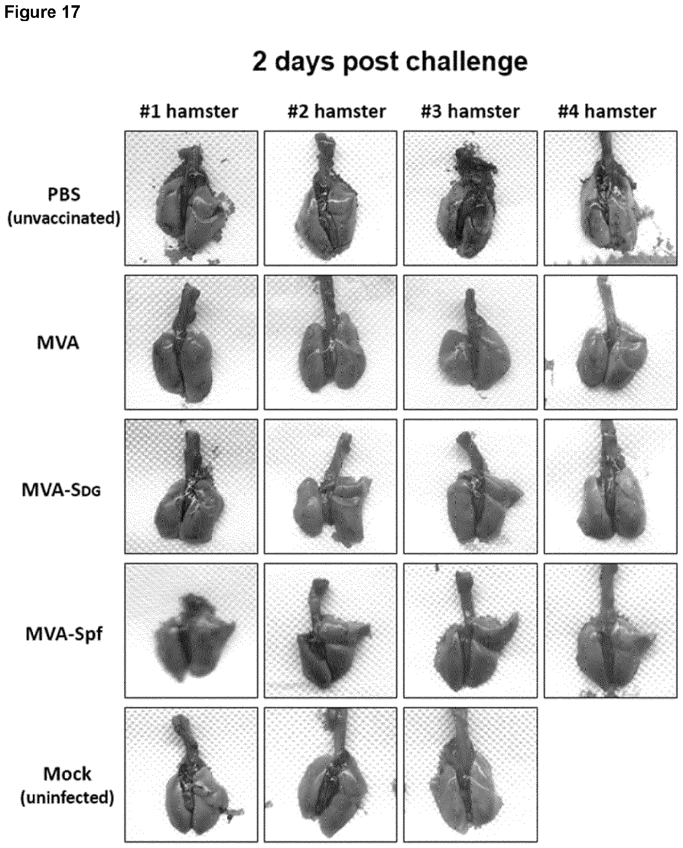

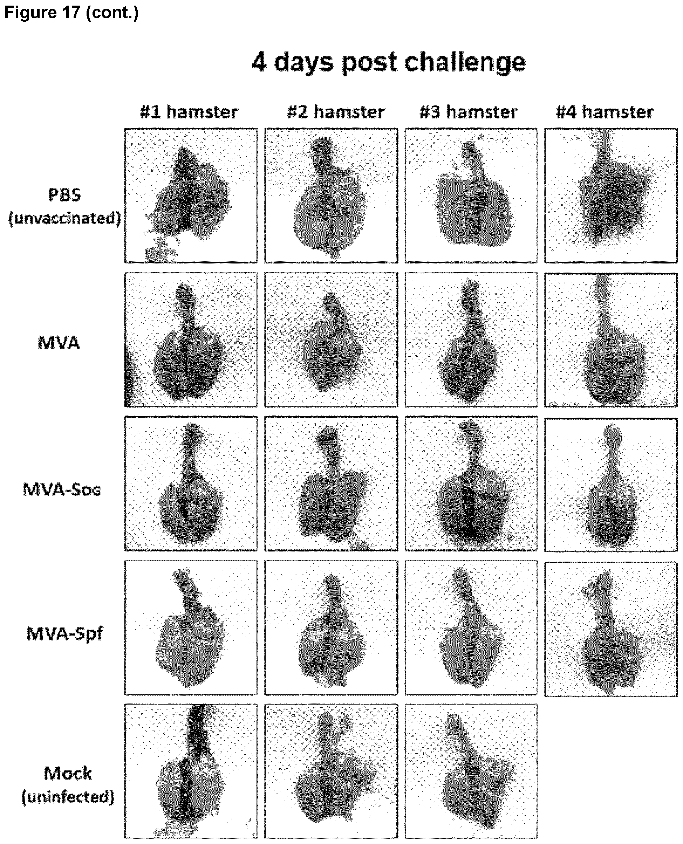

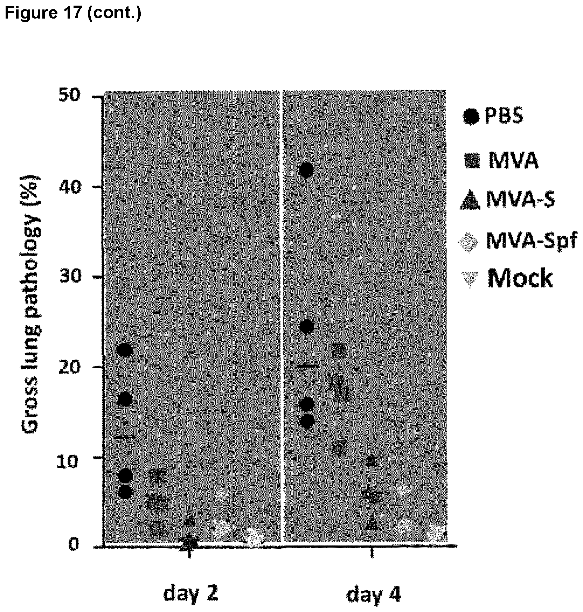

**Figure 17****. Vaccination of Syrian hamsters: lung pathology Images show the** protection from lung pathology in the hamster model. Images of the lungs from hamsters at days 2 and 4 after challenge. Note the complete absence of red lesions in animals vaccinated with MVA-Spf. Lower panel shows the pathology scores obtained for each animal. PBS corrsponds to unvaccinated animals. Mock are non-challenged animals.

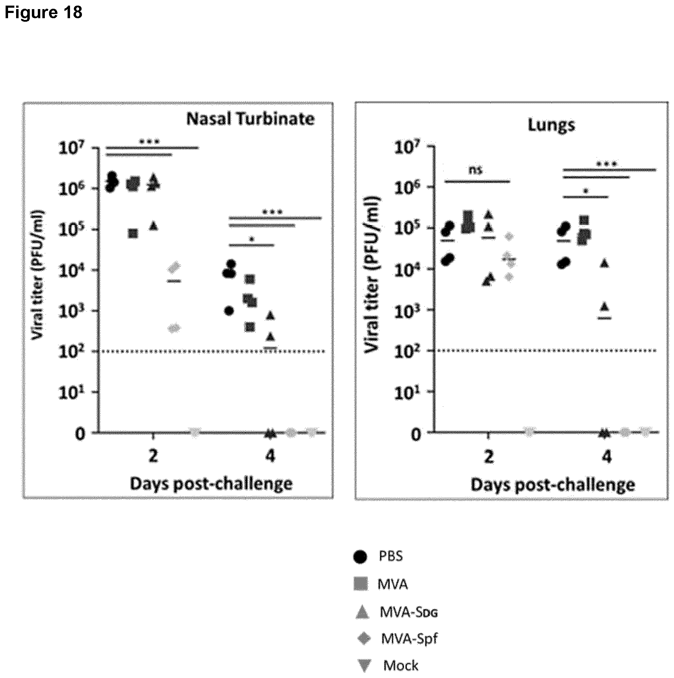

**Figure 18****. Vaccination of Syrian hamsters: viral titers SARS-CoV-2 viral titers were** obtained from lungs and nasal turbinates from the two groups of hamsters (day 2 and 4 post-challenge). In nasal turbinates, animals showed a relative reduction of viral titers after vaccination with MVA-Sdg at day 4. Animals vaccinated with MVA-Spf had decreased viral titers already at day 2, and undetectable levels of virus by day 4, indicating an effective degree of protection. Similar results were obtained in the lungs, where virus levels were undetectable by day 4 in animals vaccinated with MVA-Spf. Dashed line indicates the limit of detection in the virus plaque assay.

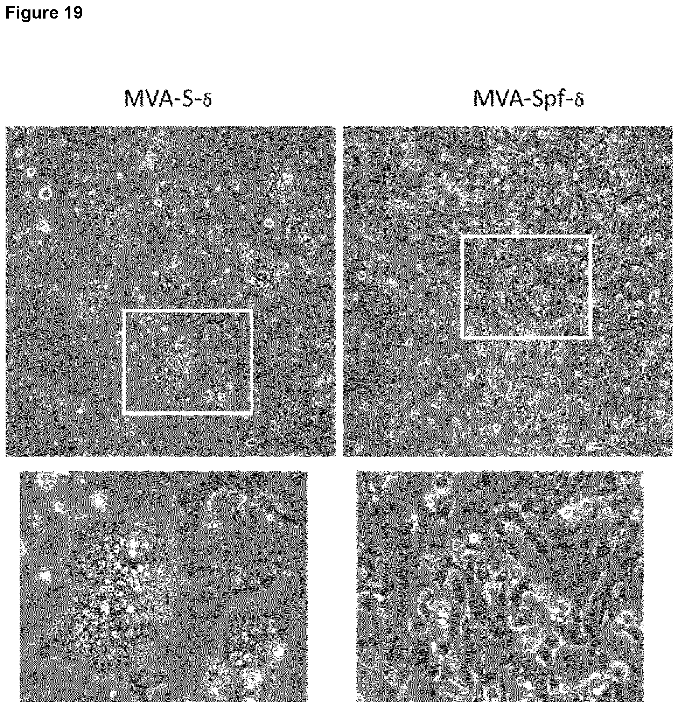

**Figure 19****. Cell fusion phenotype of MVA recombinants expressing the Delta version of SARS-CoV-2 S glycoprotein. BHK cells expressing the ACE2 receptor** were infected with the virus recombinants indicated in the top. Lower panels show enlargements of the white boxed areas. Note the large syncytia caused by expression of the unmodified S-δ protein and the lack of syncytia in cultures infected with the MVA-Spf-δ virus.

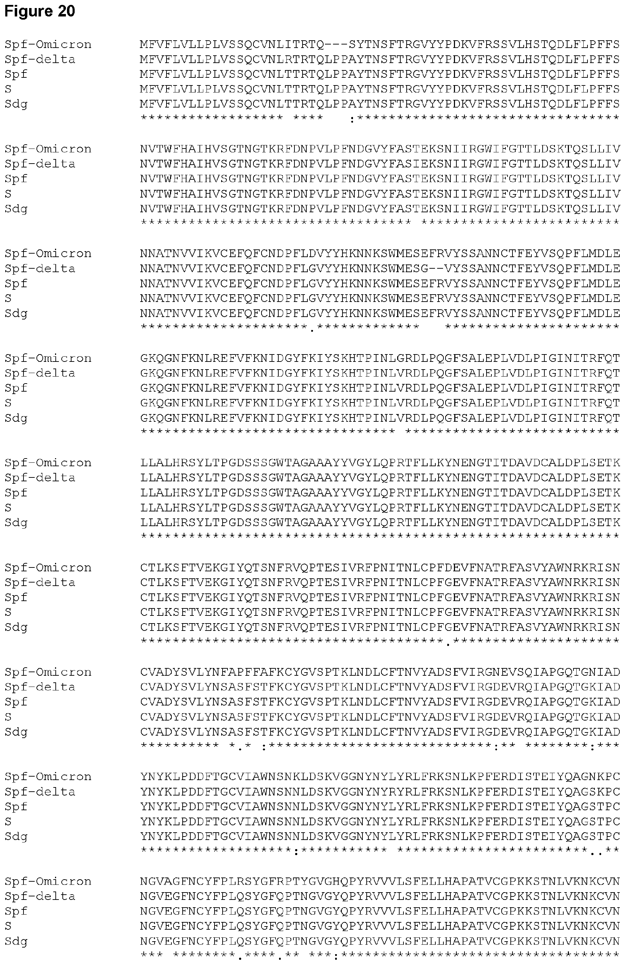

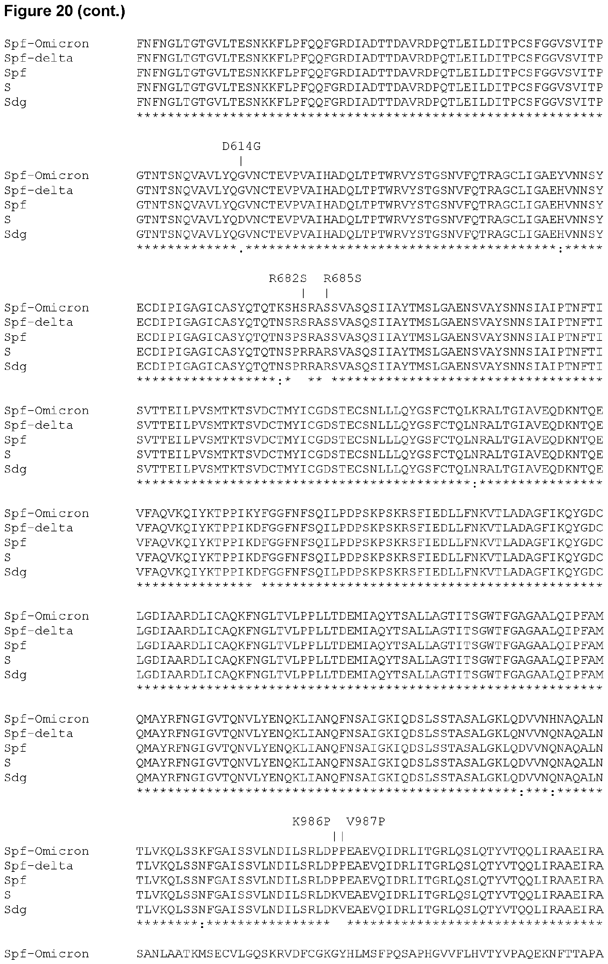

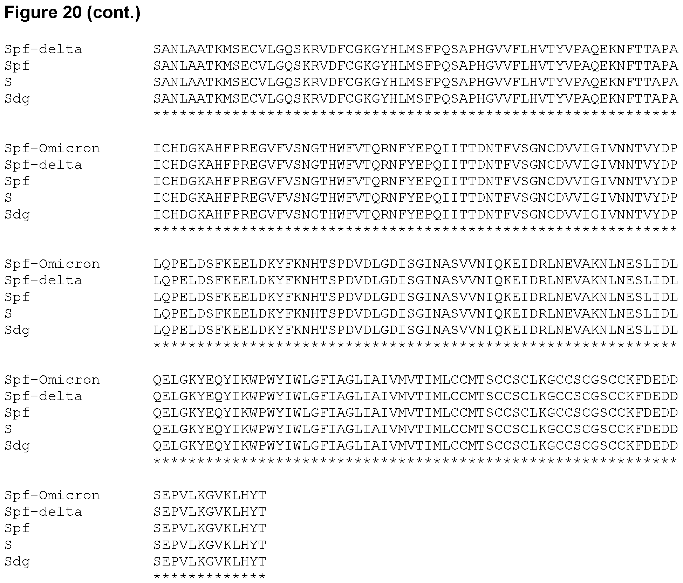

**Figure 20****. Multiple alignment of the different S proteins disclosed herein.**

### SUMMARY OF THE INVENTION

In one aspect, the present invention provides a modified vaccinia virus Ankara (MVA) vector comprising at least one nucleic acid encoding the Spike (S) protein of at least one SARS-CoV-2 variant, wherein said nucleic acid is characterized by being inserted in between the open reading frames of the F12L and F13L genes of the MVA genome without substituting, replacing, or interrupting said genes, and wherein the nucleic acid is further characterized for being codon optimized for Vaccinia virus, wherein the method for codon optimization for Vaccinia virus consists or comprises of the steps of:
i) choosing for every amino acid of the S protein of at least one SARS-CoV-2 variant or linage the synonymous codon with the highest frequency value according to Table 2 reproduced below, and
ii) reviewing the resulting nucleotide sequence of step i) so that in case the sequence TTTTTNT is present, such TTTTTNT sequence is substituted for a different nucleotide sequence with the proviso that the VACV RNA polymerase does not recognize said different sequence as an early transcription termination signal and that the amino acid sequence of the translated S protein is not altered or modified by the optimization process of i).

Preferably, the nucleic acid encoding for the S protein of at least one SARS-CoV-2 variant or linage is further characterized by being operably linked to the synthetic early/late Vaccinia virus promoter. Preferably, the S protein comprises at least the amino acid substitutions R682S, R685S, K986P and V987P, wherein the amino acid numbering is with respect to the Spike protein of the SARS-CoV-2 Wuhan-Hu-1 variant or linage (GenBank accession No QHD43416.1 or Uniprot ID: P0DTC2).

Preferably, the variant or linage of SARS-CoV-2 is selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617.1 (Kappa variant), Linage B.1.1.7 (United Kingdom variant), Linage B.1.617.2 (Delta variant), Linage B. 1.1.529 (Omicron variant), or any combination thereof.

Preferably, the nucleic acid encoding the Spike (S) protein consists of SEQ ID NO: 7, 8, or 27.

Preferably, the MVA is comprised in a composition, preferably in a vaccine composition or pharmaceutical composition, wherein said composition further comprises one or more pharmaceutically acceptable carriers, additives, antibiotics, preservatives, adjuvants, diluents and/or stabilizers.

In another aspect, the present invention refers to a vaccine **combination** comprising:
a. a composition comprising an immunologically effective amount of a modified vaccinia virus Ankara (MVA) vector as defined in previous aspect; and
b. a composition comprising an immunologically effective amount of a modified vaccinia virus Ankara (MVA) vector as defined in previous aspect,
wherein one of the compositions is a priming composition and the other composition is a boosting composition.

Furthermore, in another aspect of the invention refers to a vaccine combination comprising:
a. a composition comprising an immunologically effective amount of a modified vaccinia virus Ankara (MVA) vector as defined in the previous aspect, and
b. a composition comprising an immunologically effective amount of an immunogen of at least one SARS-CoV-2 variant;
wherein one of the compositions is a priming composition and the other composition is a boosting composition.

Preferably, the immunogen derived from at least one SARS-CoV-2 variant or linage is selected from the group consisting of viral vectors, recombinant proteins o peptides, nucleic acids, virus-like particles, nanoparticles, or any combination thereof.

In another aspect, the MVA vector and/or vaccine composition is used for generating an immunogenic and/or protective immune response in a subject in need thereof, preferably in a human subject, wherein said immune response is against at least the Spike (S) protein, or a fragment of said S protein, of at least one SARS-CoV-2 variant or linage, wherein said MVA vector and/or vaccine composition is used for priming and/or for boosting said immune response, and wherein the MVA is capable of producing said S protein, or a fragment of said S protein, in the subject to be treated.

In another aspect, the vaccine combination is used in generating an immunogenic and/or protective immune response in a subject in need thereof, preferably in a human subject, wherein said immune response is against at least the Spike (S) protein, or a fragment of said S protein, of at least one SARS-CoV-2 variant or linage, and wherein said combination is capable of producing said S protein, or fragment, in the subject to be treated.

Preferably, the MVA vector/s and/or the vaccine composition/s and/or the vaccine combinations is/are to be administered once, twice, three times or four times. Preferably, the MVA vector/s and/or the vaccine composition/s and/or the vaccine combinations are administered via the intramuscular route. Preferably, the MVA vector/s and/or the vaccine composition/s and/or the vaccine combinations are administered as a boosting composition at least 2 weeks after administering a priming composition. Preferably, the MVA vector/s and/or the vaccine composition/s and/or the vaccine combinations are administered as a boosting composition 2-12 weeks, preferably 2, 3 or 4 weeks, after administering the priming composition.

In another aspect, the present invention provides a kit comprising one, preferably two, or more doses of the MVA vector/s and/or the vaccine composition/s and/or the vaccine combinations as defined above. Preferably, the kit comprises an immunologically effective amount of an MVA vector as defined above in a first container for a first administration (priming) and in a second container for a second administration (boosting). Preferably, the kit further comprises at least another container comprising an immunologically effective amount of another immunogen derived from at least one SARS-CoV-2 variant or linage for a first or a second administration.

Preferably, the kit comprises an immunologically effective amount of an immunogen derived from at least one SARS-CoV-2 variant or linage in a first container for a first administration (priming), and an immunologically effective amount of a MVA vector as defined above in a second container for a second administration (boosting). Preferably, the kit comprises in a third, fourth or further containers comprising an MVA vector as defined above for a third, fourth or further administration.

In a further aspect, the kit is used for generating an immunogenic and/or protective immune response against at least one SARS-CoV-2 variant or linage in a subject in need thereof, preferably in a human subject. Preferably, the combination of compositions contained in the containers of the kit is capable of producing at least the Spike (S) protein, or a fragment of said S protein comprising at least one epitope, of least one SARS-CoV-2 variant or linage, in the subject to be treated, and generating an immunogenic and/or protective immune response against at least one SARS-CoV-2 variant or linage in a subject in need thereof.

Preferably, the MVA vector/s and/or the vaccine composition/s and/or the vaccine combinations are administered via the intramuscular route. Preferably, the boosting MVA vector/s and/or the vaccine composition/s and/or combinations is administered at least 2 weeks after administering the priming composition. Preferably, the boosting MVA vector/s and/or the vaccine composition/s and/or combinations is administered 2-12 weeks, preferably 2, 3 or 4 weeks, after administering the priming composition.

In a further aspect, the present invention relates to a method of generating an immunogenic and protective immune response against at least one SARS-CoV-2 variant or linage in a subject in need thereof, preferably in a human subject, the method comprising administering to the subject the MVA and/or vaccine compositions and/or combinations as defined in any of the previous aspects. Preferably, the method comprises administering the MVA and/or vaccine compositions and/or combinations for priming said immune response and/or for boosting said immune response. Preferably, said MVA and/or vaccine compositions and/or combinations of compositions contained in the containers are administered via the intramuscular route.

Preferably, the boosting MVA and/or vaccine compositions and/or combinations is administered at least 2 weeks after administering the priming composition. Preferably, the boosting MVA and/or vaccine compositions and/or combinations is administered 2-12 weeks, preferably 2, 3 or 4 weeks, after administering the priming composition. Preferably, the boosting MVA and/or vaccine compositions and/or combinations is administered two or more times to the subject.

In a further aspect, the present invention relates to a method for generating an immune response against at least one SARS-CoV-2 variant or linage in a subject in need thereof, preferably a human subject, the method comprising administering to the subject any of the MVA vector, vaccine composition and/or vaccine combinations according to any of previous aspects. Preferably, the method comprises administering the MVA and/or vaccine compositions and/or combinations for priming said immune response and/or boosting said immune response. Preferably, said MVA and/or vaccine compositions and/or combinations comprised in the containers are administered via the intramuscular route. Preferably, the boosting MVA and/or vaccine compositions and/or combinations is administered at least 2 weeks after administering the priming composition. Preferably, the boosting MVA and/or vaccine compositions and/or combinations is administered 2-12 weeks, preferably 2, 3 or 4 weeks, after administering the priming composition. Preferably, the boosting MVA and/or vaccine compositions and/or combinations is administered two or more times to the subject.

In a further aspect, the present invention relates to a method of inducing an enhanced immune response against at least one SARS-CoV-2 variant or linage in a subject in need thereof, preferably in a human subject, comprising administering to the subject the MVA and/or vaccine compositions and/or combinations according to any of the previous aspects. Preferably, said MVA and/or vaccine compositions and/or combinations comprised in the containers are administered via the intramuscular route.

In a further aspect, the present invention relates to a method for codon optimizing a nucleotide sequence for Vaccinia virus (VACV), the method comprising the steps of:
i) choosing for every amino acid of the protein whose nucleotide sequence is to be codon optimized, the most used synonymous codon according to the codon usage table from Vaccinia virus, and
ii) reviewing the resulting nucleotide sequence of step i) so that in case the sequence TTTTTNT is present, such TTTTTNT sequence is substituted for a different nucleotide sequence with the proviso that the VACV RNA polymerase does not recognize said different sequence as an early transcription termination signal and that the amino acid sequence of the translated S protein is not modified by the optimization process.

### DESCRIPTION OF THE INVENTION

### General definitions

It must be noted that, as used herein, the singular forms "a", "an", and "the", include plural references unless the context clearly indicates otherwise.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention. Thus, unless otherwise noted, the expression "at least" or "at least one of" as used herein includes individually each of the recited objects after the expression and the various combinations of two or more of the recited objects unless otherwise understood from the context and use.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of" does not exclude materials or steps that do not materially affect the basic and novel characteristics of the claim.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

The term "subject" as used herein is a living multi-cellular vertebrate organisms, including, for example, humans, non-human mammals and (non-human) primates. The term "subject" may be used interchangeably with the term "animal" herein.

As used herein, the term "enhanced" when used with respect to an immune response against SARS-CoV-2, such as an antibody response (e.g., neutralizing antigen specific antibody response), a cytokine response or a CD8 or CD4 T cell response (e.g., immunodominant T cell response), refers to an increase in the immune response in an animal administered with recombinant MVA according to the present invention relative to the corresponding immune response observed from the animal administered with a vector that does not express any SARS-CoV-2 proteins, e.g., a MVA-WT vector.

"Modified vaccinia virus Ankara (MVA) vector" as used herein is an attenuated replication-deficient vaccinia virus that has been genetically modified to include in its genome foreign DNA (that is, DNA that does not naturally belong to poxviruses). In particular, the modified vaccinia virus Ankara vector of the present invention has been genetically modified to express SARS-CoV-2 protein(s). Thus, the modified vaccinia virus Ankara vector of the present invention refers to viral particles of MVA that comprise inside the viral particle the MVA genome, wherein at least one nucleic acid encoding an antigenic protein of at least one SARS-CoV-2 virus variant or linage has been introduced in the MVA genome.

As used herein, the terms "antigenic protein" or "antigenic determinant" refers to any molecule that stimulates a host's immune system to make an antigen-specific immune response, whether a cellular response or a humoral antibody response. Antigenic determinants may include proteins, polypeptides, antigenic protein fragments, antigens, and epitopes which still elicit an immune response in a host and form part of an antigen, homologues or variants of proteins, polypeptides, and antigenic protein fragments, antigens and epitopes including, for example, glycosylated proteins, polypeptides, antigenic protein fragments, antigens and epitopes, and nucleotide sequences encoding such molecules. Thus, proteins, polypeptides, antigenic protein fragments, antigens and epitopes are not limited to particular native nucleotide or amino acid sequences but encompass sequences identical to the native sequence as well as modifications to the native sequence, such as deletions, additions, insertions and substitutions.

The term "epitope" refers to a site on an antigen to which B- and/or T-cells respond, either alone or in conjunction with another protein such as, for example, a major histocompatibility complex ("MHC") protein or a T-cell receptor. Epitopes can be formed both from contiguous amino acids or noncontiguous amino acids juxtaposed by secondary and/or tertiary folding of a protein. Epitopes formed from contiguous amino acids are typically retained on exposure to denaturing solvents, while epitopes formed by tertiary folding are typically lost on treatment with denaturing solvents. An epitope typically includes at least 5, 6, 7, 8, 9, 10 or more amino acids - but generally less than 20 amino acids - in a unique spatial conformation. Methods of determining spatial conformation of epitopes include, for example, x-ray crystallography and 2-dimensional nuclear magnetic resonance.

The term "synthetic early/late promoter" is referred herein as a vaccinia virus promoter comprising both early and late regulatory elements, wherein preferably said elements overlap in a region of at least 5 base pairs. Said promoter is widely known in the field, see e.g., Chakrabarti, S.; Sisler, J.R.; Moss, B. Compact, synthetic, vaccinia virus early/late promoter for protein expression. Biotechniques 1997, 23, 1094-1097. Preferably, the synthetic early/late promoter comprises or consists of SEQ ID NO 16, or or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16.

"Percent (%) amino acid sequence identity" with respect to proteins, polypeptides, antigenic protein fragments, antigens and epitopes described herein is defined as the percentage of amino acid residues in a candidate sequence that are identical with the amino acid residues in the reference sequence (i.e., the protein, polypeptide, antigenic protein fragment, antigen or epitope from which it is derived), after aligning the sequences and introducing gaps, if necessary, to achieve the maximum percent sequence identity. The same applies to "percent (%) nucleotide sequence identity", mutatis mutandis. Techniques for determining sequence identity between nucleic acids and amino acids are known in the art. Two or more sequences can be compared by determining their "percent identity." The percent identity of two sequences, whether nucleic acid or amino acid sequences, is the number of exact matches between two aligned sequences divided by the length of the shorter sequences and multiplied by 100.

It should be noted that the "percentage of identity" as used herein is decided in the context of a local alignment, i.e., is based on the alignment of regions of local similarity between sequences, contrary to a global alignment, which aims to align two sequences across their entire span. Thus, in the context of the present invention, percentage identity is calculated only based on local alignment comparison algorithm. Local alignments for purposes of determining percent amino acid sequence identity can be achieved in various ways that are within the skill in the art, for example, using publicly available computer software such as BLAST software. Those skilled in the art can determine appropriate parameters for measuring alignment, including any algorithms needed to achieve maximum alignment over the the sequences being compared.

The term "strain" or "clade" describes a microorganism, for instance, a virus, which shares common characteristics with other microorganisms, like basic morphology or genome structure and organization, but varies in biological properties, like host range, tissue tropism, geographic distribution, attenuation or pathogenicity. In the context of the present invention, the terms "strain", "variant", "linage" or "lineage" are considered synonymous and are used interchangeably to refer to a cluster of viral sequences derived from a common ancestor, preferably the SARS-CoV-2 Wuhan isolate GenBank accession number MN908947.3. In this specific context, it is thus preferably understood that the terms "SARS-CoV-2 strain/variant/linage/lineage" does not include viral genomes from other viruses, such as SARS or MERS viruses nor viral genomes derived from said other viruses.

Preferably, a SARS-CoV-2 variant or linage has at least about 80%, 81 %, 82%, 83%, 84%, 85%, 86%, 87%, 88%, or 89%, more typically, at least about 90%, 91 %, 92%, 93%, or 94% and even more typically at least about 95%, 96%, 97%, 98% or 99%, most typically, at least about 99% identity with the referenced protein, polypeptide, antigenic protein fragment, antigen and epitope at the level of nucleotide or amino acid sequence. Preferably, the referenced protein is the S protein of SARS-CoV-2 Wuhan-Hu-1 (GenBank accession No QHD43416.1 or Uniprot ID: P0DTC2).

More preferably, the term "variant" or "linage" includes all SARS-CoV-2 viral sequences that encode for a Spike protein with a percentage of amino acid sequence identity of at least 90%, 91%, 92%, 93%, 94%, preferably of at least 95%, 96%, 97%, 98%, or 99% to the Spike protein of the reference variant or linage SARS-CoV-2 Wuhan-Hu-1 (GenBank accession No QHD43416.1 or Uniprot ID: P0DTC2), when both Spike proteins are locally aligned, for example, by using Basic Local Alignment Search Tool (BLAST).

Thus, by "at least one variant of severe acute respiratory syndrome coronavirus 2 (SARS-CoV-2)" is meant at least one variant, strain, isolate, lineage or linage, and/or clade of the SARS-CoV-2 virus. The SARS-CoV-2 lineage nomenclature is described for example, in Rambaut A. et al. A dynamic nomenclature proposal for SARS-CoV-2 lineages to assist genomic epidemiology. Nat Microbiol. 2020; 5(11): 1403-1407. The different variants of SARS-CoV-2 can be found in databases such as Emma B. Hodcroft. 2021. "CoVariants: SARS-CoV-2 Mutations and Variations of Interest" (covariants.org/variants) or O'Toole A. et al., 2020 "A dynamic nomenclature proposal for SARS-CoV-2 lineages to assist genomic epidemiology", PANGO lineages (covlineages.org/).

In the context of multiple immunization regiments, an "homologous prime-boost combination" is referred herein as the administration of at least two doses of a vaccine, wherein the vaccine is the same in every dose. By "heterologous prime-boost combination" is referred herein as the administration of at least two doses of a vaccine, wherein the first dose comprises a vaccine that is different from the vaccine comprised in the second or subsequent doses. By "different" is meant herein that either the immunogen is different (different antigen) or that, even if the antigen is the same, it is carried by a different vector, or both (different vector and different antigen). For example, in the case of different vector, a heterologous prime-boost combination may be a first dose with a DNA-based vaccine, and a second dose with a MVA-based vaccine. By "Immunogen" is referred herein to a protein or a portion thereof that is capable of inducing an immune response in a mammal, such as a mammal infected or at risk of infection with a pathogen.

As used herein, the term "effective amount" of an agent, e.g., a MVA vector, is that amount sufficient to effect beneficial or desired results, for example, clinical results, and, as such, an "effective amount" depends upon the context in which it is being applied. For example, in the context of administering a therapeutic agent that functions as a vaccine, an effective amount is the amount capable of inducing an immunogenic and/or protective immune response, including T and B cell response, antibodies, cytokines, etc. The term "effective amount" can be used interchangeably with "effective dose," "therapeutically effective amount," or "therapeutically effective dose".

Amino acid numbering indicates the position of an amino acid residue within the protein or polypeptide. By "position" is meant herein a location in a protein or a polypeptide sequence. The positions or location of the amino acid residues of a protein or a polypeptide sequence can be numbered sequentially starting from the first amino acid residue which would then be located at position 1. Preferably, the first position or position number 1 corresponds to the first amino acid residue located at the 5-prime (5') end of the polypeptide chain that has a nitrogen atom or a free amino group. Thus, the numbering preferably starts from the first amino acid residue at the N terminal or 5' end of the protein or polypeptide and ends at the 3' end or C terminal end of the protein or polypeptide. As a mode of example, a protein of 137 amino acids will have those residues numbered 1 (first amino acid residue) until 137 (the last amino acid residue). Preferably, the positions of the amino acid residue are numbering using the amino acid sequence of the translated protein.

Additionally, in the context of the present invention and unless otherwise indicated, the amino acid numbering used herein is with respect to the amino acid sequence of the Spike protein of the SARS-CoV-2 Wuhan-Hu-1 variant or linage of GenBank accession No QHD43416.1 or Uniprot ID: P0DTC2, also disclosed as SEQ ID NO: 2. When defining specific amino acid substitutions, the amino acid numbering indicates the specific position of the amino acid residue that is to be substituted or mutated. For example, an amino acid substitution defined as R682S is to be interpreted as that the amino acid residue placed at position 682 of the reference Spike protein of the SARS-CoV-2 Wuhan-Hu-1 variant or linage of GenBank accession No QHD43416.1 or Uniprot ID: P0DTC2 or SEQ ID NO: 2, which is an R, is to be substituted by a S. Therefore, although different proteins and amino acid substitutions are defined herein, the numbering used is in reference to the S protein of SARS-CoV-2 Wuhan strain of GenBank accession No QHD43416.1 or Uniprot ID: P0DTC2 or SEQ ID NO: 2. Consequently, when an amino acid substitution is applied to a different S protein that may be longer or shorter than the reference S protein, for instance a S protein of SARS-CoV-2 Delta variant, the amino acid residue numbering needs to be extrapolated to the sequence of the protein and variant to which they are applied. For example, amino acid substitutions R682S, R685S, K986P and V987P according to the amino acid numbering of the reference S protein GenBank accession No QHD43416.1 or Uniprot ID: P0DTC2 (Wuhan parental variant or linage S protein), equals to amino acid substitution R680S, R683S, K984P and V985P in the Spike protein of Delta variant. The extrapolation of the amino acid numbering can be easily done by a skilled person by, e.g., aligning both amino acid sequences (the sequence of the reference S protein from GenBank accession No QHD43416.1 or Uniprot ID: P0DTC2 and the sequence of the S protein to which the numbering wants to be extrapolated). Fig. 20 represents the alignment between the S proteins of the SARS-CoV-2 variants defined herein and the reference S protein.

Several documents are cited throughout the text of this specification. Each of the documents cited herein (including all patents, patent applications, scientific publications, manufacturer's specifications, instructions, etc.), whether supra or infra, are hereby incorporated by reference in their entirety. To the extent the material incorporated by reference contradicts or is inconsistent with this specification, the specification will supersede any such material. Nothing herein is to be construed as an admission that the invention is not entitled to antedate such disclosure by virtue of prior invention.

### Recombinant MVA virus vector and vaccine compositions thereof

Vaccinia virus (VACV) is amongst the most extensively evaluated live vectors and has particular features in support of its use as recombinant vaccine: It is highly stable, cheap to manufacture, easy to administer, and it can accommodate large amounts of foreign DNA. It has the advantage of inducing both antibody and cytotoxic T cell responses and allows presentation of antigens to the immune system in a more natural way, and it was successfully used as vector vaccine protecting against several infectious diseases in a broad variety of animal models. Additionally, vaccinia vectors are extremely valuable research tools to analyse structure-function relationships of recombinant proteins, determine targets of humoral and cell- mediated immune responses, and investigate the type of immune parameters needed to protect against a specific disease.

Although unable to multiply in most mammalian cell lines, MVA retains its genome plasticity that allows the insertion of large amounts of foreign DNA (heterologous genes). Absence of pathogenicity for humans, inherent avirulence even in immunocompromised hosts, high-level expression of foreign antigens and adjuvant effect for immune responses make recombinant MVA (rMVA), expressing heterologous genes, an ideal vector for both prophylactic and therapeutic vaccination, as demonstrated by the wide use in prime-boost immunization strategies

Indeed, recombinant MVA (rMVA)-based vaccines comprising heterologous genes and therefore expressing heterologous proteins are able to elicit both humoral and cell-mediated adaptive immune responses and have been proven to be protective in animal models of several infectious diseases.

Cryo-electron tomography studies have elucidated the prefusion and postfusion structures of coronavirus S proteins. The S1 subunit is responsible for host-receptor binding while the S2 subunit contains the membrane-fusion machinery. During viral entry, the S1 subunit binds host receptors in an interaction thought to expose a secondary cleavage site within S2 (S2') adjacent to the fusion peptide for cleavage by host proteases. Structural studies also located the positions of the S1/S2 and S2' cleavage sites on the prefusion spike. Coronavirus S transitions from a meta-stable prefusion state to a highly stable postfusion state as part of the S protein's role in membrane fusion. Although this mechanism is necessary for viral attachment to the cell receptor and for induction of membrane fusion, antibodies against the postfusion S protein have less neutralizing capability. Thus, the instability of the prefusion state of the SARS-COV-2 S protein presents a significant challenge for the production of protein antigens for antigenic presentation of the prefusion epitopes that are most likely to lead to neutralizing responses. Therefore, vaccines able to elicit neutralizing antibodies that target the prefusion state of the S protein represent an urgent need.

In addition, due to the impact of protein expression in immunogenicity, MVA-based vectors comprising optimized nucleic acid sequences are desirable. The process of optimizing the nucleotide sequence coding for a heterologously expressed protein can be a crucial step for improving expression yields. However, several potential problems limit the usefulness of codon optimization for the expression of particular genes. For example, the secondary structure of a codon-optimized transcript may limit translation of the transcript.

In the present invention, several MVA-based vectors expressing different versions of the S protein were generated and their immunogenicity was evaluated. Further, different methods for codon optimization of the S protein were also tested. The results showed that a MVA vector named MVA-Spf, which comprises a nucleotide sequence with a particular VACV codon optimization (named hereinafter the codon optimization method of the present invention) and which expresses a stabilized prefusion S protein, elicited the highest immune response (see Figs. 8 and 9) which lead to protection upon challenge (see Fig. 12 and 13). The codon optimization method provided a superior protein expression of the S antigen as shown in Fig. 4, where a more intense signal is observed for the proteins expressed by MVA-Spf vector (which was codon optimized according to the method of the present invention) in comparison to the proteins expressed by MVA-Spfx virus (not codon optimized with the method of the present invention). These results highlight the importance of antigen optimization, not only at an amino acid level (such as introduction mutations to promote pre-fusion state), but also at a nucleotide level (codon optimizing the sequence) and provide a MVA vector as potential vaccine against SARS-CoV-2.

Thus, **a first aspect** of the present invention provides a modified vaccinia virus Ankara vector (MVA), comprising at least one nucleic acid encoding the Spike (S) protein, or a fragment of said S protein comprising at least one epitope, of at least one SARS-CoV-2 variant or linage, wherein said nucleic acid is further characterized by comprising at least one of the following:
a. being codon optimized for Vaccinia virus, and/or
b. being inserted in between the genes F12L and F13L of the MVA genome.

Preferably, the MVA vector of the first aspect is comprised in a vaccine composition. Preferably, said vaccine composition comprises an immunologically effective amount of the modified vaccinia virus Ankara (MVA) vector of the first aspect or any of its embodiments.

Preferably, the nucleic acid comprised in the MVA vector and encoding for the S protein, or a fragment of said S protein, of at least one SARS-CoV-2 variant or linage is characterized by being inserted in between the genes F12L and F13L of the MVA genome, preferably with the proviso that the insertion of said S protein does not disrupt any of said genes. Integration of the nucleic acid encoding the antigenic protein is performed preferentially by homologous recombination of the flanking regions of a so-called transfer vector, which initially comprises the nucleic acid encoding the antigenic protein according to the invention prior to its integration into the parental MVA genome. Since the nucleotide sequence of the S protein or fragment thereof is preferably inserted between the open reading frames of the F12L and F13L genes, the MVAs disclosed herein do not lack any gene, as the insertion is placed in between these two genes without substituting, replacing, or interrupting any of them.

Standard procedures to introduce nucleotide sequences or foreign genes encoding for antigenic proteins to generate recombinant VACV (rVACV) have been described in detail (Lorenzo MM, Galindo I, Blasco R. Construction and isolation of recombinant vaccinia virus using genetic markers. Methods Mol Biol. 2004;269:15-30. Doi: 10.1385/1-59259-789-0:015. PMID: 15114004) and rely on in vivo homologous recombination between acceptor VACV genome and a transfected transfer plasmid comprising the foreign gene flanked by VACV sequences. Additional approaches for the generation of recombinant MVA are, for example, disclosed in WO 04074493, WO 03023040 and WO 9702355.

In an embodiment, MVA recombinants included herein have been obtained through homologous recombination followed by selection based on plaque formation. These procedures have been used to generate rVACV and have been applied to the MVA strain (Sanchez-Puig JM, Lorenzo MM, Blasco R. Isolation of recombinant MVA using F13L selection. Methods Mol Biol. 2012; 890:93-111. Doi: 10.1007/978-1-61779-876-4_5. PMID: 22688762, and Sanchez-Puig JM, Blasco R. Isolation of vaccinia MVA recombinants using the viral F13L gene as the selective marker. Biotechniques. 2005 Nov;39(5):665-6, 668, 670 passim. Doi: 10.2144/000112012. PMID: 16312215.)

Upon homologous recombination by means of an infection with parental virus followed by a transfection with the plasmid transfer vector, selection and purification of recombinant viruses encoding the antigenic protein is performed. In an embodiment, the parental virus used lacks a gene related to virus transmission or plaque formation, and the transfected plasmid includes such gene, in such a way that recombinant viruses recover the said gene and regain the ability to form plaques in cell monolayers, Methods for generating recombinant MVAs by homologous recombination using plaque formation as a selectable marker are described in the literature:
Sanchez-Puig JM, Lorenzo MM, Blasco R. Isolation of recombinant MVA using F13L selection. Methods Mol Biol. 2012;890:93-111. Doi: 10.1007/978-1-61779-876-4_5. PMID: 22688762.
Lorenzo MM, Sanchez-Puig JM, Blasco R. Genes A27L and F13L as Genetic Markers for the Isolation of Recombinant Vaccinia Virus. Sci Rep. 2019 Oct 30;9(1):15684. Doi: 10.1038/s41598-019-52053-4. PMID: 31666569; PMCID: PMC6821840.

With regard to the codon optimization of the nucleic acid comprised in the MVA vector of the present invention, it is preferred that said nucleic acid encoding for the S protein, or a fragment of said S protein, of at least one SARS-CoV-2 variant or linage is characterized by being codon optimized for Vaccinia virus. Thus, in a preferred embodiment, the MVA vectors described herein comprise a nucleotide sequence that, instead of being codon optimized for the host organism (e.g., human being), are codon optimized for Vaccinia virus or comprise codon-usage bias for the Vaccinia virus.

By "codon optimization" is referred herein to as a the process used to improve gene expression and increase the translational efficiency of a gene of interest (in this case, the protein S or a fragment thereof of at least a SARS-CoV-2 variant or linage) by accommodating codon composition using synonymous codons without altering the encoded amino acid sequence. Usually, this is done by accommodating synonymous codon usage of the gene with respect to a reference (in this case, the Vaccinia virus). Codon optimization thus refers to nucleotide sequence modifications that use synonymous codon changes to increase performance of a given coding sequence in a particular application, usually to increase production of a protein product. "Being codon optimized" and "comprising a codon-usage bias" are synonymous and can be used interchangeable and refer to the preferential use of a particular codon (as opposed to other, synonymous codons) coding for an amino acid within an organism. Codon usage is known to vary among different organisms and also among different genes. Evaluation of the closeness of a particular gene sequence to the average codon usage of a species is usually performed by obtaining a "Codon adaptation index" (CAI) in which the codon usage table (CUT) of the gene is compared with that of the relevant species. The Codon adaptation index can be calculated using an online program "CAI calculation from DNA or RNA sequences: http://genomes.urv.es/CAIcal/". For example, a CAI=1 in a nucleotide sequence indicates that said nucleotide sequence comprises the most frequently used synonymous triplet in said species.

The terms "codon usage table (CUT)" or "codon frequency table" are used interchangeably and describe a table which correlates each codon that may be used to encode a particular amino acid with the frequencies with which each codon is used to encode that amino acid in a specific organism or virus, in this case Vaccinia virus. Codon usage tables are derived directly from the genomes sequence. There are repositories with codon usage statistics for many organisms and viruses that has available compiled sequencing data. CUT tables for multiple species can be obtained from public databases such as "the codon usage database: https://www.kazusa.or.jp/codon/".

Codon usage bias is observed in genomes of all organisms including viruses, and is the deviation (bias) from the statistically expected frequency for each synonymous codon in a coding sequence. By "synonymous codon" is referred herein as different codons that encode the same amino acid.

Several codon optimization methods are known to those of skill in the art. In some embodiments, the codon optimization method is performed using the codon adaptation index (CAI), which is essentially a measurement of the distance of a gene's codon usage to the codon usage of a predefined set of highly expressed genes. Alternative codon optimization methods for determining a codon usage bias include MILC (measure independent of length and composition) and relative synonymous codon usage (RSCU), which is the observed frequency of a particular codon divided by the frequency expected from equal usage of all the synonymous codons for that amino acid. RSCU values close to 1.0 indicate a lack of bias for the particular codon, whereas departure from 1.0 reflects codon usage bias.

Described herein is a specific method to codon optimize a sequence, named from herein after "the codon optimization method of the invention". The codon optimization method of the invention comprises or consists of the steps of i) choosing for every amino acid of the protein whose nucleotide sequence is to be codon optimized, the most used synonymous codon according to the codon usage table from the organism of interest (i.e., the organism that will express said codon optimized protein, or the organism to which the nucleotide sequence is to be optimized for). By "choosing the most used synonymous codon" is meant to include in the optimized sequence the codon that, according to the codon usage table for said organism, has the highest value of frequency per thousand. The resulting sequence of step i) is an optimized sequence.

In a preferred embodiment, the codon optimization method of the present invention is used to codon optimize a sequence for Vaccinia virus, and therefore the VACV is the organism of interest according to step i) of the method. In this case, it is preferred that the codon usage table for Vaccinia virus used to choose the most used synonymous codon is Table 2 (retrieved by the Codon Usage Database https://www.kazusa.or.jp/codon/, using Data Source NCBI-GenBank Flat File Release 160.0 [June 15 2007]:

### Vaccinia WR codon usage table for carrying out the codon optimization method of the present invention

### Vaccinia virus WR [gbvrl]: 218 CDS's (57854 codons) fields: [triplet] [frequency: per thousand] ([number])

UUU 33.0( 1912) UCU 23.8( 1375) UAU 36.5( 2114) UGU 15.8( 916)
UUC 13.2( 762) UCC 9.4( 545) UAC 15.1( 873) UGC 4.9( 283)
UUA 25.8( 1494) UCA 14.8( 857) UAA 2.3( 135) UGA 0.9( 50)
UUG 18.5( 1068) UCG 8.3( 479) UAG 0.6( 33) UGG 7.3( 422)

CUU 11.5( 668) CCU 10.9( 628) CAU 14.7( 852) CGU 6.4( 371)
CUC 6.0( 349) CCC 4.3( 246) CAC 6.0( 350) CGC 2.5( 142)
CUA 18.1( 1045) CCA 12.3( 714) CAA 16.0( 924) CGA 5.9( 343)
CUG 6.8( 395) CCG 6.2( 360) CAG 6.3( 363) CGG 1.6( 92)

AUU 37.9( 2193) ACU 22.3( 1292) AAU 48.0( 2779) AGU 14.6( 846)
AUC 18.5( 1073) ACC 8.5( 489) AAC 20.4( 1180) AGC 5.8( 333)
AUA 38.2( 2208) ACA 21.5( 1244) AAA 50.8( 2941) AGA 22.8( 1318)
AUG 28.1( 1628) ACG 8.7( 506) AAG 22.9( 1324) AGG 4.0( 233)

GUU 20.6( 1191) GCU 13.1( 756) GAU 51.1( 2955) GGU 13.0( 750)
GUC 8.1( 471) GCC 6.6( 384) GAC 16.2( 939) GGC 3.6( 206)
GUA 23.1( 1335) GCA 11.4( 657) GAA 39.7( 2299) GGA 20.8( 1202)
GUG 9.4( 545) GCG 7.1( 413) GAG 14.5( 840) GGG 2.8( 164)

Coding GC 34.03% 1st letter GC 39.67% 2nd letter GC 32.18% 3rd letter GC 30.23%

As a mode of example, if the following amino acid sequence "FV" wants to be codon optimized for Vaccinia virus according to the codon optimization method of the present invention, step i) of the method would be carried out as follows:
F: To encode for Phe, the codon chosen among those coding for Phe would be "UUU" and not "UUC", as "UUU" has a value of 33.0 frequency per thousand versus 13.2 value of "UUC".
V: To encode for Val, the codon chosen among those coding for Val would be "GUA" and not "GUC" nor "GUU" as "GUA" has a value of 23.1 frequency per thousand versus 20.6 and 8.1 value of "GUC" or "GUU", respectively.

The codon optimized sequence according to the step i) of the codon optimization method of the present invention would then be "UUUGUA".

It is important to note that, as a result of step i), some undesired nucleotide sequences might appear in the codon optimized sequence. For example, in the case of codon optimization for Vaccinia virus, the sequence "TTTTTNT" corresponds to the Vaccinia virus early transcription termination signal, that induces termination of vaccinia mRNAs at early times in infection (before DNA replication). Thus, it would be desirable and preferred to modify/remove said sequence from the codon optimized sequence resulting of step i) in order to avoid premature termination of the transcription and ensure good expression levels throughout the replication cycle. Importantly, the modification of this sequence must be done without altering the amino acid sequence of the translated protein nor its open reading frame. The modification of the early transcription termination signal may be done in a step ii) by introducing specific nucleotide substitutions in said transcription termination signal that disrupt said signal but do not alter nor modify the amino acid sequence encoded by the optimized nucleotide sequence, so that the RNA polymerase does not recognize the modified sequence as the early transcription termination signal anymore, but at the same time the resulting amino acid sequence or the open reading frame of the translated protein is not changed. In this context, the expression "the VACV RNA polymerase does not recognize said different sequence as an early transcription termination signal" means that the RNA polymerase does not identify the modified sequence as an early transcription termination signal, so that the transcription of the gene of interest is not prematurely finished. In an embodiment, at least 1, 2, 3, 4, 5, 6, or 7 nucleotide substitutions are introduced in the optimized sequence of step i) in order to remove the early transcription termination signal and/or make it unrecognizable by the RNA polymerase.

Thus, the resulting sequence of step ii) is an optimized sequence without early transcription termination signal.

Consequently, in an embodiment, the codon optimization method of the present invention includes a second step consisting of ii) reviewing the resulting nucleotide sequence of step i) so that, if an early transcription termination signal has been generated in the resulting optimized nucleotide sequence of step i), said early transcription termination sequence is modified or substituted for a different nucleotide sequence considering the following conditions:
- that the amino acid sequence and the open reading frame of the translated protein is maintained (i.e., it is not altered or modified), and
- that the early transcription termination signal sequence is modified so that the RNA polymerase does not recognize it as such, thereby avoiding early termination of the transcription.

Preferably, the early transcription termination signal is the sequence TTTTTNT (Vaccinia virus early transcription termination signal). Preferably, at least one nucleotide substitution is introduced to change the early transcription signal and to make it unrecognizable by the polymerase.

As a mode of example, if the sequence "AGTTTTTGTAC", encoding the amino acid sequence SerPheCysThr, is present in the optimized sequence resulting from step i) of the method, since it comprises the VACV early transcription termination signal "TTTTTNT", step ii) of the method is performed, in which the sequence "AGTTTTTGTAC" is modified to "AGTTTCTGTAC". Thus, in step ii) of the method, the TTT codon for Phe was substituted by TTC, thereby removing the "TTTTTNT" sequence present in the former sequence resulting of step i) and eliminating the VACV early transcription termination signal.

In a most preferred embodiment, the codon optimization method of the present invention is used to codon optimize a nucleic acid sequence for Vaccinia virus, wherein the method comprises or consists of the steps of i) choosing for every amino acid of the protein to be codon optimized for Vaccinia virus, the most used synonymous codon according to the codon usage table from Vaccinia virus, preferably Table 2, and ii) reviewing the resulting nucleotide sequence of step i) so that if the sequence "TTTTTNT" is present in the sequence resulting of step i), such "TTTTTNT" sequence is substituted/modified/changed for a different nucleotide sequence with the proviso that the VACV RNA polymerase does not recognize said different sequence as an early transcription termination signal and that the amino acid sequence and the open reading frame of the translated protein is not altered or modified by the optimization process or method.

In a preferred embodiment, the nucleic acid comprised in the MVA vectors of the present invention that encodes for the S protein, or a fragment of said S protein, of at least one SARS-CoV-2 variant or linage is characterized by being codon optimized for Vaccinia virus using the codon optimization method of the present invention.

In an embodiment, the MVA vector according to the first aspect or any of its embodiments comprises at least one nucleic acid encoding the full-length Spike (S) protein, wherein said nucleic acid is further characterized by being codon optimized for Vaccinia virus, preferably using the codon optimization method of the present invention, and/or by being inserted in between the open reading frames of the F12L and F13L genes of the MVA genome without substituting, replacing, or interrupting said genes.

With regard to the heterologous protein expressed by the MVA vectors of the invention, in some embodiments, the S protein encoded by the nucleic acid comprised in the MVA vector comprises, consists or consists essentially of the full-length S protein of at least a SARS-CoV-2 variant or linage. The full-length S protein may comprise or consist of amino acids 1 to 1288.

In an embodiment, the full-length Spike protein encoded by the nucleic acid comprised in the MVA vector corresponds to the S protein from the original Wuhan variant or linage (GenBank accession No QHD43416.1 or Uniprot ID: P0DTC2). In other embodiments, the MVA vector according to the first aspect or any of its embodiments comprises at least one nucleic acid encoding the full-length Spike (S) protein, wherein said S protein comprises at least one amino acid substitution with respect to the original Wuhan variant or linage. Said amino acid substitution(s) can be artificially introduced in the gene encoding for the protein, or it can be a naturally generated substitution giving rise to the different SARS-CoV-2 variants, or both.

In some embodiments, the S protein or a fragment thereof comprises artificial substitutions that inactivate the S1/S2 cleavage site, and mutations in HR1 that remove any strain in the turn region between HR1 and CH, i.e., to prevent the formation of a straight helix during fusion. In some embodiments, the designed S protein can additionally contain a truncation of the HR2 motif. Truncation of the HR2 domain leads to disruption of the HR1/HR2 fusion core and also stabilizes the prefusion S structure.

Thus, in some of the MVA vectors described herein, the inactivation of the S1/S2 cleavage site is achieved by introducing in the S protein at least the two amino acid substitutions R682S and R685S. In some embodiments, the S protein or an immunogenic fragment of said S protein comprising at least one epitope comprises three or more amino acid substitutions to inactivate the S1/S2 furin cleavage site. Inactivation of this cleavage site can be achieved by a number of sequence alterations (e.g., deletions or substitutions) within or around the site.

In addition to inactivation of the cleavage site, the soluble SARS-CoV-2 immunogen polypeptides can additionally contain a double mutation in the HR1 region that remove strain in the turn region (between HR1 and CH motifs) during fusion by preventing the formation of a straight helix. Thus, in an embodiment, the S protein, or an immunogenic fragment thereof, encoded by the nucleic acid comprised in the MVA vector of the present invention further comprises other amino acid substitutions aimed at maintaining the quaternary structure of the S protein in a prefusion state or form. As referred herein, a "prefusion protein" or a "protein in a prefusion state or form" is meant a S protein or a fragment of said protein that are designed to be stabilized in a prefusion conformation, preventing structural rearrangements that usually take place when the protein interacts with its receptor or other protein. A strategy to stabilize the S protein in a prefusion conformation is eliminating the causes of metastability by amino acid substitutions in various regions of S, particularly HR1 and in HR2. In an embodiment, amino acid substitutions to proline are performed in the S protein to provide a prefusion S protein.

In some of the MVA vectors described herein, the prefusion stabilization is achieved by introducing in the S protein at least the two amino acid substitutions K986P and V987P. In an embodiment, additional amino acid substitutions aimed at maintaining the prefusion quaternary structure of the S protein may be included in the S protein, or fragment thereof. Preferably, said mutations are selected from the group including, but not limited to, G769E, F817P, T859K, S884C, A892P, A893C, Q895P, A899P, T912P, K921P, L938F, G946P, A942P, Q957E, T961D, S975P, or any combination thereof.

In some embodiments, additional mutations of the wildtype soluble S sequence can be introduced in order to destabilize the postfusion S structure. In some embodiments, one or more proline and/or glycine substitution can be engineered in the region of HR1 that interacts with HR2 to form the fusion core. These substitutions function to disrupt the six-helix-bundle fusion core. In various embodiments, the mutations can include A942P, S943P, A944P, A942G, S943G and A944G. In further embodiments, the substitutions function to disrupt the six-helix-bundle fusion core. In various embodiments, the mutations can include S924P, T925P, A926P, S924G, T925G, and A926G. In some embodiments, one or more extra amino acid residues can be inserted into the region of HR1 that interacts with HR2 to form the fusion core. Similarly, these insertions also function to disrupt helical pattern of the fusion core. In various embodiments, the insertions can include insertion of G or GS between any residues in A942-A944. In some embodiments, the substitutions may be double amino acid substitutions such as K986G/V987G, K986P/V987P, K986G/V987P or K986P/V987G. In a most preferred embodiment, the encoded S protein has inactivated the S1/S2 cleavage site and is stabilized in a prefusion state, so that the S protein of at least one SARS-CoV-2 variant or linage comprises at least the amino acid substitutions R682S, R685S, K986P and V987P.

The substitutions comprised in the S protein or a fragment thereof may include, alternatively or additionally to the above-mentioned substitutions, natural mutations found in Variants of Concern (VOC) variants or linages that are circulating worldwide.

In a preferred embodiment, the at least one amino acid substitution is D614G, which is one of the first natural substitutions described for SARS-CoV-2 virus. In another preferred embodiment, the at least one amino acid substitution is E484Q. In another preferred embodiment, the amino acid substitutions consist of D614G mutation (DG) and the K986P and V987P (PP) mutations. In a most preferred embodiment, the encoded S protein comprises at least the amino acid substitutions D614G, R682S, R685S, K986P and V987P. As explained above, here and throughout the entire document, the amino acid residues are numbered with respect to the amino acid sequence of the Spike protein of the SARS-CoV-2 Wuhan-Hu-1 variant or linage (GenBank accession No QHD43416.1 or Uniprot ID: P0DTC2, also SEQ ID NO: 2), wherein said amino acid numbering is consecutive and starts from the N terminal end of the protein.

In an embodiment, the MVA vector comprising at least one nucleic acid encoding the Spike (S) protein, or a fragment of said S protein comprising at least one epitope, of at least one SARS-CoV-2 variant or linage according to the first aspect or any of its embodiments is derived from a variant of concern (VOC) as defined by the Centers for Disease Control and Prevention (CDC) "SARS-CoV-2 Variant Classifications and Definitions". The SARS-CoV-2 is observed to mutate, with certain combinations of specific point mutations proving to be more concerning than others. These mutations are the reason of the increased transmissibility, increased virulence, and possible emergence of escape mutations in new variants. The term "variant of concern" (VOC) is a designation used in newly emerged variants or linages of SARS-CoV-2 with mutations that provide an increased transmissibility and/or morbidity and/or mortality and/or decreased susceptibility to antiviral or therapeutic drugs and/or have the ability to evade immunity and/or ability to infect vaccinated individuals, among others.

In a preferred embodiment, the S protein, or a fragment of said S protein comprising at least one epitope, of at least one SARS-CoV-2 variant or linage is derived from the Wuhan-Hu-1 seafood market pneumonia virus isolate (GenBank accession number: MN908947). In another embodiment, the S protein, or a fragment of said S protein comprising at least one epitope, of at least one SARS-CoV-2 variant or linage is derived from the United Kingdom SARS-CoV-2 variant VOC 202012/01 (Lineage B.1.1.7). According to WHO, on 14 December 2020, authorities of the United Kingdom reported to WHO a variant referred to by the United Kingdom as SARS-CoV-2 VOC 202012/01 (Variant of Concern, year 2020, month 12, variant 01) also known as Lineage B.1.1.7 or 501Y.V1. This variant contains 23 nucleotide substitutions and is not phylogenetically related to the SARS-CoV-2 virus circulating in the United Kingdom at the time the variant was detected. Among the variant's several mutations there is one in the receptor-binding domain (RBD) of the spike protein that changes the asparagine at position 501 to tyrosine (N501Y). Another of the mutations in the VOC 202012/01 variant, the deletion at position 69/70del was found to affect the performance of some diagnostic PCR assays with an S gene target.

In another embodiment, the S protein, or a fragment of said S protein comprising at least one epitope, of at least one SARS-CoV-2 variant or linage according to the first aspect or any of its embodiments is derived from the South African SARS-CoV-2 variant (Lineage B.1.351). On 18 December 2020, national authorities in South Africa announced the detection of a new variant of SARS-CoV-2 that is rapidly spreading in three provinces of South Africa. South Africa has named this variant as Lineage B.1.351, also known as 501Y.V2. SARS-CoV-2 South African variant is characterised by three mutations K417N, E484K and N501Y in the RBD. While SARS-CoV-2 VOC 202012/01 from the UK also has the N501Y mutation, phylogenetic analysis has shown that the virus from South Africa are different virus variants.

In another embodiment, the S protein, or a fragment of said S protein comprising at least one epitope, of at least one SARS-CoV-2 variant or linage according to the first aspect or any of its embodiments is derived from the Brazilian SARS-CoV-2 variant VOC-202101/02 (Linage B.1.1.28). Brazilian variant is also known as Lineage P.1, also known as 20J/501Y.V3, Variant of Concern 202101/02 (VOC-202101/02). This variant of SARS-CoV-2 has 17 unique amino acid changes, ten of which are in its spike protein, including these three designated to be of particular concern: N501Y, E484K and K417T. This variant of SARS-CoV-2 was first detected by the National Institute of Infectious Diseases (NIID), Japan, on 6 January 2021.

A California variant has been also known as Variant of Concern CAL.20C. Thus, in another embodiment, the S protein, or a fragment of said S protein comprising at least one epitope, of at least one SARS-CoV-2 variant or linage according to the first aspect or any of its embodiments is derived from the Californian SARS-CoV-2 (Linage B.1.427 or B.1.429). This variant is characterized by the mutations S131, W152C in the N-terminal domain (NTD) of the spike protein and by the L452R mutation in the RBD of the spike protein. This variant was originally detected in California (Linage B.1.427 or B.1.429).

Many other variants have been described, such as the B.1.207 variant in Nigeria, which has a mutation in the spike protein (P681H) that is also found in the VOC 202012/01 variant, the B.1.617 variant in India (Linage B.1.617, Kappa (B.1.617.1), or Delta (B.1.617.2) variant), which has the mutations P681R, E484Q and L425R in the Spike protein, or the Danish variant, referred to as the "Cluster 5" variant by Danish authorities, and which has a combination of mutations not previously observed. Other VOC are the Delta variant (Linage B.1.617.2 and AY lineages) or the Omicron variant (Linage B.1.1.529). Thus, in another embodiment, the nucleic acid comprised in the MVA and encoding the S protein, or a fragment of said S protein comprising at least one epitope, of at least one SARS-CoV-2 variant or linage according to the first aspect or any of its embodiments, is derived from the SARS-CoV-2 Delta variant or linage (Linage B.1.617.2 and AY Pango lineages). Preferably, said nucleic acid derived from the SARS-CoV-2 Delta variant or linage encodes for a S protein that comprises at least the substitutions R682S, R685S, K986P, V987P, G142D, 156del, R158G, L452R, T478K, D614G, P681R, D950N. Preferably, the SARS-CoV-2 Delta variant or lineage is hCoV-19/Spain/GA-VITHAS-51592630/2021 (GISAID database Accession ID: EPI_ISL_1824684).

In another embodiment, the nucleic acid comprised in the MVA and encoding the S protein, or a fragment of said S protein comprising at least one epitope, of at least one SARS-CoV-2 variant or linage according to the first aspect or any of its embodiments, is derived from the Omicron SARS-CoV-2 variant or lineage (Linage B.1.1.529 including BA.1, BA.1.1, BA.2, BA.3, BA.4 and BA.5 Pango Linages), preferably Linage B.1.1.529 BA2. Preferably, said nucleic acid derived from the SARS-CoV-2 Omicron variant or linage encodes for a S protein that comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, N 969K. Preferably, said nucleic acid derived from the SARS-CoV-2 Omicron variant or linage encodes for a S protein that comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, and N969K, A67V, Del69-70, T95I, Del143-145, Del211, L212I, Ins214EPE, S371L, G446S, G496S, R346K, T547K, N856K, and L981F. Preferably, said nucleic acid derived from the SARS-CoV-2 Omicron variant or linage encodes for a S protein that comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, N969K, T19I, Del24-26, A27S, V213G, S371F, T376A, D405N, and R408S. Most preferably, the SARS-CoV-2 Omicron variant or lineage is hCoV-19/Spain/GA-CHUVI-21822434/2022 (GISAID database Accession ID: EPI_ISL_12057271).

The rising variants worldwide can be easily found by the skilled person, e.g., in the website databases such as disclosed by Emma B. Hodcroft. 2021. "CoVariants: SARS-CoV-2 Mutations and Variations of Interest." (covariants.org/variants) or by O'Toole A. et al., 2020 "PANGO lineages" (cov-lineages.org/). It is thus to be understood that the present invention covers MVAs encoding for S proteins or fragments of said proteins that are derived from any strain or clade or variant or lineage or isolate of SARS-CoV-2.

In a preferred embodiment, the S protein, or a fragment of said S protein comprising at least one epitope, of at least one SARS-CoV-2 variant or linage according to the first aspect or any of its embodiments is derived from a SASR-CoV-2 variant selected from the group including, but not limited to, Wuhan-Hu-1 seafood market pneumonia virus isolate (GenBank accession number: MN908947), Linage B.1.1.28 (Brazilian variant) , Linage B.1.351 (South African variant), Linage B.1.427 or Linage B.1.429 (California variant), Linage B.1.617.1 (Kappa variant), or Linage B.1.1.7 (United Kingdom variant), Linage B.1.617.2 (Delta variant), Linage B.1.1.529 (Omicron variant) and/or any combination thereof. In a preferred embodiment, the S protein, or a fragment of said S protein comprising at least one epitope, of at least one SARS-CoV-2 variant or linage according to the first aspect or any of its embodiments is derived from a SASR-CoV-2 variant selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617.1 (Kappa variant), or Linage B.1.1.7 (United Kingdom variant), Linage B.1.617.2 (Delta variant), Linage B.1.1.529 (Omicron variant) and/or any combination thereof.

In the context of the present invention, the nucleic acid comprised in the MVA described herein may optionally encode antigenic domains or antigenic protein fragments comprising at least one epitope of the S protein rather than the entire full-length antigenic S protein. Fragments can be at least 300 amino acids long, but can be longer, such as, e.g., at least 350, 400, 500, 600, 700, 800, 900, 1000, 1200 amino acids long, or any length in between. In some embodiments, at least one nucleic acid fragment encoding an antigenic S protein fragment or immunogenic S polypeptide thereof is inserted into the genome of the recombinant viral vector of the invention.

In some embodiments, the S protein encoded by the nucleic acid comprised in the MVA vectors of the present invention or any of its embodiments consists of a truncated protein lacking at least 5, 10, 15, 20, 15, 30, 35, 40, 45, 50, 60, 70, 80, 90, 100, 200, 300, 400, or more than 400 amino acid of the full length S protein. In some embodiments, the S protein comprises or consists of a truncated protein where the N-terminal domain has been removed. In some embodiments, the S protein comprises or consists of a truncated protein where the C-terminal domain has been removed. In some embodiments, the S protein comprises or consists of a truncated protein consisting of only the S1 and S2 subunits. In some embodiments, the S protein comprises or consists of a truncated protein where the cytosolic domain has been removed. In some embodiments, the S protein comprises or consists of a truncated protein where the transmembrane domain has been removed. In some embodiments, the S protein comprises or consists of a soluble S protein wherein the transmembrane and the cytosolic domains has been removed. In some embodiments, the S protein comprises or consists of a soluble S protein comprising or consisting of the RBD.

Thus, a "fragment" of the S protein or a "truncated S protein" of at least a SARS-CoV-2 virus according to the present invention is a partial amino acid sequence of the SARS-CoV-2 S protein or a functional equivalent of such a fragment. A fragment is shorter than the full-length complete SARS-CoV-2 S protein and preferably comprises or consists of the amino acids 682-987 of the full length S protein. A fragment of the SARS-CoV-2 S protein also includes peptides having at least 15, 20 or 25 contiguous amino acid residues having at least about 70%, at least about 80%, at least about 90%, preferably at least about 95%, more preferably at least 98% sequence identity with at least about 15, 20 or 25 contiguous amino acid residues of any of the S proteins described in the present invention.

A fragment that "corresponds substantially to" a fragment of the SARS-CoV-2 S protein is a fragment that has substantially the same amino acid sequence and has substantially the same functionality as the specified fragment of the SARS-CoV-2 S protein. A fragment that has "substantially the same amino acid sequence" as a fragment of the SARS-CoV-2 S protein typically has more than 90% amino acid identity with this fragment. Included in this definition are conservative amino acid substitutions.

In a further embodiment, the at least one nucleic acid comprised in the MVA vectors described above according to the first aspect or any of its embodiments is operably linked and under the control of a VACV-specific promoter, an orthopox virus-specific promoter, a poxvirus-specific promoter or a synthetic promoter. A number of promoters may be used for the present invention. For the expression of the nucleic acid encoding an antigenic protein according to the invention several promoters, such the 30K and 40K promoters (US 5,747,324, A Strong Synthetic Early-Late Promoter (Sutter, et al., Vaccine (1994), 12, 1032-1040)), the P7.5 promoter (Endo et al., J. Gen. Virol. (1991) 72, 699-703) and a promoter derived from the cow pox virus type A (inclusion ATI gene) (Lee et al., J. Gen. Virol. 1998), 79, 613) can be used.

In another preferred embodiment, the nucleic acid is operably linked and under the control of a synthetic promoter. Synthetic promoters are DNA sequences that do not exist in nature and which are designed to regulate the activity of genes to which they are operably linked, controlling a gene's ability to produce its encoded protein. In a preferred embodiment, the synthetic promoter drives the expression of the antigenic protein early during infection. For instance, in an embodiment, the expression of the antigenic protein can be detected as early as 3-hour post-infection. In another embodiment, the synthetic promoter may also drive the expression of the antigenic protein late during infection. In a preferred embodiment, the expression of the antigenic protein is operably linked and under the control of a synthetic promoter that drives the expression of the antigenic protein both, early and late, during viral infection. The synthetic promoter may be comprised by one or more elements driving early expression of said antigen. The synthetic promoter may be comprised by one or more elements driving late expression of said antigen.

In another embodiment, the promoter is the vaccinia virus synthetic early/late promoter (sE/L), which is a particular type of promoter formed by both early and late elements that are overlapping, and which was firstly defined in Chakrabarti, S.; Sisler, J.R.; Moss, B. Compact, synthetic, vaccinia virus early/late promoter for protein expression. Biotechniques 1997, 23, 1094-1097. Preferably, the nucleic acid comprised in the MVA vector according to the first aspect or any of its embodiments is operably linked and under the control of the vaccinia virus synthetic early/late promoter (sE/L), preferably wherein said synthetic early/late VACV promoter comprises or consists of SEQ ID NO: 16, or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16.

The following vectors are included in the present invention as preferred embodiments:

### MVA-S

In an embodiment, the MVA vector comprises a nucleic acid encoding the S protein of SARS-CoV-2 virus, wherein said S protein comprises an amino acid sequence derived from the wildtype protein of the SARS-CoV-2 Wuhan-Hu-1 (reference Spike protein GenBank accession No QHD43416.1 or Uniprot ID: P0DTC2) and wherein the nucleotide sequence of the S protein expressed by this vector is codon optimized for Vaccinia virus using the codon optimization method of the present invention (from hereinafter, this MVA vector will be referred to as MVA-S).

In a preferred embodiment, the MVA vector according to the first aspect or any of its embodiments comprises at least one nucleic acid encoding a full-length Spike (S) protein derived from SARS-CoV-2 Wuhan-Hu-1 (reference Spike protein GenBank accession No QHD43416.1 or Uniprot ID: P0DTC2), wherein said nucleic acid is further characterized by being codon optimized for Vaccinia virus with the codon optimization method of the present invention and by being inserted in between the open reading frames of the F12L and F13L genes of the MVA genome without substituting, replacing, or interrupting said genes.

In a more preferred embodiment, the nucleotide sequence encoding for the S protein comprised in MVA-S vector derives from SARS-CoV-2 Wuhan-Hu-1 variant (reference Spike protein GenBank accession No QHD43416.1 or Uniprot ID: P0DTC2) and comprises, consists, or consists essentially of SEQ ID NO: 1 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 1. Preferably, the antigenic protein encoded by the nucleotide sequence comprised in MVA-S vector derives from SARS-CoV-2 Wuhan-Hu-1 variant (reference Spike protein GenBank accession No QHD43416.1 or Uniprot ID: P0DTC2) and comprises, consists or consists essentially of SEQ ID NO: 2 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 2.

### MVA-Sdg

In an embodiment, the MVA vector comprises a nucleic acid encoding the S protein of SARS-CoV-2 virus, wherein said S protein comprises an amino acid sequence derived from the wildtype protein of the SARS-CoV-2 Wuhan-Hu-1 (reference Spike protein GenBank accession No QHD43416.1 or Uniprot ID: P0DTC2), wherein said S protein further comprises at least the substitution D614G, and wherein the nucleotide sequence of the S protein expressed by this vector is codon optimized for Vaccinia virus using the codon optimization method of the present invention (from hereinafter, this MVA vector will be referred to as MVA-Sdg).

In a preferred embodiment, the MVA vector according to the first aspect or any of its embodiments comprises at least one nucleic acid encoding a full-length Spike (S) protein derived from SARS-CoV-2 Wuhan-Hu-1 (reference Spike protein GenBank accession No QHD43416.1 or Uniprot ID: P0DTC2), wherein said S protein comprises at least the substitution D614G, and wherein said nucleic acid is further characterized by being codon optimized for Vaccinia virus with the codon optimization method of the present invention and by being inserted in between the open reading frames of the F12L and F13L genes of the MVA genome without substituting, replacing, or interrupting said genes.

In a more preferred embodiment, the nucleotide sequence comprised in MVA-Sdg vector derives from SARS-CoV-2 Wuhan-Hu-1 variant (reference Spike protein GenBank accession No QHD43416.1 or Uniprot ID: P0DTC2) and comprises, consists, or consists essentially of SEQ ID NO: 3 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 3, with the proviso that the amino acid residue in position 614 in the translated sequence of SEQ ID NO: 3 is maintained as a G (D614G mutation). Preferably, the antigenic protein encoded by the nucleic acid comprised in MVA-Sdg vector derives from SARS-CoV-2 Wuhan-Hu-1 variant (reference Spike protein GenBank accession No QHD43416.1 or Uniprot ID: P0DTC2) and comprises, consists or consists essentially of SEQ ID NO: 4 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 4 with the proviso that the amino acid residue in position 614 is maintained in SEQ ID NO: 4 as G (D614G mutation).

### MVA-Spfx

In an embodiment, the MVA vector comprises a nucleic acid encoding the S protein of SARS-CoV-2 virus, wherein said S protein comprises an amino acid sequence derived from the S wildtype protein of the SARS-CoV-2 Wuhan-Hu-1 (reference Spike protein GenBank accession No QHD43416.1 or Uniprot ID: P0DTC2), wherein said S protein further comprises at least the substitutions R682S and R685S to avoid the furin cleavage in combination with the substitutions K986P and V987P to maintain the S protein in a prefusion state, and wherein the nucleotide sequence of the S protein expressed by this vector is codon optimized for Vaccinia virus using a codon optimization method that is different from the codon optimization method of the present invention (from hereinafter, this MVA vector will be referred to as MVA-Spfx).

Preferably, the S protein expressed by MVA-Spfx vector further comprises the substitution D614G.

In a preferred embodiment, the MVA vector according to the first aspect or any of its embodiments comprises at least one nucleic acid encoding a full-length Spike (S) protein derived from SARS-CoV-2 Wuhan-Hu-1 (reference Spike protein GenBank accession No QHD43416.1 or Uniprot ID: P0DTC2), wherein said S protein comprises at least the substitutions D614G, R682S, R685S, K986P and V987P, and wherein said nucleic acid is further characterized by being codon optimized for Vaccinia virus using a codon optimization method that is different from the codon optimization method of the present invention and by being inserted in between the open reading frames of the F12L and F13L genes of the MVA genome without substituting, replacing, or interrupting said genes.

In a more preferred embodiment, said nucleotide sequence comprised in MVA-Spfx vector derives from SARS-CoV-2 Wuhan-Hu-1 variant (reference Spike protein GenBank accession No QHD43416.1 or Uniprot ID: P0DTC2) and comprises, consists, or consists essentially of SEQ ID NO: 6 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 6, with the proviso that the amino acid residues in positions 614, 682, 685, 986 and 987 in the translated sequence of SEQ ID NO: 6 are maintained, respectively, as 614G, 682S, 685S, 986P and 987P (D614G, R682S, R685S, K986P and V987P mutations). Preferably, the antigenic protein encoded by the nucleic acid comprised in MVA-Spfx vector derives from SARS-CoV-2 Wuhan-Hu-1 variant (reference Spike protein GenBank accession No QHD43416.1 or Uniprot ID: P0DTC2) and comprises, consists or consists essentially of SEQ ID NO: 5 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 5, with the proviso that amino acid residues in positions 614, 682, 685, 986 and 987 are maintained in SEQ ID NO: 5 as 614G, 682S, 685S, 986P and 987P, respectively (D614G, R682S, R685S, K986P and V987P mutations).

### MVA-Spf

In an embodiment, the MVA vector comprises a nucleic acid encoding the S protein of SARS-CoV-2 virus, wherein said S protein comprises an amino acid sequence derived from the wildtype protein of the SARS-CoV-2 Wuhan-Hu-1 (reference Spike protein GenBank accession No QHD43416.1 or Uniprot ID: P0DTC2), wherein said S protein further comprises at least the substitutions R682S and R685S to avoid the furin cleavage in combination with the substitutions K986P and V987P to maintain the S protein in a prefusion state, and wherein the nucleotide sequence of the S protein expressed by this vector is codon optimized for Vaccinia virus using the codon optimization method of the present invention (from hereinafter, this MVA vector will be referred to as MVA-Spf). Preferably, the S protein expressed by MVA-Spf vector further comprises the substitution D614G.

Thus, in an embodiment, the MVA vector according to the first aspect or any of its embodiments comprises at least one nucleic acid encoding a full-length Spike (S) protein derived from SARS-CoV-2 Wuhan-Hu-1 (reference Spike protein GenBank accession No QHD43416.1 or Uniprot ID: P0DTC2), wherein said S protein comprises at least the substitutions D614G, R682S, R685S, K986P and V987P, and wherein said nucleic acid is further characterized by being codon optimized for Vaccinia virus using the codon optimization method of the present invention and by being inserted in between the open reading frames of the F12L and F13L genes of the MVA genome without substituting, replacing, or interrupting said genes.

In a more preferred embodiment, the nucleotide sequence comprised in MVA-Spf derives from SARS-CoV-2 Wuhan-Hu-1 variant (reference Spike protein GenBank accession No QHD43416.1 or Uniprot ID: P0DTC2) and comprises, consists, or consists essentially of SEQ ID NO: 7 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 7, with the proviso that the amino acid positions 614, 682, 685, 986 and 987 in the translated sequence of SEQ ID NO: 7 are maintained, respectively, as 614G, 682S, 685S, 986P and 987P (D614G, R682S, R685S, K986P and V987P mutations). Preferably, the antigenic protein encoded by the nucleic acid comprised in the MVA-Spf vector derives from SARS-CoV-2 Wuhan-Hu-1 variant (reference Spike protein GenBank accession No QHD43416.1 or Uniprot ID: P0DTC2) and comprises, consists or consists essentially of SEQ ID NO: 5 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 5, with the proviso that amino acid residues in positions 614, 682, 685, 986 and 987 are maintained in SEQ ID NO: 5 as 614G, 682S, 685S, 986P and 987P, respectively (D614G, R682S, R685S, K986P and V987P mutations).

### MVA-Spf-δ

As stated above, artificial substitutions can be combined with natural occurring substitutions found in the amino acid sequence of the S proteins of the different VOC. In an embodiment, the MVA vector comprises a nucleic acid encoding the S protein of a SARS-CoV-2 virus, wherein said S protein comprises at least the substitutions R682S, R685S, K986P, V987P and the natural substitutions derived from SARS-CoV-2 Delta variant (Linage B.1.617.2 and AY Pango lineages), and wherein the nucleotide sequence of the S protein expressed by this vector is codon optimized for Vaccinia virus using the codon optimization method of the present invention (from hereinafter, this MVA vector will be referred to as MVA-Spf-δ). Preferably, the natural substitutions comprised in the S protein expressed by MVA-Spf- δ are derived from SARS-CoV-2 Delta VOC of 19/Spain/GA-VITHAS-5 1592630/2021 (GISAID database Accession ID: EPI_ISL_1824684).

In a preferred embodiment, the MVA vector according to the first aspect or any of its embodiments comprises at least one nucleic acid encoding a full-length Spike (S) protein derived from SARS-CoV-2 Delta variant or linage (i.e. Linage B.1.617.2 and AY Pango lineages), wherein said S protein comprises at least the substitutions R682S, R685S, K986P and V987P, and wherein said nucleic acid is further characterized by being codon optimized for Vaccinia virus using the codon optimization method of the present invention and by being inserted in between the open reading frames of the F12L and F13L genes of the MVA genome without substituting, replacing, or interrupting said genes.

Preferably, the S protein encoded by the MVA-Spf-δ which derives from the SARS-CoV-2 Delta variant (Linage B.1.617.2 and AY Pango lineages) comprises at least the substitutions R682S, R685S, K986P, V987P, G142D, 156del, R158G, L452R, T478K, D614G, P681R, D950N. More preferably, the S protein encoded by the MVA-Spf-o derives from the SARS-CoV-2 Linage B.1.617+ VOC of 19/Spain/GA-VITHAS-5 1592630/2021 (GISAID database Accession ID: EPI_ISL_1824684) and comprises at least the substitutions R682S, R685S, K986P, V987P, D614G, D950N, E156G, F157del, L452R, P681R, R158del, T19R, T95I, T478K (see Example 2).

In a preferred embodiment, the nucleotide sequence comprised in MVA-Spf-δ vector derives from the SARS-CoV-2 Delta variant (Linage B.1.617.2 and AY Pango lineages) and comprises, consists, or consists essentially of SEQ ID NO: 8 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 8, with the proviso that the amino acid residues in positions 614, 682, 685, 986 and 987 are maintained, respectively, as 614G, 682S, 685S, 986P and 987P, wherein the amino acid numbering is with respect to the Spike protein of the SARS-CoV-2 sequence Wuhan-Hu-1 of GenBank accession No QHD43416.1 or Uniprot ID: P0DTC2, also disclosed as SEQ ID NO: 2, and wherein the positions of the amino acid residues are numbered consecutively starting from the N terminal end of the protein. In a preferred embodiment, the nucleotide sequence comprised in MVA-Spf-δ vector derives from the SARS-CoV-2 Delta variant (Linage B.1.617.2 and AY Pango lineages) and comprises, consists, or consists essentially of SEQ ID NO: 8.

Preferably, the antigenic protein encoded by the nucleic acid comprised in the MVA-Spf-δ vector derives from the SARS-CoV-2 Delta variant (Linage B.1.617.2 and AY Pango lineages) and comprises, consists or consists essentially of SEQ ID NO: 9 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 9, with the proviso that amino acid residues in positions 614, 682, 685, 986 and 987 are maintained as 614G, 682S, 685S, 986P and 987P, respectively (D614G, R682S, R685S, K986P and V987P mutations), wherein the amino acid numbering is with respect to the Spike protein of the SARS-CoV-2 sequence Wuhan-Hu-1 of GenBank accession No QHD43416.1 or Uniprot ID: P0DTC2, also disclosed as SEQ ID NO: 2, and wherein the positions of the amino acid residues are numbered consecutively starting from the N terminal end of the protein. Preferably, the antigenic protein encoded by the nucleic acid comprised in the MVA-Spf-δ vector derives from the SARS-CoV-2 Delta variant (Linage B.1.617.2 and AY Pango lineages) and comprises, consists or consists essentially of SEQ ID NO: 9.

Please note that the amino acid substitutions included in the S protein of the Delta variant are defined herein as D614G, R682S, R685S, K986P and V987P with respect to the numbering of the reference Spike protein of SARS-CoV-2 Wuhan-Hu-1 variant of GenBank accession No QHD43416.1 or Uniprot ID: P0DTC2, SEQ ID NO: 2. However, when adapting the numbering to the Delta variant's sequence, said substitutions correspond to D612G, R680S, R683S, K984P and V985P (see Fig. 20 and SEQ ID NO: 9).

### MVA-Spf-o

In an embodiment, the MVA vector comprises a nucleic acid encoding the S protein of a SARS-CoV-2 virus, wherein said S protein comprises at least the substitutions R682S, R685S, K986P, V987P and the natural substitutions derived from SARS-CoV-2 Omicron variant (i.e. Linage B.1.1.529.2 including BA.1, BA.1.1, BA.2, BA.3, BA.4 and BA.5 Pango Linages, preferably Omicron Linage B.1.1.529.2 BA2); and wherein the nucleotide sequence of the S protein expressed from this vector is codon optimized for Vaccinia virus using the codon optimization method of the present invention (from hereinafter, this MVA vector will be referred to as MVA-Spf-o). Preferably, the natural substitutions comprised in the S protein expressed by MVA-Spf-o are derived from SARS-CoV-2 Omicron VOC of hCoV-19/Spain/GA-CHUVI-21822434/2022 (GISAID database Accession ID: EPI_ISL_12057271).

In a preferred embodiment, the MVA vector according to the first aspect or any of its embodiments comprises at least one nucleic acid encoding a full-length Spike (S) protein derived from SARS-CoV-2 Omicron variant or linage (i.e. Linage B.1.1.529.2 including BA.1, BA.1.1, BA.2, BA.3, BA.4 and BA.5 Pango Linages, preferably Linage B.1.1.529.2 BA2), wherein said S protein comprises at least the substitutions R682S, R685S, K986P and V987P, and wherein said nucleic acid is further characterized by being codon optimized for Vaccinia virus using the codon optimization method of the present invention and by being inserted in between the open reading frames of the F12L and F13L genes of the MVA genome without substituting, replacing, or interrupting said genes.

Preferably, the S protein encoded by the MVA-Spf-o which derives from the SARS-CoV-2 Omicron variant (i.e. Linage B.1.1.529.2 including BA.1, BA.1.1, BA.2, BA.3, BA.4 and BA.5, preferably Linage B.1.1.529.2 BA2), comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, and N969K.

Preferably, the S protein encoded by the MVA-Spf-o which derives from the SARS-CoV-2 Omicron variant (i.e. Linage B.1.1.529.2, preferably BA.1), comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, and N969K, A67V, Del69-70, T95I, Del143-145, Del211, L212I, Ins214EPE, S371L, G446S, G496S, R346K, T547K, N856K, and L981F.

Preferably, the S protein encoded by the MVA-Spf-o which derives from the SARS-CoV-2 Omicron variant (i.e. Linage B.1.1.529.2, preferably BA.2), comprises at least the substitutions R682S, R685S, K986P, V987P, G142D, G339D, S373P, S375F, K417N, N440K, S477N, T478K, E484A, Q493R, Q498R, N501Y, Y505H, D614G, H655Y, N679K, P681H, N764K, D796Y, Q954H, N969K, T19I, Del24-26, A27S, V213G, S371F, T376A, D405N, and R408S.

Most preferably, the S protein encoded by the MVA-Spf-o derives from the SARS-CoV-2 Linage B.1.1.529+ VOC hCoV-19/Spain/GA-CHUVI-21822434/2022 (GISAID database Accession ID: EPI_ISL_12057271) and comprises at least the substitutions R682S, R685S, K986P, V987P, A27S, D405N, D614G, D796Y, E484A, G142D, G339D, H655Y, K417N, L24del, N440K, N501Y, Spike N679K, N764K, N969K, P25del, P26del, P681H, Q493R, Q498R, Q954H, R408S, S371F, S373P, S375F, S477N, T19I, T376A, T478K, V213G, and Y505H (see Example 3).

In a preferred embodiment, the nucleotide sequence comprised in MVA-Spf-o vector derives from the SARS-CoV-2 Omicron variant (i.e. Linage B.1.1.529.2 including BA.1, BA.1.1, BA.2, BA.3, BA.4 and BA.5, preferably Linage B.1.1.529.2 BA2) and comprises, consists, or consists essentially of SEQ ID NO: 27 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 27, with the proviso that the amino acid residues in positions 614, 682, 685, 986 and 987 in the translated sequence of SEQ ID NO: 27 are maintained, respectively, as 614G, 682S, 685S, 986P and 987P (D614G, R682S, R685S, K986P and V987P mutations), wherein the amino acid numbering is with respect to the Spike protein of the SARS-CoV-2 sequence Wuhan-Hu-1 of GenBank accession No QHD43416.1 or Uniprot ID: P0DTC2, also disclosed as SEQ ID NO: 2, and wherein the positions of the amino acid residues are numbered consecutively starting from the N terminal end of the protein. Preferably, the nucleotide sequence comprised in MVA-Spf-o vector derives from the SARS-CoV-2 Omicron variant (i.e. Linage B.1.1.529.2 including BA.1, BA.1.1, BA.2, BA.3, BA.4 and BA.5, preferably Linage B.1.1.529.2 BA2) and comprises, consists, or consists essentially of SEQ ID NO: 27.

Preferably, the antigenic protein encoded by the nucleic acid comprised in the MVA-Spf-o vector derives from the SARS-CoV-2 Omicron variant (i.e. Linage B.1.1.529.2 including BA.1, BA.1.1, BA.2, BA.3, BA.4 and BA.5, preferably Linage B.1.1.529.2 BA2) and comprises, consists or consists essentially of SEQ ID NO: 28 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 28, with the proviso that amino acid residues in positions 614, 682, 685, 986 and 987 are maintained as 614G, 682S, 685S, 986P and 987P, respectively, wherein the amino acid numbering is with respect to the Spike protein of the SARS-CoV-2 sequence Wuhan-Hu-1 of GenBank accession No QHD43416.1 or Uniprot ID: P0DTC2, also disclosed as SEQ ID NO: 2, and wherein the positions of the amino acid residues are numbered consecutively starting from the N terminal end of the protein. Preferably, the antigenic protein encoded by the nucleic acid comprised in the MVA-Spf-o vector derives from the SARS-CoV-2 Omicron variant (i.e. Linage B.1.1.529.2 including BA.1, BA.1.1, BA.2, BA.3, BA.4 and BA.5, preferably Linage B.1.1.529.2 BA2) and comprises, consists or consists essentially of SEQ ID NO: 28.

Please note that the amino acid substitutions included in the S protein of the Omicron variant are defined herein as D614G, R682S, R685S, K986P and V987P with respect to the numbering of the reference Spike protein of SARS-CoV-2 Wuhan-Hu-1 variant (GenBank accession No QHD43416.1 or Uniprot ID: P0DTC2, SEQ ID NO: 2). However, when adapting the numbering to the Omicron variant's sequence, said substitutions correspond to D611G, R679S, R682S, K983P and V984P (see Fig. 20 and SEQ ID NO: 28).

As stated above, unless otherwise indicated, the amino acid numbering used here and throughout the whole document is with respect to the Spike protein of the SARS-CoV-2 sequence Wuhan-Hu-1 (GenBank accession No QHD43416.1 or Uniprot ID: P0DTC2, also disclosed as SEQ ID NO: 2), wherein the positions of the amino acid residues are numbered consecutively starting from the N terminal end of the protein.

In a preferred embodiment, the at least one nucleic acid encoding for the S protein comprised in the MVA vectors MVA-S, MVA-Sdg, MVA-Spfx, MVA-Spf, MVA-Spf-δ, MVA-Spf-o according to the first aspect or any of its embodiments is operably linked and under the control of an early-late promoter from Vaccinia virus, preferably the synthetic VACV early-late promoter, preferably the synthetic VACV early-late promoter comprising or consisting of SEQ ID NO: 16.

In a preferred embodiment, the at least one nucleic acid encoding for the S protein comprised in the MVA vectors MVA-S, MVA-Sdg, MVA-Spf, MVA-Spf-δ and MVA-Spf-o according to the first aspect or any of its embodiments is inserted in between the open reading frames of the F12L and F13L genes of the MVA genome without substituting, replacing, or interrupting said genes; is codon optimized for Vaccinia virus following the codon optimization method of the present invention; and is operably linked and under the control of an early-late promoter from Vaccinia Virus, preferably the synthetic VACV early-late promoter, preferably the synthetic VACV early-late promoter of SEQ ID NO: 16.

In a preferred embodiment, the MVA vector provided in the first aspect or the present invention or in any of its embodiments comprises a nucleic acid that in turn comprises, from 5' to 3', at least the following sequences:
i) the synthetic early/late promoter comprising or consisting of SEQ ID NO: 16, or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16,
ii) at least one nucleic acid encoding the S protein, or a fragment of said S protein comprising at least one epitope, of at least one SARS-CoV-2 variant or linage, wherein said S protein or fragment comprises at least the substitutions D614G, R682S, R685S, K986P and V987P (numbering according to the amino acid sequence of the Spike protein of the SARS-CoV-2 Wuhan-Hu-1 GenBank accession No QHD43416.1 or Uniprot ID: P0DTC2, taken as reference), wherein said nucleic acid is codon optimized for Vaccinia virus with the codon optimization method of the present invention,
wherein the nucleic acid encoding for the S protein or the fragment thereof as defined in ii) is operably linked to the synthetic early/late promoter as defined in i), wherein said nucleic acid is inserted in between the open reading frames of the F12L and F13L genes of the MVA genome without substituting, replacing, or interrupting said genes, and wherein the MVA vector regulates the expression of the nucleic acid encoding the S protein, or the fragment thereof.

In a preferred embodiment, the MVA vector provided in the first aspect or the present invention or in any of its embodiments comprises a nucleic acid that in turn comprises, from 5' to 3', at least the following sequences:
i) the synthetic early/late promoter comprising or consisting of SEQ ID NO: 16, or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16,
ii) at least one nucleic acid encoding the S protein, comprising or consisting of any of SEQ ID NO: 1, 3, 7, 8 or 27, or a sequence with at least 50%, 60%, 70%, 80%, 85%, 90%, 91%, 92%, 93%, 94%, 95%, 96%, 97%, 98%, 99% or 100% sequence identity over the full length with any of SEQ ID NO: 1, 3, 7, 8 or 27,
wherein the nucleic acid encoding for the S protein as defined in ii) is operably linked to the synthetic early/late promoter as defined in i), wherein said nucleic acid is inserted in between the open reading frames of the F12L and F13L genes of the MVA genome without substituting, replacing, or interrupting said genes; and wherein the MVA vector regulates the expression of the nucleic acid encoding the S protein.

In a preferred embodiment, the MVA vector provided in the first aspect or the present invention or in any of its embodiments comprises a nucleic acid comprising or consisting of SEQ ID NO: 7, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

In a preferred embodiment, the MVA vector provided in the first aspect or the present invention or in any of its embodiments comprises a nucleic acid comprising or consisting of SEQ ID NO: 8, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

In a preferred embodiment, the MVA vector provided in the first aspect or the present invention or in any of its embodiments comprises a nucleic acid comprising or consisting of SEQ ID NO: 27, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

In a preferred embodiment, the MVA vector provided in the first aspect or the present invention or in any of its embodiments comprises a nucleic acid that in turn comprises, from 5' to 3', at least the following sequences:
i) the synthetic early/late promoter comprising or consisting of SEQ ID NO: 16, or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 16,
ii) at least one nucleic acid encoding the S protein, wherein said S protein has an amino acid sequence that comprises or consists of any of SEQ ID NO: 2, 4, 5, 9, 28, or a sequence with at least 50%, 60%, 70%, 80%, 85%, 90%, 91%, 92%, 93%, 94%, 95%, 96%, 97%, 98%, 99% or 100% sequence identity over the full length with any of SEQ ID NO: 2, 4, 5, 9, 28,
wherein the nucleic acid encoding for the S protein as defined in ii) is operably linked to the synthetic early/late promoter as defined in i), wherein said nucleic acid is inserted in between the open reading frames of the F12L and F13L genes of the MVA genome without substituting, replacing, or interrupting said genes; and wherein the MVA vector regulates the expression of the nucleic acid encoding the S protein.

In a preferred embodiment, the MVA vector provided in the first aspect or the present invention or in any of its embodiments comprises a nucleic acid encoding for the S protein whose amino acid sequence comprises or consists of SEQ ID NO: 5, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

In a preferred embodiment, the MVA vector provided in the first aspect or the present invention or in any of its embodiments comprises a nucleic acid encoding for the S protein whose amino acid sequence comprises or consists of SEQ ID NO: 9, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

In a preferred embodiment, the MVA vector provided in the first aspect or the present invention or in any of its embodiments comprises a nucleic acid encoding for the S protein whose amino acid sequence comprises or consists of SEQ ID NO: 28, wherein said nucleic acid is inserted in between the open reading frames of the F12L and F13L genes of the MVA genome without substituting, replacing, or interrupting said genes, and is operably linked to the synthetic early/late Vaccinia virus promoter comprising or consisting of SEQ ID NO: 16.

It is noted that the recombinant MVA viruses described herein are highly replication restricted and, thus, highly attenuated, and thus they are ideal candidates for the treatment of a wide range of mammals including humans and even immune-compromised humans. Hence, provided herein are MVA vectors and pharmaceutical compositions and vaccines, as described above in the first aspect of the invention, suitable for inducing an immune response in a living animal body, including a human against the SARS-CoV-2 virus.

The vaccines compositions of the first aspect preferably comprise the MVA vector described herein formulated in solution in a concentration range of from 1 × 107 to 1 × 10⁸ PFUs per dosis.

The vaccine compositions comprising the MVA vector of the first aspect may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

For the preparation of vaccine compositions, the recombinant MVA viruses provided herein can be converted into a physiologically acceptable form. This can be done based on experience in the preparation of poxvirus vaccines used for vaccination against smallpox as described elsewhere.

For example, purified viruses can be stored at -80°C with a titer of 5×10⁸ a PFU/ml formulated in about 10 mM Tris, 140 mM NaCl pH 7.4. For the preparation of vaccine shots, e.g., 10²-10⁸ or 10²-10⁹ particles of the virus can be lyophilized in 100 ml of phosphate-buffered saline (PBS) in the presence of 2% peptone and 1% human albumin in an ampoule, preferably a glass ampoule. Alternatively, the vaccine shots can be produced by stepwise freeze-drying of the virus in a formulation. This formulation can contain additional additives such as mannitol, dextran, sugar, glycine, lactose or polyvinylpyrrolidone or other aids such as antioxidants or inert gas, stabilizers or recombinant proteins (e.g., human serum albumin) suitable for in vivo administration. The glass ampoule is then sealed and can be stored between 45°C and room temperature for several months. However, as long as no need exists, the ampoule is stored preferably at temperatures below -20°C.

For vaccination or therapy, the lyophilizate can be dissolved in an aqueous solution, preferably physiological saline or Tris buffer, and administered either systemically or locally, i.e., parenteral, subcutaneous, intravenous, intramuscular, intranasal, or any other path of administration known to the skilled practitioner. The mode of administration, the dose and the number of administrations can be optimized by those skilled in the art in a known manner. However, most commonly a patient is vaccinated with a second shot about one month to six weeks after the first vaccination shot, although booster doses can also be administered years after the first dose.

In addition, for working the present invention any avirulent MVA vector suitable for human vaccination could be used. "Derivatives" or "variants" of MVA are also suitable for working the present invention.

In the context of the present invention, "derivatives" or "variants" of MVA refer to viruses exhibiting essentially the same replication characteristics as MVA but exhibiting differences in one or more parts of their genomes. These "derivatives" or "variants" of MVA must fail to reproductively replicate in vivo in humans and mice, even in severely immune suppressed mice. Tests and assays for the properties of MVA variants are described in WO 02/42480 (US 2003/0206926) and WO 03/048184 (US 2006/0159699).

The term "fails to reproductively replicate" refers to a virus that has a virus amplification ratio at 4 days after infection of less than 1. Assays described in WO 02/42480 or in U.S. Patent No. 6,761 ,893 are applicable for the determination of the virus amplification ratio.

The amplification or replication of a virus is normally expressed as the ratio of virus produced from an infected cell (output) to the amount originally used to infect the cell in the first place (input) referred to as the "amplification ratio". An amplification ratio greater than "1" defines an amplification status where the amount of virus produced from the infected cells is at least the same as the amount initially used to infect the cells, meaning that the infected cells are permissive for virus infection and reproduction. In contrast, an amplification ratio of less than 1, i.e., a decrease in output compared to the input level, indicates a lack of reproductive replication and therefore attenuation of the virus.

The advantages of MVA-based vaccine include their safety profile as well as availability for large scale vaccine production. In a preferred embodiment, the recombinant MVA vector of any of the embodiments used for generating the recombinant virus is a MVA virus or a derivative having the capability of reproductive replication in vitro in chicken embryo fibroblasts (CEF) cells and in established chick cells DF-1, but no capability of reproductive replication in many mammalian cells, including human cells such as the human cervix adenocarcinoma cell line HeLa.

MVA vectors useful for the present invention can be prepared using methods known in the art, such as those described in WO 02/042480 and WO 02/24224, both of which are incorporated by reference herein.

In another aspect, a MVA viral strain suitable for generating the recombinant viruses of the present invention may be strain MVA-572, MVA-575 or any similarly attenuated MVA strain. Also suitable may be a mutant MVA, such as the deleted chorioallantoic vaccinia virus Ankara (dCVA). A dCVA comprises del I, del II, del III, del IV, del V, and del VI deletion sites of the MVA genome. The sites are particularly useful for the insertion of multiple heterologous sequences. The dCVA can reproductively replicate (with an amplification ratio of greater than 10) in a human cell line (such as human 293, 143B, and MRC-5 cell lines), which then enable the optimization by further mutation useful for a virus-based vaccination strategy (see WO 201 1/092029).

### Combination Vaccines Using Homologous/Heterologous Prime-Boost Regimens

The MVA vectors, vaccine compositions and methods described herein may be used as part of a homologous prime-boost regimen. In the homologous prime-boost, a first priming vaccination is given followed by one or more subsequent boosting vaccinations. The boosting vaccinations are configured to boost the immune response generated in the first vaccination by administration of the same recombinant MVA that was used in the first vaccination.

In one exemplary embodiment a homologous prime-boost regimen may be employed wherein a MVA viral vector as defined herein in the first aspect of the invention or in any of its embodiments is administered in a first dosage. One or more subsequent administrations of a MVA viral vector as defined herein in the first aspect or any of its embodiments can be given to boost the immune response provided in the first administration. Preferably, the one or more antigenic determinants are the same or similar to those of the first administration. In a preferred embodiment, the MVA vectors provided herein are administered as a boost (i.e., as a second, third, fourth, or successive vaccinations).

The MVA recombinant viral vectors according to the present invention may also be used in prime-boost regimens in combination with other immunogens (heterologous prime/boost combination), such as in combination with a DNA or RNA vector or another viral vector, such as an adenoviruses. In this case, one or more subsequent boosting vaccinations may be done with the MVA vectors provided herein and not used in the prime vaccination, e.g., if another immunogenic vector is given in a prime, then subsequent boosting vaccinations may be performed with the MVA provided herein, and vice versa.

Thus, a **second aspect** of the invention refers to a vaccine combination (from hereinafter referred to as homologous vaccine combination) comprising:
a. a composition comprising an immunologically effective amount of a MVA vector as defined in the first aspect or any of its embodiments; and
b. a composition comprising an immunologically effective amount of a MVA vector as defined in the first aspect or any of its embodiments;
wherein one of the compositions is a priming composition and the other composition is a boosting composition, preferably wherein the boosting composition comprises or consists of two or more doses of the vector of the boosting composition.

Furthermore, in an alternative second aspect of the invention refers to a vaccine combination (from hereinafter referred to as heterologous vaccine combination) comprising:
a. a composition comprising an immunologically effective amount of a MVA vector as defined in the first aspect or any of its embodiments; and
b. a composition comprising an immunologically effective amount of at least one immunogen of at least one SARS-CoV-2 variant, preferably selected from the group consisting of viral vectors, recombinant proteins or polypeptides, nucleic acids (including DNA and RNA), virus-like particles, nanoparticles, or any combination thereof,
wherein one of the compositions is a priming composition and the other composition is a boosting composition, in any order. Preferably, the composition comprising the MVA vector as defined in the first aspect or any of its embodiments is administered as a boost, after a prime with a composition comprising an immunologically effective amount of an immunogen of at least one SARS-CoV-2 variant. Preferably, the boost composition comprises or consists of two or more doses of the vector or immunogen of the boosting composition. Preferably, the vaccine of the invention is administered as a boost, preferably as a third, fourth, fifth, or sixth boost.

It is noted that the recombinant heterologous or homologous vaccine combinations may be either monovalent, i.e., comprising only one heterologous antigenic determinant of SARS-CoV-2, or multivalent, i.e., comprising at least two heterologous antigenic determinants of SARS-CoV-2, such as those selected from the list consisting of structural proteins S, E or M of SARS-CoV-2.

In one preferred embodiment of the second aspect of the invention, the immunologically effective amount of an immunogen of at least one SARS-CoV-2 variant or linage comprises an antigenic protein or nucleic acid encoding for an antigenic protein, wherein the antigenic protein is selected from any of the group of structural proteins S, E, N or M of SARS-CoV-2, or any combination or fragments thereof. In an embodiment, the at least one immunogen of at least one SARS-CoV-2 variant or linage is a nucleotide sequence of a DNA or RNA vector that encodes for an antigenic protein of at least one SARS-CoV-2 variant. In an embodiment, said nucleotide sequence encoding for an antigenic protein is preferably the structural protein S of SARS-CoV-2, or a fragment thereof such as the RBD.

In a preferred embodiment of the heterologous vaccine combination, the other immunogen that is combined with the MVA vector provided herein is a RNA molecule. In a preferred embodiment of the heterologous vaccine combination, the other immunogen that is combined with the MVA vector provided herein is an adenovirus. In a preferred embodiment of the heterologous vaccine combination, the other immunogen that is combined with the MVA vector provided herein is a recombinant protein or polypeptide.

### Vaccines and Kits Comprising Recombinant MVA Viruses

Also provided herein are vaccines and kits comprising any one or more of the recombinant MVAs described herein. The kit can comprise one or multiple containers or vials of the recombinant MVA, together with instructions for the administration of the recombinant MVA to a subject at risk of SARS-CoV-2 infection. In certain embodiments, the subject is a human. In certain embodiments, the instructions indicate that the recombinant MVA is administered to the subject in a single dose, or in multiple (i.e., 2, 3, 4, etc.) doses. In certain embodiments, the instructions indicate that the recombinant MVA virus and/or composition is administered in a single dose to naive or non-naive subjects. Thus, in a preferred embodiment, the vaccines and kits comprising recombinant MVA vectors and/or vaccine compositions and/or combinations as described in the first or second aspects or any of their embodiments comprise a single dose to be administrated to a subject in need thereof. In certain embodiments, the instructions indicate that the recombinant MVA vectors and/or vaccine compositions and/or combinations is administered in a first (priming) and second (boosting) administration to naive or non-naive subjects. In certain embodiments, the instructions indicate that the recombinant MVA vectors and/or vaccine compositions and/or combinations is administered as a boost to naive or non-naive subjects. Preferably, a kit comprises at least two vials or container for prime/boost immunization comprising the recombinant MVA vectors and/or vaccine compositions and/or combinations as described herein for a first inoculation ("priming inoculation") in a first vial/container and for an at least second and/or third and/or further inoculation ("boosting inoculation") in a second and/or further vial/container. More preferably, the vaccines and kits and compositions described herein comprise a first (priming) and second (boosting) dose of an immunologically effective amount of the recombinant MVAs as described herein, for use in homologous or heterologous prime boost inoculations.

Thus, a **third aspect** of the invention refers to kits comprising one, two, or more doses of any of the vaccine compositions comprising the MVA vector as defined in the first aspect of the invention or any of its embodiments, or of any of the homologous or heterologous vaccine combinations of the second aspect of the invention or any of its embodiments. Preferably, the kit comprises an immunologically effective amount of a MVA-based vaccine composition, preferably MVAs vectors according to the first aspect of the invention in a first vial or container for a first administration (priming) and in a second vial or container for a second administration (boosting).

A preferred embodiment of the third aspect of the invention refers to a kit comprising an immunologically effective amount of a MVA vector according to the first aspect or any of its embodiment in a container for a first or a second or subsequent administrations, wherein the kit further comprises at least another container comprising an immunologically effective amount of another immunogen of at least one SARS-CoV-2 variant or linage for a first or a second or subsequent administrations. A preferred embodiment refers to kits comprising an immunologically effective amount of at least one immunogen of at least one SARS-CoV-2 variant or linage in a first vial or container for a first administration (priming), and an immunologically effective amount of a MVA vector according to the first aspect or any of its embodiments, in a second vial or container for a second administration (boosting).

In another preferred embodiment of the third aspect of the invention, any of the kits referred to herein, comprise a third, fourth or further vial or container comprising any of the MVA and/or vaccine compositions and/or compositions indicated throughout the present invention for a third, fourth or further administration. Preferably, the MVA vectors and/or vaccine compositions and/or combinations provided herein is used in a second, third, fourth, or further administrations.

In a preferred embodiment of the third aspect, the MVA vectors and/or the compositions present in the containers are capable of producing at least the Spike (S) protein, or a fragment thereof, comprising at least the substitutions R682S, R685S, K986P and V987P derived from at least one SARS-CoV-2 variant or linage, in the subject to be treated, and generating an immunogenic and/or protective immune response against at least one SARS-CoV-2 variant or linage in a subject in need thereof.

In a **fourth aspect** of the present invention provides the vaccines compositions and/or combinations and/or kits provided in any of the previous aspects are **for use** in generating an immunogenic and/or protective immune response against at least one SARS-CoV-2 variant or linage, wherein vaccines compositions and/or combinations and/or kits comprise one dose of an immunologically effective amount of the MVA provided herein. Preferably, the vaccines compositions, combinations and/or kits provided in any of the previous aspects are for use in generating an immunogenic and/or a protective immune response against at least one SARS-CoV-2 variant or linage, wherein the vaccines compositions, combinations and/or kits comprise at least one dose, preferably at least two doses, of an immunologically effective amount of the vectors MVA defined in the first aspect or in any of its embodiments. Preferably, only one dose of the MVA vectors and/or vaccine compositions as defined herein is sufficient to produce immunological protection upon one, preferably more than one, SARS-CoV-2 challenges. Preferably, the one dose of the MVA vectors and/or vaccine compositions as defined herein is administrated as a prime. Preferably, the one dose of the MVA vectors and/or vaccine compositions as defined herein is administrated as a boost.

As used herein, the term "immunogenic and protective immune response, "protective immunity" or "protective immune response" means that the vaccinated subject is able to control an infection with the pathogenic agent against which the vaccination was done or any of their variants or linages. Usually, the subject having developed a "protective immune response" develops only mild to moderate clinical symptoms or no symptoms at all. Usually, a subject having a "protective immune response" or "protective immunity" against a certain agent will not die as a result of the infection with said agent. In certain embodiments, the subject animal is a mammal. The mammal may be an adult cow, a calf, in particular a juvenile calf, a rat, rabbit, pig, mouse, preferably a human or a cat, and the method comprises administering at least a dose of the recombinant MVA or compositions or combinations thereof as described in the fourth aspect of the invention.

In certain embodiments, the subject is a domestic mammal, preferably a cat. In certain embodiments, the cat is over the age of 1, 2, 3, 4, 5, 6, 7, 8 or more than 8 years.

In certain embodiments, the subject is a human. In certain embodiments, the subject is an adult. In certain embodiments, the adult is immune-compromised. In certain embodiments, the adult is over the age of 10, 15, 20, 25, 30, 25, 40, 45, 50, 55, 60, 65, 70, 75, 80, or 85 years.

In a preferred embodiment, the MVAs and/or vaccine composition and/or combinations according to the first and second aspect or any of its embodiment and/or the kits of the third aspect or any of its embodiments is/are for use in generating an immunogenic and/or protective immune response in a subject in need thereof, preferably in a human subject, wherein said immune response is against at least the S protein, or a fragment of said S protein, comprising at least the substitutions R682S, R685S, K986P and V987P and derived from at least one SARS-CoV-2 variant or linage, wherein said MVA and/or vaccine composition and/or combinations and/or kits are used for priming and/or for boosting said immune response, and wherein the MVA is capable of producing said S protein, or fragment thereof, in the subject to be treated.

In a further preferred embodiment the vaccine combinations and/or kits provided in any of the previous aspects are for use in generating a protective immune response against at least one SARS-CoV-2 variant or linage, wherein if the vaccine combinations and/or kits are used, the first composition is used for priming said immune response and the second composition is used for boosting said immune response or for use in generating a protective immune response against at least one SARS-CoV-2 variant or linage, wherein the second composition is used for priming said immune response and the first composition is used for boosting said immune response. In any of the vaccine combinations and kits provided herein the boosting composition can comprise two or more doses of the vector of the boosting composition.

Thus, in a preferred embodiment, the vaccine combination according to the second aspect or any of its embodiments is for use in generating an immunogenic and/or protective immune response in a subject in need thereof, preferably in a human subject, wherein said immune response is against at least the S protein, or a fragment of said S protein, comprising at least the substitutions R682S, R685S, K986P and V987P and derived from at least one SARS-CoV-2 variant or linage, and wherein said combination is capable of producing said S protein, or fragment thereof, in the subject to be treated.

In a preferred embodiment, the protective immune response generated with the MVAs described herein is able to protect a vaccinated subject against at least one, two, three, four, five, six, seven, eight, nine, ten, or more than ten SARS-CoV-2 infections, wherein preferably said protective immune response is generated after at least one dose of said MVA, preferably as a prime or as a boost. In a preferred embodiment, said protective immune response is able to protect the vaccinated subject against an infection caused by any SARS-CoV-2 variant or linage, preferably selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617.1 (Kappa variant), Linage B.1.1.7 (United Kingdom variant), Linage B.1.617.2 (Delta variant), Linage B.1.1.529 (Omicron variant), or any combination thereof. Thus, in an embodiment, said protective immune response provides cross-protection. Preferably, said cross-protection is achieved after one dose of the MVA or of the vaccine composition of the present invention.

As stated above, the present invention provides heterologous vaccine programs that consists of a combination vaccine and/or vaccination kit which comprises:
a. a composition comprising an immunologically effective amount of a MVA vector as defined in the first aspect or any of its embodiments, and
b. a composition comprising an immunologically effective amount of at least one immunogen of at least one SARS-CoV-2 variant,
wherein one of the compositions is a priming composition and the other composition is a boosting composition, in any order, and preferably wherein the boosting composition comprises two or more doses. Preferably, the MVA vector as defined in the first aspect or any of its embodiments is the boosting composition. Preferably, the MVA vector as defined in the first aspect or any of its embodiments is the priming composition.

The present invention also provides homologous vaccination regimes using two equal non-replicating viral vectors. For homologous vaccine programs, the present invention thus provides a combination vaccine and/or vaccination kit which comprises:
a. a composition comprising an immunologically effective amount of a MVA vector as defined in the first aspect or any of its embodiments, and
b. a composition comprising an immunologically effective amount of a MVA vector as defined in the first aspect or any of its embodiments,
wherein one of the compositions is a priming composition and the other composition is a boosting composition, preferably wherein the boosting composition comprises two or more doses.

In this embodiment, the combination vaccines and/or kit comprises at least two vials or containers for prime/boost immunization comprising the recombinant MVA according to the first aspect or any of its embodiments for a first inoculation ("priming inoculation") in a first vial or container and for an at least second and/or third and/or further inoculation ("boosting inoculation") in a second and/or further vial or container.

The heterologous vaccine combination and/or kit can comprise multiple containers or vials of the recombinant MVA/other immunogen, together with instructions for the administration of the recombinant MVA/other immunogen to a subject at risk of SARS-CoV-2 infection. In certain embodiments, the subject is a human. In certain embodiments, the instructions indicate that the recombinant MVA/other immunogen is administered to the subject in a single dose, or in multiple (i.e., 2, 3, 4, etc.) doses. In certain embodiments, the instructions indicate that the recombinant MVA/other immunogen virus is administered in a first (priming) and second (boosting) administration to naive or non-naive subjects. As indicated above, the other immunogen of at least one SARS-CoV-2 variant or linage is selected from the group including, but not limited to, viral vectors, recombinant proteins or polypeptides, nucleic acids, virus-like particles, nanoparticles, or any combination thereof. Preferably, said immunogen is selected from the group consisting of viral vectors, recombinant proteins or polypeptides, nucleic acids, virus-like particles, nanoparticles, or any combination thereof.

The first and/or second composition and/or other immunogen of any combination vaccine, vaccination kit and/or any homologous or heterologous vaccine program of the invention can comprise any of the MVA vector described herein and any combination thereof.

### Methods and Uses of Recombinant MVA Viruses

Also provided herein are methods of use the MVA vector and/or vaccine compositions and/or combinations and/or kits as described in the previous aspects of the invention or any of its embodiments, for immunizing a subject animal or for affecting an immune response in a subject against SARS-CoV-2 infection. Also covered are uses of the said recombinant MVA vector and/or vaccine compositions and/or combinations and/or kits described herein for the preparation of a medicament or pharmaceutical for the immunization of a subject animal, in particular for the preparation of a medicament or vaccine for treating and/or preventing a SARS-CoV-2-caused disease in a subject. Provided are also said MVA vector and/or vaccine compositions and/or combinations and/or kits according to any embodiment described herein for use in priming or boosting an immune response against a SARS-CoV-2 infection, preferably wherein the recombinant MVA is administered once, twice, three times or four times.

Further covered herein are vaccine combinations of any of the embodiments for use as a medicament or vaccine for inducing an enhanced immune response against a SARS-CoV-2 infection wherein the combination is capable of producing the antigenic determinant, and/or SARS-CoV-2 -like particles in the subject to be treated, preferably, wherein the combination vaccine is producing the encoded antigenic determinant in the subject to be treated.

Accordingly, also in the **fourth aspect** of the present invention it is provided a method of generating an immunogenic and/or protective immune response against at least one SARS-CoV-2 variant or linage in a subject in need thereof, preferably in a human subject, the method comprising administering to the subject the recombinant MVA vector and/or vaccine compositions as described in the first aspect of the invention or any of its embodiments; or a kit or any of the vaccine combinations, including the homologous or heterologous vaccination regimes, as described in any of the second or third aspects of the invention or any of their embodiments. In an embodiment, said method is for priming the immune response and/or for boosting said immune response.

The recombinant MVA and/or compositions provided in the first aspect of the invention, or any of the kits or vaccine combinations, including the homologous or heterologous vaccination regimes, as described in any of the second or third aspects of the invention, may be preferably administered to the subject at a dose of 1×10^7 or 1×10^8 PFU/dose.

In certain embodiments, any of the recombinant MVA and/or compositions provided in the first aspect of the invention, or any of the kits or vaccine combinations, including the homologous or heterologous vaccination regimes, as described in any of the second or third aspects of the invention, are administered to the subject at any of the doses provided herein prior to SARS-CoV-2 virus exposure as, e.g., 1 , 2, 3, or 4 weeks or 1, 2, 3, 4, 5, 6, 7, 8, 9, 10, 11, or 12 months before SARS-CoV-2 virus exposure. In certain embodiments, any of the said MVA and/or vaccine compositions and/or combinations and/or kits provided herein is administered to the subject at any of the doses provided herein after SARS-CoV-2 virus exposure as, e.g., 1, 2, 3, 4, 5, 6, 7, 8, 9. 10, 11, 12, 13, 14, 15, 16, 17, 18, 19, 20, 21, 22, 23, or 24 hours or 1, 2, 3, 4, 5, 6, or 7 days after SARS-CoV-2 exposure.

In certain embodiments, the recombinant MVA and/or composition provided in the first aspect of the invention, or any of the kits or vaccine combinations, including the homologous or heterologous vaccination regimes, as described in any of the second or third aspects of the invention, are administered to the subject in a single dose, or in multiple (i.e., 2, 3, 4, etc.) doses. In certain embodiments, the said kits or MVA and/or vaccine compositions and/or combinations are administered in a first (priming) and/or second (boosting) administration.

Boosting MVA and/or vaccine compositions are generally administered only once or multiple times weeks or months after administration of the priming MVA and/or vaccine composition, for example, about 1 or 2 weeks or 3 weeks, or 4 weeks, or 6 weeks, or 8 weeks, or 16 weeks, or 20 weeks, or 24 weeks, or 28 weeks, or 32 weeks or one to two years. Preferably, the initial boosting inoculation is administered 1-12 weeks or 2-12 weeks after priming, more preferably 1, 2, 3, 4 or 8 weeks after priming. In a preferred embodiment, the initial boosting inoculation is administered 4 or 8 weeks after priming. In additional preferred embodiments, the initial boosting is conducted at least 2 weeks or at least 4 weeks after priming. In still another preferred embodiment, the initial boosting is conducted 4-12 weeks or 4-8 weeks after priming.

The recombinant MVA and/or compositions provided in the first aspect of the invention, or any of the kits or vaccine combinations, including the homologous or heterologous vaccination regimes, as described in any of the second or third aspects of the invention, or in any of their embodiments, can be administered systemically or locally. In certain embodiments, the said recombinant MVAs and/or vaccine compositions and/or combinations are administered parenterally, subcutaneously, intravenously, intramuscularly, or intranasally, in particular intranasally or intramuscularly. Preferably, the recombinant MVA or the containers comprising the recombinant MVA are administered intranasally. In other embodiments, the said recombinant MVAs and/or vaccine compositions and/or combinations are administered by any other path of administration known to the skilled practitioner. In a further preferred embodiment, the said recombinant MVAs and/or vaccine composition and/or combination is administered intramuscularly, preferably the recombinant MVAs and/or vaccine composition and/or combination is administered intramuscularly in a volume ranging between about 0.10 and 1.0 ml, preferably containing concentrations of e.g., about 10⁷ to 10^9 virus particles/ml. Preferably, the said recombinant MVAs and/or vaccine composition and/or combinations is administered in a volume ranging between 0.25 and 1.0 ml. More preferably, the said MVAs and/or vaccine composition and/or combinations is administered in a volume of about 0.5 ml.

Other embodiments of the invention will be apparent to those skilled in the art from consideration of the specification and practice of the invention disclosed herein. It is intended that the specification and examples be considered as exemplary only, with a true scope and spirit of the invention being indicated by the appended claims.

Lastly, it is also found herein that a particular method for codon optimization, called the codon optimization method of the present invention, resulted in increased expression of a codon-optimized GFP protein versus the non-optimized counterpart (see Fig 2). The increased expression due to the codon optimization method of the present invention was also observed in the expression levels of the S protein encoded by MVA-Spf, in comparison to the S protein expressed by MVA-Spfx, which was codon optimized following a different optimization method (see Fig. 4). These results of increased expression are relevant in the context of a vaccine, asi t is known that the ability of a vector to express very efficiently a heterologous antigen, such as the S protein of SARS-CoV-2 virus.

In view of this, in a **fifth aspect,** the present invention provides the codon optimization method of the invention, the method comprising or consisting the step of i) choosing for every amino acid of the protein to be codon optimized, the most used synonymous codon according to the codon usage table from the organism of interest, and ii) reviewing the resulting nucleotide sequence of step i) so that, if an early transcription termination signal has been generated as a result of step i), said early transcription termination sequence is substituted for a different nucleotide sequence considering the following conditions:
- that the amino acid sequence and the open reading frame of the translated protein is maintained (i.e., it is not altered), and
- that the early transcription termination signal sequence is modified so that the RNA polymerase does not recognize it as such, thereby avoiding early termination of the transcription.

By "choosing the most used synonymous codon" is meant to choose the codon that, according to the codon usage table, has the highest value of frequency per thousand among those coding for that particular amino acid. In a preferred embodiment, the codon usage Table 2 for Vaccinia virus is used to determine the most used synonymous codon. In some embodiments, a method of the fifth aspect or the codon optimization method of the invention may be used to design a synthetic nucleic acid sequence that encodes a heterologous or endogenous polypeptide of interest. In some of these embodiments, the synthetic nucleic acid sequence may be optimized for expression in a host organism, for example, by codon-optimization to reflect the codon usage of the expression host. In some other embodiments, the synthetic nucleic acid sequence may not be optimized for expression in a host organism but optimized for expression according to the vector carrying said nucleotide. Optionally, the method further comprises a step of removing the early transcription termination signal (TTTTTNT) from the sequence.

In an embodiment, the method is a computer-implemented method.

In an **eight aspect,** the present invention relates to a modified vaccinia virus Ankara (MVA) vector, wherein the MVA vector comprises at least one nucleic acid encoding the Spike (S) protein of at least one SARS-CoV-2 variant or linage, wherein said nucleic acid has been obtained by following the codon optimization method of the present invention.

### SEQUENCE LISTING

**SEQ ID NO: 1** Nucleic acid encoding for the S protein from MVA-S 
ATGTTTGTATTTTTAGTATTATTACCATTAGTATCTTCTCAATGTGTAAATTTAACTACTAGAA
CTCAATTGCCACCAGCTTATACTAATTCTTTTACTAGAGGAGTATATTATCCAGATAAAGTATT
TAGATCTTCTGTATTACATTCTACTCAAGATTTATTTTTACCATTTTTTTCTAATGTAACTTGG
TTTCATGCTATACATGTATCTGGAACTAATGGAACTAAAAGATTTGATAATCCAGTATTACCAT
TTAATGATGGAGTATATTTTGCTTCTACTGAAAAATCTAATATAATAAGAGGATGGATATTTGG
AACTACTTTAGATTCTAAAACTCAATCTTTATTAATAGTAAATAATGCTACTAATGTAGTAATA
AAAGTATGTGAATTTCAATTTTGTAATGATCCATTTTTAGGAGTATATTATCATAAAAATAATA
AATCTTGGATGGAATCTGAATTTAGAGTATATTCTTCTGCTAATAATTGTACTTTTGAATATGT
ATCTCAACCATTTTTAATGGATTTAGAAGGAAAACAAGGAAATTTTAAAAATTTAAGAGAATTT
GTATTTAAAAATATAGATGGATATTTTAAAATATATTCTAAACATACTCCAATAAATTTAGTAA
GAGATTTACCACAAGGATTTTCTGCTTTAGAACCATTAGTAGATTTACCAATAGGAATAAATAT
AACTAGATTTCAAACTTTATTAGCTTTACATAGATCTTATTTAACTCCAGGAGATTCTTCTTCT
GGATGGACTGCTGGAGCTGCTGCTTATTATGTAGGATATTTACAACCAAGAACTTTTTTATTAA
AATATAATGAAAATGGAACTATAACTGATGCTGTAGATTGTGCTTTAGATCCATTATCTGAAAC
TAAATGTACTTTAAAATCTTTTACTGTAGAAAAAGGAATATATCAAACTTCTAATTTTAGAGTA
CAACCAACTGAATCTATAGTAAGATTTCCAAATATAACTAATTTATGTCCATTTGGAGAAGTAT
TTAATGCTACTAGATTTGCTTCTGTATATGCTTGGAATAGAAAAAGAATATCTAATTGTGTAGC
TGATTATTCTGTATTATATAATTCTGCTTCTTTTTCTACTTTTAAATGTTATGGAGTATCTCCA
ACTAAATTAAATGATTTATGTTTTACTAATGTATATGCTGATTCTTTTGTAATAAGAGGAGATG
AAGTAAGACAAATAGCTCCAGGACAAACTGGAAAAATAGCTGATTATAATTATAAATTACCAGA
TGATTTTACTGGATGTGTAATAGCTTGGAATAGTAATAATTTAGATTCTAAAGTAGGAGGAAAT
TATAATTATTTATATAGATTATTTAGAAAATCTAATTTAAAACCATTTGAAAGAGATATATCTA
CTGAAATATATCAAGCTGGATCTACTCCATGTAATGGAGTAGAAGGATTTAATTGTTATTTTCC
ATTACAATCTTATGGATTTCAACCAACTAATGGAGTAGGATATCAACCATATAGAGTAGTAGTA
TTATCTTTTGAATTATTACATGCTCCAGCTACTGTATGTGGACCAAAAAAATCTACTAATTTAG
TAAAAAATAAATGTGTAAATTTTAATTTTAATGGATTAACTGGAACTGGAGTATTAACTGAATC
TAATAAAAAATTTTTACCATTTCAACAATTTGGAAGAGATATAGCTGATACTACTGATGCTGTA
AGAGATCCACAAACTTTAGAAATATTAGATATAACTCCATGTTCTTTTGGAGGAGTATCTGTAA
TAACTCCAGGAACTAATACTTCTAATCAAGTAGCTGTATTATATCAAGATGTAAATTGTACTGA
AGTACCAGTAGCTATACATGCTGATCAATTAACTCCAACTTGGAGAGTATATTCTACTGGATCT
AATGTATTTCAAACTAGAGCTGGATGTTTAATAGGAGCTGAACATGTAAATAATTCTTATGAAT
GTGATATACCAATAGGAGCTGGAATATGTGCTTCTTATCAAACTCAAACTAATTCTCCAAGAAG
AGCTAGATCTGTAGCTAGCCAATCTATAATAGCTTATACTATGTCTTTAGGAGCTGAAAATTCT
GTAGCTTATTCTAATAATTCTATAGCTATACCAACTAATTTTACTATATCTGTAACTACTGAAA
TATTACCAGTATCTATGACTAAAACTTCTGTAGATTGTACTATGTATATATGTGGAGATTCTAC
TGAATGTTCTAATTTATTATTACAATATGGATCTTTTTGTACTCAATTAAATAGAGCTTTAACT
GGAATAGCTGTAGAACAAGATAAAAATACTCAAGAAGTATTTGCTCAAGTAAAACAAATATATA
AAACTCCACCAATAAAAGATTTTGGAGGATTTAATTTTTCTCAAATATTACCAGATCCATCTAA
ACCATCTAAAAGATCTTTTATAGAAGATTTATTATTTAATAAAGTAACTTTAGCTGATGCTGGA
TTTATAAAACAATATGGAGATTGTTTAGGAGATATAGCTGCTAGAGATTTAATATGTGCTCAAA
AATTTAATGGATTAACTGTATTACCACCATTATTAACTGATGAAATGATAGCTCAATATACTTC
TGCTTTATTAGCTGGAACTATAACTTCTGGATGGACTTTTGGAGCTGGAGCTGCTTTACAAATA
CCATTTGCTATGCAAATGGCTTATAGATTTAATGGAATAGGAGTAACTCAAAATGTATTATATG
AAAATCAAAAATTAATAGCTAATCAATTTAATTCTGCTATAGGAAAAATACAAGATTCTTTATC
 
TTCTACTGCTTCTGCTTTAGGAAAATTACAAGATGTAGTAAATCAAAATGCTCAAGCATTAAAT
ACTTTAGTAAAACAATTATCTTCTAATTTTGGAGCTATATCTTCTGTATTAAATGATATATTAT
CTAGATTAGATAAAGTAGAAGCTGAAGTACAAATAGATAGATTAATAACTGGAAGATTACAATC
TTTACAAACTTATGTAACTCAACAATTAATAAGAGCTGCTGAAATAAGAGCTTCTGCTAATTTA
GCTGCTACTAAAATGTCTGAATGTGTATTAGGACAATCTAAAAGAGTAGATTTTTGTGGAAAAG
GATATCATTTAATGTCTTTTCCACAATCTGCTCCACATGGAGTAGTATTTTTACATGTAACTTA
TGTACCAGCTCAAGAAAAAAATTTTACTACTGCTCCAGCTATATGTCATGATGGAAAAGCTCAT
TTTCCAAGAGAAGGAGTATTTGTATCTAATGGAACTCATTGGTTTGTAACTCAAAGAAATTTTT
ATGAACCACAAATAATAACTACTGATAATACTTTTGTATCTGGAAATTGTGATGTAGTAATAGG
AATAGTAAATAATACTGTATATGATCCATTACAACCAGAATTAGATTCTTTTAAAGAAGAATTA
GATAAATATTTTAAAAATCATACTTCTCCAGATGTAGATTTAGGAGATATATCTGGAATAAATG
CTTCTGTAGTAAATATACAAAAAGAAATAGATAGATTAAATGAAGTAGCTAAAAATTTAAATGA
ATCTTTAATAGATTTACAAGAATTAGGAAAATATGAACAATATATAAAATGGCCATGGTATATA
TGGTTAGGATTTATAGCTGGATTAATAGCTATAGTAATGGTAACTATAATGTTATGTTGTATGA
CTTCTTGTTGTTCTTGTTTAAAAGGATGTTGTTCTTGTGGATCTTGTTGTAAATTTGATGAAGA
TGATTCTGAACCAGTATTAAAAGGAGTAAAATTACATTATACTTAA
 **SEQ ID NO: 2** Amino acid sequence of S protein from MVA-S (GenBank accession No QHD43416.1 or Uniprot ID: P0DTC2) 
 50
 100
 150
 200
 250
 300
 350
 400
 450
 500
 550
 600
 650
 700
 750
 800
 850
 900
 950
 1000
 1050
 1100
 1150
 1200
 1250
*
 **SEQ ID NO: 3** Nucleic acid encoding for the S protein from MVA-Sdg 
ATGTTTGTATTTTTAGTATTATTACCATTAGTATCTTCTCAATGTGTAAATTTAACTACTAGAA
CTCAATTGCCACCAGCTTATACTAATTCTTTTACTAGAGGAGTATATTATCCAGATAAAGTATT
 
TAGATCTTCTGTATTACATTCTACTCAAGATTTATTTTTACCATTTTTTTCTAATGTAACTTGG
TTTCATGCTATACATGTATCTGGAACTAATGGAACTAAAAGATTTGATAATCCAGTATTACCAT
TTAATGATGGAGTATATTTTGCTTCTACTGAAAAATCTAATATAATAAGAGGATGGATATTTGG
AACTACTTTAGATTCTAAAACTCAATCTTTATTAATAGTAAATAATGCTACTAATGTAGTAATA
AAAGTATGTGAATTTCAATTTTGTAATGATCCATTTTTAGGAGTATATTATCATAAAAATAATA
AATCTTGGATGGAATCTGAATTTAGAGTATATTCTTCTGCTAATAATTGTACTTTTGAATATGT
ATCTCAACCATTTTTAATGGATTTAGAAGGAAAACAAGGAAATTTTAAAAATTTAAGAGAATTT
GTATTTAAAAATATAGATGGATATTTTAAAATATATTCTAAACATACTCCAATAAATTTAGTAA
GAGATTTACCACAAGGATTTTCTGCTTTAGAACCATTAGTAGATTTACCAATAGGAATAAATAT
AACTAGATTTCAAACTTTATTAGCTTTACATAGATCTTATTTAACTCCAGGAGATTCTTCTTCT
GGATGGACTGCTGGAGCTGCTGCTTATTATGTAGGATATTTACAACCAAGAACTTTTTTATTAA
AATATAATGAAAATGGAACTATAACTGATGCTGTAGATTGTGCTTTAGATCCATTATCTGAAAC
TAAATGTACTTTAAAATCTTTTACTGTAGAAAAAGGAATATATCAAACTTCTAATTTTAGAGTA
CAACCAACTGAATCTATAGTAAGATTTCCAAATATAACTAATTTATGTCCATTTGGAGAAGTAT
TTAATGCTACTAGATTTGCTTCTGTATATGCTTGGAATAGAAAAAGAATATCTAATTGTGTAGC
TGATTATTCTGTATTATATAATTCTGCTTCTTTTTCTACTTTTAAATGTTATGGAGTATCTCCA
ACTAAATTAAATGATTTATGTTTTACTAATGTATATGCTGATTCTTTTGTAATAAGAGGAGATG
AAGTAAGACAAATAGCTCCAGGACAAACTGGAAAAATAGCTGATTATAATTATAAATTACCAGA
TGATTTTACTGGATGTGTAATAGCTTGGAATAGTAATAATTTAGATTCTAAAGTAGGAGGAAAT
TATAATTATTTATATAGATTATTTAGAAAATCTAATTTAAAACCATTTGAAAGAGATATATCTA
CTGAAATATATCAAGCTGGATCTACTCCATGTAATGGAGTAGAAGGATTTAATTGTTATTTTCC
ATTACAATCTTATGGATTTCAACCAACTAATGGAGTAGGATATCAACCATATAGAGTAGTAGTA
TTATCTTTTGAATTATTACATGCTCCAGCTACTGTATGTGGACCAAAAAAATCTACTAATTTAG
TAAAAAATAAATGTGTAAATTTTAATTTTAATGGATTAACTGGAACTGGAGTATTAACTGAATC
TAATAAAAAATTTTTACCATTTCAACAATTTGGAAGAGATATAGCTGATACTACTGATGCTGTA
AGAGATCCACAAACTTTAGAAATATTAGATATAACTCCATGTTCTTTTGGAGGAGTATCTGTAA
TAACTCCAGGAACTAATACTTCTAATCAAGTAGCTGTATTATATCAAGGAGTAAATTGTACTGA
AGTACCAGTAGCTATACATGCTGATCAATTAACTCCAACTTGGAGAGTATATTCTACTGGATCT
AATGTATTTCAAACTAGAGCTGGATGTTTAATAGGAGCTGAACATGTAAATAATTCTTATGAAT
GTGATATACCAATAGGAGCTGGAATATGTGCTTCTTATCAAACTCAAACTAATTCTCCAAGAAG
AGCTAGATCTGTAGCTAGCCAATCTATAATAGCTTATACTATGTCTTTAGGAGCTGAAAATTCT
GTAGCTTATTCTAATAATTCTATAGCTATACCAACTAATTTTACTATATCTGTAACTACTGAAA
TATTACCAGTATCTATGACTAAAACTTCTGTAGATTGTACTATGTATATATGTGGAGATTCTAC
TGAATGTTCTAATTTATTATTACAATATGGATCTTTTTGTACTCAATTAAATAGAGCTTTAACT
GGAATAGCTGTAGAACAAGATAAAAATACTCAAGAAGTATTTGCTCAAGTAAAACAAATATATA
AAACTCCACCAATAAAAGATTTTGGAGGATTTAATTTTTCTCAAATATTACCAGATCCATCTAA
ACCATCTAAAAGATCTTTTATAGAAGATTTATTATTTAATAAAGTAACTTTAGCTGATGCTGGA
TTTATAAAACAATATGGAGATTGTTTAGGAGATATAGCTGCTAGAGATTTAATATGTGCTCAAA
AATTTAATGGATTAACTGTATTACCACCATTATTAACTGATGAAATGATAGCTCAATATACTTC
TGCTTTATTAGCTGGAACTATAACTTCTGGATGGACTTTTGGAGCTGGAGCTGCTTTACAAATA
CCATTTGCTATGCAAATGGCTTATAGATTTAATGGAATAGGAGTAACTCAAAATGTATTATATG
AAAATCAAAAATTAATAGCTAATCAATTTAATTCTGCTATAGGAAAAATACAAGATTCTTTATC
TTCTACTGCTTCTGCTTTAGGAAAATTACAAGATGTAGTAAATCAAAATGCTCAAGCATTAAAT
ACTTTAGTAAAACAATTATCTTCTAATTTTGGAGCTATATCTTCTGTATTAAATGATATATTAT
CTAGATTAGATAAAGTAGAAGCTGAAGTACAAATAGATAGATTAATAACTGGAAGATTACAATC
TTTACAAACTTATGTAACTCAACAATTAATAAGAGCTGCTGAAATAAGAGCTTCTGCTAATTTA
GCTGCTACTAAAATGTCTGAATGTGTATTAGGACAATCTAAAAGAGTAGATTTTTGTGGAAAAG
GATATCATTTAATGTCTTTTCCACAATCTGCTCCACATGGAGTAGTATTTTTACATGTAACTTA
TGTACCAGCTCAAGAAAAAAATTTTACTACTGCTCCAGCTATATGTCATGATGGAAAAGCTCAT
TTTCCAAGAGAAGGAGTATTTGTATCTAATGGAACTCATTGGTTTGTAACTCAAAGAAATTTTT
ATGAACCACAAATAATAACTACTGATAATACTTTTGTATCTGGAAATTGTGATGTAGTAATAGG
AATAGTAAATAATACTGTATATGATCCATTACAACCAGAATTAGATTCTTTTAAAGAAGAATTA
GATAAATATTTTAAAAATCATACTTCTCCAGATGTAGATTTAGGAGATATATCTGGAATAAATG
 
CTTCTGTAGTAAATATACAAAAAGAAATAGATAGATTAAATGAAGTAGCTAAAAATTTAAATGA
ATCTTTAATAGATTTACAAGAATTAGGAAAATATGAACAATATATAAAATGGCCATGGTATATA
TGGTTAGGATTTATAGCTGGATTAATAGCTATAGTAATGGTAACTATAATGTTATGTTGTATGA
CTTCTTGTTGTTCTTGTTTAAAAGGATGTTGTTCTTGTGGATCTTGTTGTAAATTTGATGAAGA
TGATTCTGAACCAGTATTAAAAGGAGTAAAATTACATTATACTTAA
 **SEQ ID NO: 4** Amino acid sequence of S protein from MVA-Sdg

| | | |
|---|---|---|
| Start | = | 1869 |
| End | = | 5688 |
| Length | = | 3819 bp |
| MW | = | 141119 dalton |
| A280 | = | 131520 /M/cm reduced, 134040 /M/cm oxidised |

MFVFLVLLPLVSSQCVNLTTRTQLPPAYTNSFTRGVYYPDKVFRSSVLHS 50
TQDLFLPFFSNVTWFHAIHVSGTNGTKRFDNPVLPFNDGVYFASTEKSNI 100
IRGWIFGTTLDSKTQSLLIVNNATNVVIKVCEFQFCNDPFLGVYYHKNNK 150
SWMESEFRVYSSANNCTFEYVSQPFLMDLEGKQGNFKNLREFVFKNIDGY 200
FKIYSKHTPINLVRDLPQGFSALEPLVDLPIGINITRFQTLLALHRSYLT 250
PGDSSSGWTAGAAAYYVGYLQPRTFLLKYNENGTITDAVDCALDPLSETK 300
CTLKSFTVEKGIYQTSNFRVQPTESIVRFPNITNLCPFGEVFNATRFASV 350
YAWNRKRISNCVADYSVLYNSASFSTFKCYGVSPTKLNDLCFTNVYADSF 400
VIRGDEVRQIAPGQTGKIADYNYKLPDDFTGCVIAWNSNNLDSKVGGNYN 450
YLYRLFRKSNLKPFERDISTEIYQAGSTPCNGVEGFNCYFPLQSYGFQPT 500
NGVGYQPYRVVVLSFELLHAPATVCGPKKSTNLVKNKCVNFNFNGLTGTG 550
VLTESNKKFLPFQQFGRDIADTTDAVRDPQTLEILDITPCSFGGVSVITP 600
GTNTSNQVAVLYQGVNCTEVPVAIHADQLTPTWRVYSTGSNVFQTRAGCL 650
IGAEHVNNSYECDIPIGAGICASYQTQTNSPRRARSVASQSIIAYTMSLG 700
AENSVAYSNNSIAIPTNFTISVTTEILPVSMTKTSVDCTMYICGDSTECS 750
NLLLQYGSFCTQLNRALTGIAVEQDKNTQEVFAQVKQIYKTPPIKDFGGF 800
NFSQILPDPSKPSKRSFIEDLLFNKVTLADAGFIKQYGDCLGDIAARDLI 850
CAQKFNGLTVLPPLLTDEMIAQYTSALLAGTITSGWTFGAGAALQIPFAM 900
QMAYRFNGIGVTQNVLYENQKLIANQFNSAIGKIQDSLSSTASALGKLQD 950
VVNQNAQALNTLVKQLSSNFGAISSVLNDILSRLDKVEAEVQIDRLITGR 1000
LQSLQTYVTQQLIRAAEIRASANLAATKMSECVLGQSKRVDFCGKGYHLM 1050
SFPQSAPHGVVFLHVTYVPAQEKNFTTAPAICHDGKAHFPREGVFVSNGT 1100
HWFVTQRNFYEPQIITTDNTFVSGNCDVVIGIVNNTVYDPLQPELDSFKE 1150
ELDKYFKNHTSPDVDLGDISGINASVVNIQKEIDRLNEVAKNLNESLIDL 1200
QELGKYEQYIKWPWYIWLGFIAGLIAIVMVTIMLCCMTSCCSCLKGCCSC 1250
GSCCKFDEDDSEPVLKGVKLHYT

**SEQ ID NO: 5** Amino acid sequence of S protein from MVA- Spfx and MVA-Spf

| | | |
|---|---|---|
| **Start** | **=** | **1869** |
| **End** | **=** | **5688** |
| **Length** | **=** | **3819 bp** |
| **MW** | **=** | **140948 dalton** |
| **A280** | **=** | **131520 /M/cm reduced, 134040 /M/cm oxidised** |

MFVFLVLLPLVSSQCVNLTTRTQLPPAYTNSFTRGVYYPDKVFRSSVLHS 50
TQDLFLPFFSNVTWFHAIHVSGTNGTKRFDNPVLPFNDGVYFASTEKSNI 100
IRGWIFGTTLDSKTQSLLIVNNATNVVIKVCEFQFCNDPFLGVYYHKNNK 150
SWMESEFRVYSSANNCTFEYVSQPFLMDLEGKQGNFKNLREFVFKNIDGY 200

FKIYSKHTPINLVRDLPQGFSALEPLVDLPIGINITRFQTLLALHRSYLT 250
PGDSSSGWTAGAAAYYVGYLQPRTFLLKYNENGTITDAVDCALDPLSETK 300
CTLKSFTVEKGIYQTSNFRVQPTESIVRFPNITNLCPFGEVFNATRFASV 350
YAWNRKRISNCVADYSVLYNSASFSTFKCYGVSPTKLNDLCFTNVYADSF 400
VIRGDEVRQIAPGQTGKIADYNYKLPDDFTGCVIAWNSNNLDSKVGGNYN 450
YLYRLFRKSNLKPFERDISTEIYQAGSTPCNGVEGFNCYFPLQSYGFQPT 500
NGVGYQPYRVVVLSFELLHAPATVCGPKKSTNLVKNKCVNFNFNGLTGTG 550
VLTESNKKFLPFQQFGRDIADTTDAVRDPQTLEILDITPCSFGGVSVITP 600
GTNTSNQVAVLYQGVNCTEVPVAIHADQLTPTWRVYSTGSNVFQTRAGCL 650
IGAEHVNNSYECDIPIGAGICASYQTQTNSPSRASSVASQSIIAYTMSLG 700
AENSVAYSNNSIAIPTNFTISVTTEILPVSMTKTSVDCTMYICGDSTECS 750
NLLLQYGSFCTQLNRALTGIAVEQDKNTQEVFAQVKQIYKTPPIKDFGGF 800
NFSQILPDPSKPSKRSFIEDLLFNKVTLADAGFIKQYGDCLGDIAARDLI 850
CAQKFNGLTVLPPLLTDEMIAQYTSALLAGTITSGWTFGAGAALQIPFAM 900
QMAYRFNGIGVTQNVLYENQKLIANQFNSAIGKIQDSLSSTASALGKLQD 950
VVNQNAQALNTLVKQLSSNFGAISSVLNDILSRLDPPEAEVQIDRLITGR 1000
LQSLQTYVTQQLIRAAEIRASANLAATKMSECVLGQSKRVDFCGKGYHLM 1050
SFPQSAPHGVVFLHVTYVPAQEKNFTTAPAICHDGKAHFPREGVFVSNGT 1100
HWFVTQRNFYEPQIITTDNTFVSGNCDVVIGIVNNTVYDPLQPELDSFKE 1150
ELDKYFKNHTSPDVDLGDISGINASVVNIQKEIDRLNEVAKNLNESLIDL 1200
QELGKYEQYIKWPWYIWLGFIAGLIAIVMVTIMLCCMTSCCSCLKGCCSC 1250
GSCCKFDEDDSEPVLKGVKLHYT*

**SEQ ID NO: 6** Nucleic acid encoding for the S protein from MVA-Spfx 
ATGTTTGTATTTTTAGTATTATTACCATTAGTATCTTCTCAATGTGTAAATTTAACTACTAGAA
CTCAATTGCCCCCGGCCTATACTAATTCGTTTACTCGTGGTGTTTACTATCCTGATAAAGTGTT
TCGATCGTCTGTGCTTCATTCGACTCAAGATCTATTTTTGCCCTTCTTTAGTAATGTTACATGG
TTCCATGCTATACATGTATCGGGTACAAACGGTACGAAGCGTTTCGACAACCCAGTCTTACCAT
TCAATGACGGAGTTTATTTTGCCAGTACCGAGAAATCAAATATCATCAGAGGATGGATTTTTGG
TACTACTTTGGATAGTAAGACTCAGTCTCTATTAATCGTCAATAACGCAACGAACGTTGTAATC
AAAGTATGCGAGTTTCAATTCTGCAACGATCCATTTCTAGGTGTTTACTACCACAAGAACAATA
AGTCGTGGATGGAAAGTGAGTTCCGTGTGTACTCTTCTGCCAATAACTGCACTTTCGAATATGT
TTCACAGCCGTTTTTAATGGACTTAGAGGGTAAACAAGGTAATTTCAAAAACTTGAGAGAATTT
GTCTTCAAAAATATTGATGGTTATTTCAAAATATATTCAAAGCATACGCCCATTAATCTTGTTC
GAGACCTACCGCAAGGTTTTTCCGCATTAGAACCCTTAGTTGATCTACCCATTGGAATCAACAT
TACAAGATTCCAGACATTGTTAGCTTTACACCGTTCGTATCTTACACCCGGTGATTCGTCATCA
GGATGGACCGCTGGAGCTGCTGCCTATTATGTCGGTTACTTGCAACCGCGTACATTCCTATTGA
AGTACAATGAAAATGGAACTATTACAGACGCCGTGGACTGTGCCTTGGATCCTTTATCTGAAAC
TAAGTGTACATTAAAATCTTTTACAGTAGAAAAAGGTATTTACCAAACGTCCAACTTCCGTGTC
CAACCCACGGAATCGATAGTTAGATTTCCGAATATTACGAACCTATGCCCATTTGGAGAAGTCT
TTAACGCCACGCGTTTTGCCTCAGTTTACGCTTGGAATCGTAAGCGTATTAGTAACTGCGTAGC
GGATTATTCCGTACTATACAATTCCGCGTCCTTTTCGACTTTCAAATGTTACGGAGTGTCGCCC
ACTAAACTTAACGATCTTTGTTTCACCAACGTGTACGCAGACTCATTTGTAATCAGAGGAGACG
AAGTGCGACAAATTGCGCCTGGTCAGACCGGAAAAATCGCAGATTACAATTATAAACTTCCGGA
CGATTTCACTGGTTGTGTCATTGCATGGAATTCCAACAACCTAGACAGTAAAGTAGGAGGTAAC
TATAATTACCTATATAGATTATTCCGAAAATCAAATTTAAAACCATTTGAACGTGACATCAGTA
CAGAGATATACCAAGCGGGATCTACGCCGTGCAACGGAGTAGAGGGATTTAATTGCTATTTCCC
GTTACAATCGTACGGATTCCAACCCACGAACGGAGTAGGTTACCAACCGTACCGTGTCGTGGTC
CTTAGTTTTGAGTTGTTGCACGCACCTGCCACCGTGTGCGGTCCCAAAAAGTCAACAAACTTAG
TCAAAAACAAATGTGTCAACTTCAATTTTAATGGACTAACAGGAACCGGAGTCTTGACTGAATC
 
AAACAAAAAATTCCTTCCTTTCCAACAATTCGGTCGTGATATTGCGGATACGACAGATGCGGTA
CGAGACCCGCAAACACTTGAGATATTGGATATAACCCCTTGCTCATTCGGTGGTGTTTCGGTAA
TCACCCCTGGAACAAACACGAGTAACCAGGTAGCCGTACTTTACCAGGGTGTGAACTGTACAGA
GGTGCCAGTCGCGATACATGCGGATCAGCTAACGCCAACGTGGAGAGTATATTCTACCGGAAGT
AATGTGTTCCAAACCAGAGCCGGATGTCTTATCGGTGCAGAACATGTAAACAATAGTTACGAGT
GTGATATCCCCATTGGAGCGGGTATTTGCGCAAGTTATCAAACCCAAACAAACTCTCCCTCTAG
AGCCTCTAGTGTTGCCTCTCAGAGTATCATTGCTTACACGATGTCGTTGGGAGCAGAAAATTCT
GTTGCATATTCTAACAATTCGATAGCAATTCCAACCAATTTCACGATATCCGTAACCACCGAGA
TCTTGCCAGTTAGTATGACTAAAACATCGGTTGACTGCACAATGTATATATGCGGTGATTCTAC
TGAGTGTTCAAACTTGCTTCTTCAGTATGGTTCGTTTTGTACTCAGCTAAATCGAGCCTTGACG
GGAATAGCCGTTGAACAAGACAAAAATACGCAGGAGGTGTTTGCTCAAGTCAAACAAATTTACA
AGACCCCACCTATCAAGGATTTCGGTGGTTTTAACTTTTCCCAAATATTACCAGACCCATCAAA
GCCAAGTAAGAGATCCTTCATAGAGGACCTATTGTTTAATAAGGTAACGTTGGCGGACGCGGGT
TTTATTAAGCAATACGGTGACTGCTTAGGTGATATAGCTGCCAGAGACTTAATCTGCGCTCAGA
AATTCAACGGATTAACCGTTTTACCACCATTATTGACGGACGAGATGATCGCGCAGTATACCTC
GGCTTTGCTAGCTGGTACGATTACATCCGGTTGGACTTTCGGTGCCGGTGCAGCCCTACAGATA
CCATTTGCGATGCAAATGGCCTATCGTTTTAATGGAATCGGTGTCACCCAGAACGTGCTTTACG
AGAACCAGAAGTTAATTGCTAATCAATTCAACTCTGCAATCGGAAAGATTCAAGACTCATTATC
TAGTACAGCCTCCGCCTTAGGAAAGTTACAGGACGTTGTGAACCAGAATGCTCAGGCGTTAAAT
ACCTTGGTCAAACAACTATCCTCAAATTTCGGTGCGATATCTTCAGTCTTGAATGATATCCTAA
GTCGACTAGATCCACCTGAGGCTGAAGTACAGATTGACCGACTTATCACAGGTCGTCTACAAAG
TTTGCAGACGTATGTGACCCAACAGCTAATAAGAGCGGCTGAGATACGTGCTTCTGCCAATTTA
GCAGCTACAAAAATGTCAGAGTGTGTGCTAGGACAATCGAAACGAGTTGACTTCTGTGGAAAGG
GTTACCACTTGATGTCATTCCCCCAAAGTGCGCCTCATGGTGTGGTTTTCTTGCATGTAACGTA
CGTACCCGCGCAAGAAAAGAATTTTACTACCGCCCCGGCCATTTGTCATGACGGAAAAGCTCAT
TTTCCGCGTGAAGGTGTATTCGTATCCAACGGTACGCACTGGTTTGTGACCCAAAGAAACTTTT
ATGAACCGCAGATTATAACGACCGACAATACGTTCGTGTCTGGAAATTGCGACGTTGTAATTGG
AATCGTAAACAATACAGTTTACGACCCATTGCAACCCGAGCTAGATAGTTTTAAGGAAGAACTT
GACAAGTATTTTAAGAACCACACGTCACCGGACGTCGACTTGGGAGATATTTCGGGAATTAACG
CTAGTGTTGTGAATATCCAGAAAGAAATAGATCGACTAAATGAAGTGGCCAAGAACCTTAACGA
GTCCCTAATTGACTTACAGGAATTGGGTAAGTATGAACAATACATAAAATGGCCATGGTATATA
TGGTTAGGATTTATAGCTGGATTAATAGCTATAGTAATGGTTACTATCATGTTATGCTGCATGA
CCTCCTGCTGTTCATGTTTAAAAGGATGTTGCTCCTGCGGTTCGTGTTGTAAGTTCGATGAAGA
TGACTCCGAACCTGTCTTGAAGGGAGTGAAATTGCATTACACTTAA
 **SEQ ID NO: 7** Nucleic acid encoding for the S protein from MVA-Spf 
ATGTTTGTATTTTTAGTATTATTACCATTAGTATCTTCTCAATGTGTAAATTTAACTACTAGAA
CTCAATTGCCACCAGCTTATACTAATTCTTTTACTAGAGGAGTATATTATCCAGATAAAGTATT
TAGATCTTCTGTATTACATTCTACTCAAGATTTATTTTTACCATTTTTTTCTAATGTAACTTGG
TTTCATGCTATACATGTATCTGGAACTAATGGAACTAAAAGATTTGATAATCCAGTATTACCAT
TTAATGATGGAGTATATTTTGCTTCTACTGAAAAATCTAATATAATAAGAGGATGGATATTTGG
AACTACTTTAGATTCTAAAACTCAATCTTTATTAATAGTAAATAATGCTACTAATGTAGTAATA
AAAGTATGTGAATTTCAATTTTGTAATGATCCATTTTTAGGAGTATATTATCATAAAAATAATA
AATCTTGGATGGAATCTGAATTTAGAGTATATTCTTCTGCTAATAATTGTACTTTTGAATATGT
ATCTCAACCATTTTTAATGGATTTAGAAGGAAAACAAGGAAATTTTAAAAATTTAAGAGAATTT
GTATTTAAAAATATAGATGGATATTTTAAAATATATTCTAAACATACTCCAATAAATTTAGTAA
GAGATTTACCACAAGGATTTTCTGCTTTAGAACCATTAGTAGATTTACCAATAGGAATAAATAT
AACTAGATTTCAAACTTTATTAGCTTTACATAGATCTTATTTAACTCCAGGAGATTCTTCTTCT
GGATGGACTGCTGGAGCTGCTGCTTATTATGTAGGATATTTACAACCAAGAACTTTTTTATTAA
 
AATATAATGAAAATGGAACTATAACTGATGCTGTAGATTGTGCTTTAGATCCATTATCTGAAAC
TAAATGTACTTTAAAATCTTTTACTGTAGAAAAAGGAATATATCAAACTTCTAATTTTAGAGTA
CAACCAACTGAATCTATAGTAAGATTTCCAAATATAACTAATTTATGTCCATTTGGAGAAGTAT
TTAATGCTACTAGATTTGCTTCTGTATATGCTTGGAATAGAAAAAGAATATCTAATTGTGTAGC
TGATTATTCTGTATTATATAATTCTGCTTCTTTTTCTACTTTTAAATGTTATGGAGTATCTCCA
ACTAAATTAAATGATTTATGTTTTACTAATGTATATGCTGATTCTTTTGTAATAAGAGGAGATG
AAGTAAGACAAATAGCTCCAGGACAAACTGGAAAAATAGCTGATTATAATTATAAATTACCAGA
TGATTTTACTGGATGTGTAATAGCTTGGAATAGTAATAATTTAGATTCTAAAGTAGGAGGAAAT
TATAATTATTTATATAGATTATTTAGAAAATCTAATTTAAAACCATTTGAAAGAGATATATCTA
CTGAAATATATCAAGCTGGATCTACTCCATGTAATGGAGTAGAAGGATTTAATTGTTATTTTCC
ATTACAATCTTATGGATTTCAACCAACTAATGGAGTAGGATATCAACCATATAGAGTAGTAGTA
TTATCTTTTGAATTATTACATGCTCCAGCTACTGTATGTGGACCAAAAAAATCTACTAATTTAG
TAAAAAATAAATGTGTAAATTTTAATTTTAATGGATTAACTGGAACTGGAGTATTAACTGAATC
TAATAAAAAATTTTTACCATTTCAACAATTTGGAAGAGATATAGCTGATACTACTGATGCTGTA
AGAGATCCACAAACTTTAGAAATATTAGATATAACTCCATGTTCTTTTGGAGGAGTATCTGTAA
TAACTCCAGGAACTAATACTTCTAATCAAGTAGCTGTATTATATCAAGGAGTAAATTGTACTGA
AGTACCAGTAGCTATACATGCTGATCAATTAACTCCAACTTGGAGAGTATATTCTACTGGATCT
AATGTATTTCAAACTAGAGCTGGATGTTTAATAGGAGCTGAACATGTAAATAATTCTTATGAAT
GTGATATACCAATAGGAGCTGGAATATGTGCTTCTTATCAAACTCAAACTAATTCTCCATCTAG
AGCCTCTTCTGTAGCTAGCCAATCTATAATAGCTTATACTATGTCTTTAGGAGCTGAAAATTCT
GTAGCTTATTCTAATAATTCTATAGCTATACCAACTAATTTTACTATATCTGTAACTACTGAAA
TATTACCAGTATCTATGACTAAAACTTCTGTAGATTGTACTATGTATATATGTGGAGATTCTAC
TGAATGTTCTAATTTATTATTACAATATGGATCTTTTTGTACTCAATTAAATAGAGCTTTAACT
GGAATAGCTGTAGAACAAGATAAAAATACTCAAGAAGTATTTGCTCAAGTAAAACAAATATATA
AAACTCCACCAATAAAAGATTTTGGAGGATTTAATTTTTCTCAAATATTACCAGATCCATCTAA
ACCATCTAAAAGATCTTTTATAGAAGATTTATTATTTAATAAAGTAACTTTAGCTGATGCTGGA
TTTATAAAACAATATGGAGATTGTTTAGGAGATATAGCTGCTAGAGATTTAATATGTGCTCAAA
AATTTAATGGATTAACTGTATTACCACCATTATTAACTGATGAAATGATAGCTCAATATACTTC
TGCTTTATTAGCTGGAACTATAACTTCTGGATGGACTTTTGGAGCTGGAGCTGCTTTACAAATA
CCATTTGCTATGCAAATGGCTTATAGATTTAATGGAATAGGAGTAACTCAAAATGTATTATATG
AAAATCAAAAATTAATAGCTAATCAATTTAATTCTGCTATAGGAAAAATACAAGATTCTTTATC
TTCTACTGCTTCTGCTTTAGGAAAATTACAAGATGTAGTAAATCAAAATGCTCAAGCATTAAAT
ACTTTAGTAAAACAATTATCTTCTAATTTTGGAGCTATATCTTCTGTATTAAATGATATATTAT
CTAGATTAGATCCACCTGAAGCTGAAGTACAAATAGATAGATTAATAACTGGAAGATTACAATC
TTTACAAACTTATGTAACTCAACAATTAATAAGAGCTGCTGAAATAAGAGCTTCTGCTAATTTA
GCTGCTACTAAAATGTCTGAATGTGTATTAGGACAATCTAAAAGAGTAGATTTTTGTGGAAAAG
GATATCATTTAATGTCTTTTCCACAATCTGCTCCACATGGAGTAGTATTTTTACATGTAACTTA
TGTACCAGCTCAAGAAAAAAATTTTACTACTGCTCCAGCTATATGTCATGATGGAAAAGCTCAT
TTTCCAAGAGAAGGAGTATTTGTATCTAATGGAACTCATTGGTTTGTAACTCAAAGAAATTTTT
ATGAACCACAAATAATAACTACTGATAATACTTTTGTATCTGGGAATTGTGATGTAGTAATAGG
AATAGTAAATAATACTGTATATGATCCATTACAACCAGAATTAGATTCTTTTAAAGAAGAATTA
GATAAATATTTTAAAAATCATACTTCTCCAGATGTAGATTTAGGAGATATATCTGGAATAAATG
CTTCTGTAGTAAATATACAAAAAGAAATAGATAGATTAAATGAAGTAGCTAAAAATTTAAATGA
ATCTTTAATAGATTTACAAGAATTAGGAAAATATGAACAATATATAAAATGGCCATGGTATATA
TGGTTAGGATTTATAGCTGGATTAATAGCTATAGTAATGGTAACTATAATGTTATGTTGTATGA
CTTCTTGTTGTTCTTGTTTAAAAGGATGTTGTTCTTGTGGATCTTGTTGTAAATTTGATGAAGA
TGATTCTGAACCAGTATTAAAAGGAGTAAAATTACATTATACTTAA
 **SEQ ID NO: 8** Nucleic acid sequence of S protein from MVA-Spf-δ 
ATGTTTGTATTTTTAGTATTATTACCATTAGTATCTTCTCAATGTGTAAATTTAAGAACTAGAA
CTCAATTACCACCAGCTTATACTAATTCTTTTACTAGAGGAGTATATTATCCAGATAAAGTATT
TAGATCTTCTGTATTACATTCTACTCAAGATTTATTTTTACCATTCTTTTCTAATGTAACTTGG
TTTCATGCTATACATGTATCTGGAACTAATGGAACTAAAAGATTTGATAATCCAGTATTACCAT
TTAATGATGGAGTATATTTTGCTTCTATAGAAAAATCTAATATAATAAGAGGATGGATATTTGG
AACTACTTTAGATTCTAAAACTCAATCTTTATTAATAGTAAATAATGCTACTAATGTAGTAATA
AAAGTATGTGAATTTCAATTTTGTAATGATCCATTTTTAGGAGTATATTATCATAAAAATAATA
AATCTTGGATGGAATCTGGAGTATATTCTTCTGCTAATAATTGTACTTTTGAATATGTATCTCA
ACCATTTTTAATGGATTTAGAAGGAAAACAAGGAAATTTTAAAAATTTAAGAGAATTTGTATTT
AAAAATATAGATGGATATTTTAAAATATATTCTAAACATACTCCAATAAATTTAGTAAGAGATT
TACCACAAGGATTTTCTGCTTTAGAACCATTAGTAGATTTACCAATAGGAATAAATATAACTAG
ATTTCAAACTTTATTAGCTTTACATAGATCTTATTTAACTCCAGGAGATTCTTCTTCTGGATGG
ACTGCTGGAGCTGCTGCTTATTATGTAGGATATTTACAACCAAGAACTTTCTTATTAAAATATA
ATGAAAATGGAACTATAACTGATGCTGTAGATTGTGCTTTAGATCCATTATCTGAAACTAAATG
TACTTTAAAATCTTTTACTGTAGAAAAAGGAATATATCAAACTTCTAATTTTAGAGTACAACCA
ACTGAATCTATAGTAAGATTTCCAAATATAACTAATTTATGTCCATTTGGAGAAGTATTTAATG
CTACTAGATTTGCTTCTGTATATGCTTGGAATAGAAAAAGAATATCTAATTGTGTAGCTGATTA
TTCTGTATTATATAATTCTGCTTCTTTCTCTACTTTTAAATGTTATGGAGTATCTCCAACTAAA
TTAAATGATTTATGTTTTACTAATGTATATGCTGATTCTTTTGTAATAAGAGGAGATGAAGTAA
GACAAATAGCTCCAGGACAAACTGGAAAAATAGCTGATTATAATTATAAATTACCAGATGATTT
TACTGGATGTGTAATAGCTTGGAATTCTAATAATTTAGATTCTAAAGTAGGAGGAAATTATAAT
TATAGATATAGATTATTTAGAAAATCTAATTTAAAACCATTTGAAAGAGATATATCTACTGAAA
TATATCAAGCTGGATCTAAACCATGTAATGGAGTAGAAGGATTTAATTGTTATTTTCCATTACA
ATCTTATGGATTTCAACCAACTAATGGAGTAGGATATCAACCATATAGAGTAGTAGTATTATCT
TTTGAATTATTACATGCTCCAGCTACTGTATGTGGACCAAAAAAATCTACTAATTTAGTAAAAA
ATAAATGTGTAAATTTTAATTTTAATGGATTAACTGGAACTGGAGTATTAACTGAATCTAATAA
AAAATTTTTACCATTTCAACAATTTGGAAGAGATATAGCTGATACTACTGATGCTGTAAGAGAT
CCACAAACTTTAGAAATATTAGATATAACTCCATGTTCTTTTGGAGGAGTATCTGTAATAACTC
CAGGAACTAATACTTCTAATCAAGTAGCTGTATTATATCAAGGAGTAAATTGTACTGAAGTACC
AGTAGCTATACATGCTGATCAATTAACTCCAACTTGGAGAGTATATTCTACTGGATCTAATGTA
TTTCAAACTAGAGCTGGATGTTTAATAGGAGCTGAACATGTAAATAATTCTTATGAATGTGATA
TACCAATAGGAGCTGGAATATGTGCTTCTTATCAAACTCAAACTAATTCTAGATCTAGAGCTTC
TTCTGTAGCTTCTCAATCTATAATAGCTTATACTATGTCTTTAGGAGCTGAAAATTCTGTAGCT
TATTCTAATAATTCTATAGCTATACCAACTAATTTTACTATATCTGTAACTACTGAAATATTAC
CAGTATCTATGACTAAAACTTCTGTAGATTGTACTATGTATATATGTGGAGATTCTACTGAATG
TTCTAATTTATTATTACAATATGGATCTTTCTGTACTCAATTAAATAGAGCTTTAACTGGAATA
GCTGTAGAACAAGATAAAAATACTCAAGAAGTATTTGCTCAAGTAAAACAAATATATAAAACTC
CACCAATAAAAGATTTTGGAGGATTTAATTTCTCTCAAATATTACCAGATCCATCTAAACCATC
TAAAAGATCTTTTATAGAAGATTTATTATTTAATAAAGTAACTTTAGCTGATGCTGGATTTATA
AAACAATATGGAGATTGTTTAGGAGATATAGCTGCTAGAGATTTAATATGTGCTCAAAAATTTA
ATGGATTAACTGTATTACCACCATTATTAACTGATGAAATGATAGCTCAATATACTTCTGCTTT
ATTAGCTGGAACTATAACTTCTGGATGGACTTTTGGAGCTGGAGCTGCTTTACAAATACCATTT
GCTATGCAAATGGCTTATAGATTTAATGGAATAGGAGTAACTCAAAATGTATTATATGAAAATC
AAAAATTAATAGCTAATCAATTTAATTCTGCTATAGGAAAAATACAAGATTCTTTATCTTCTAC
TGCTTCTGCTTTAGGAAAATTACAAAATGTAGTAAATCAAAATGCTCAAGCTTTAAATACTTTA
GTAAAACAATTATCTTCTAATTTTGGAGCTATATCTTCTGTATTAAATGATATATTATCTAGAT
TAGATCCACCAGAAGCTGAAGTACAAATAGATAGATTAATAACTGGAAGATTACAATCTTTACA
AACTTATGTAACTCAACAATTAATAAGAGCTGCTGAAATAAGAGCTTCTGCTAATTTAGCTGCT
ACTAAAATGTCTGAATGTGTATTAGGACAATCTAAAAGAGTAGATTTCTGTGGAAAAGGATATC
ATTTAATGTCTTTTCCACAATCTGCTCCACATGGAGTAGTATTTTTACATGTAACTTATGTACC
AGCTCAAGAAAAAAATTTTACTACTGCTCCAGCTATATGTCATGATGGAAAAGCTCATTTTCCA
 
AGAGAAGGAGTATTTGTATCTAATGGAACTCATTGGTTTGTAACTCAAAGAAATTTCTATGAAC
CACAAATAATAACTACTGATAATACTTTTGTATCTGGAAATTGTGATGTAGTAATAGGAATAGT
AAATAATACTGTATATGATCCATTACAACCAGAATTAGATTCTTTTAAAGAAGAATTAGATAAA
TATTTTAAAAATCATACTTCTCCAGATGTAGATTTAGGAGATATATCTGGAATAAATGCTTCTG
TAGTAAATATACAAAAAGAAATAGATAGATTAAATGAAGTAGCTAAAAATTTAAATGAATCTTT
AATAGATTTACAAGAATTAGGAAAATATGAACAATATATAAAATGGCCATGGTATATATGGTTA
GGATTTATAGCTGGATTAATAGCTATAGTAATGGTAACTATAATGTTATGTTGTATGACTTCTT
GTTGTTCTTGTTTAAAAGGATGTTGTTCTTGTGGATCTTGTTGTAAATTTGATGAAGATGATTC
TGAACCAGTATTAAAAGGAGTAAAATTACATTATACTTAA
 **SEQ ID NO: 9** Amino acid sequence of S protein from MVA-Spf-δ 
MFVFLVLLPLVSSQCVNLRTRTQLPPAYTNSFTRGVYYPDKVFRSSVLHS 50
TQDLFLPFFSNVTWFHAIHVSGTNGTKRFDNPVLPFNDGVYFASIEKSNI 100
IRGWIFGTTLDSKTQSLLIVNNATNVVIKVCEFQFCNDPFLGVYYHKNNK 150
SWMESGVYSSANNCTFEYVSQPFLMDLEGKQGNFKNLREFVFKNIDGYFK 200
IYSKHTPINLVRDLPQGFSALEPLVDLPIGINITRFQTLLALHRSYLTPG 250
DSSSGWTAGAAAYYVGYLQPRTFLLKYNENGTITDAVDCALDPLSETKCT 300
LKSFTVEKGIYQTSNFRVQPTESIVRFPNITNLCPFGEVFNATRFASVYA 350
WNRKRISNCVADYSVLYNSASFSTFKCYGVSPTKLNDLCFTNVYADSFVI 400
RGDEVRQIAPGQTGKIADYNYKLPDDFTGCVIAWNSNNLDSKVGGNYNYR 450
YRLFRKSNLKPFERDISTEIYQAGSKPCNGVEGFNCYFPLQSYGFQPTNG 500
VGYQPYRVVVLSFELLHAPATVCGPKKSTNLVKNKCVNFNFNGLTGTGVL 550
TESNKKFLPFQQFGRDIADTTDAVRDPQTLEILDITPCSFGGVSVITPGT 600
NTSNQVAVLYQGVNCTEVPVAIHADQLTPTWRVYSTGSNVFQTRAGCLIG 650
AEHVNNSYECDIPIGAGICASYQTQTNSRSRASSVASQSIIAYTMSLGAE 700
NSVAYSNNSIAIPTNFTISVTTEILPVSMTKTSVDCTMYICGDSTECSNL 750
LLQYGSFCTQLNRALTGIAVEQDKNTQEVFAQVKQIYKTPPIKDFGGFNF 800
SQILPDPSKPSKRSFIEDLLFNKVTLADAGFIKQYGDCLGDIAARDLICA 850
QKFNGLTVLPPLLTDEMIAQYTSALLAGTITSGWTFGAGAALQIPFAMQM 900
AYRFNGIGVTQNVLYENQKLIANQFNSAIGKIQDSLSSTASALGKLQNVV 950
NQNAQALNTLVKQLSSNFGAISSVLNDILSRLDPPEAEVQIDRLITGRLQ 1000
SLQTYVTQQLIRAAEIRASANLAATKMSECVLGQSKRVDFCGKGYHLMSF 1050
PQSAPHGVVFLHVTYVPAQEKNFTTAPAICHDGKAHFPREGVFVSNGTHW 1100
FVTQRNFYEPQIITTDNTFVSGNCDVVIGIVNNTVYDPLQPELDSFKEEL 1150
DKYFKNHTSPDVDLGDISGINASVVNIQKEIDRLNEVAKNLNESLIDLQE 1200
LGKYEQYIKWPWYIWLGFIAGLIAIVMVTIMLCCMTSCCSCLKGCCSCGS 1250
CCKFDEDDSEPVLKGVKLHYT*
 **SEQ ID NO: 10** S4800-Q5-F:
TAGATTAGATccacctGAAGCTGAAGTACAAATAGATAGATTAATAAC
**SEQ ID NO: 11** S4800-Q5-R: GATAATATATCATTTAATACAGAAGATATAG
**SEQ ID NO: 12** S3910-Q5-F: gcctctTCTGTAGCTAGCCAATCTATAATAG
**SEQ ID NO: 13** S3910-Q5-R: tctagaTGGAGAATTAGTTTGAGTTTGATAAG,
**SEQ ID NO: 14** S3690-Q5-F: TTATATCAAGgaGTAAATTGTACTGAAGTAC
**SEQ ID NO: 15** S3690-Q5-R: TACAGCTACTTGATTAGAAG
**SEQIDNO: 16:** Vaccinia virus Synthetic early/late promoter:
AAAAATTGAAATTTTATTTTTTTTTTTTTGGAATATAA
**SEQ ID NO: 17:** >GreenLantern_WR_CD 
ATGGTCAGCAAGGGGGAGGAGCTCTTCACCGGGGTCGTCCCCATCCTCGTCGAGCTCGACGGGG
ACGTCAACGGGCACAAGTTCAGCGTCCGGGGGGAGGGGGAGGGGGACGCCACCAACGGGAAGCT
CACCCTCAAGTTCATCTGCACCACCGGGAAGCTCCCCGTCCCCTGGCCCACCCTCGTCACCACC
CTCGGGTACGGGGTCGCCTGCTTCGCCCGGTACCCCGACCACATGAAGCAGCACGACTTCTTCA
AGAGCGCCATGCCCGAGGGGTACGTCCAGGAGCGGACCATCAGCTTCAAGGACGACGGGACCTA
CAAGACGCGGGCCGAGGTCAAGTTCGAGGGGGACACCCTCGTCAACCGGATCGTCCTCAAGGGG
ATCGACTTCAAGGAGGACGGGAACATCCTCGGGCACAAGCTCGAGTACAACTTCAACAGCCACA
AGGTCTACATCACCGCCGACAAGCAGAAGAACGGGATCAAGGCCAACTTCAAGACCCGGCACAA
CGTCGAGGACGGGGGGGTCCAGCTCGCCGACCACTACCAGCAGAACACCCCCATCGGGGACGGG
CCCGTCCTCCTCCCCGACAACCACTACCTCAGCCACCAGAGCAAGCTCAGCAAGGACCCCAACG
AGAAGCGGGACCACATGGTCCTCAAGGAGCGGGTCACCGCCGCCGGGATCACCCACGACATGGA
CGAGCTCTACAAGTAA
 **SEQ ID NO: 18:** >GreenLantern_WR_OP 
ATGGTATCTAAAGGAGAAGAATTATTTACTGGAGTAGTACCAATATTAGTAGAATTAGATGGAG
ATGTAAATGGACATAAATTTTCTGTAAGAGGAGAAGGAGAAGGAGATGCTACTAATGGAAAATT
AACTTTAAAATTTATATGTACTACTGGAAAATTACCAGTACCATGGCCAACTTTAGTAACTACT
TTAGGATATGGAGTAGCTTGTTTTGCTAGATATCCAGATCATATGAAACAACATGATTTCTTTA
AATCTGCTATGCCAGAAGGATATGTACAAGAAAGAACTATATCTTTTAAAGATGATGGAACTTA
TAAAACTAGAGCTGAAGTAAAATTTGAAGGAGATACTTTAGTAAATAGAATAGTATTAAAAGGA
ATAGATTTTAAAGAAGATGGAAATATATTAGGACATAAATTAGAATATAATTTTAATTCTCATA
AAGTATATATAACTGCTGATAAACAAAAAAATGGAATAAAAGCTAATTTTAAAACTAGACATAA
TGTAGAAGATGGAGGAGTACAATTAGCTGATCATTATCAACAAAATACTCCAATAGGAGATGGA
CCAGTATTATTACCAGATAATCATTATTTATCTCATCAATCTAAATTATCTAAAGATCCAAATG
AAAAAAGAGATCATATGGTATTAAAAGAAAGAGTAACTGCTGCTGGAATAACTCATGATATGGA
TGAATTATATAAATAA
 **SEQ ID NO: 19:** >GreenLantern_HS_CD 
ATGGTATCGAAAGGTGAAGAACTATTTACGGGTGTAGTACCGATACTAGTAGAACTAGATGGTG
ATGTAAATGGTCATAAATTTTCGGTACGTGGTGAAGGTGAAGGTGATGCGACGAATGGTAAACT
AACGCTAAAATTTATATGTACGACGGGTAAACTACCGGTACCGTGGCCGACGCTAGTAACGACG
CTAGGTTATGGTGTAGCGTGTTTTGCGCGTTATCCGGATCATATGAAACAACATGATTTCTTTA
AATCGGCGATGCCGGAAGGTTATGTACAAGAACGTACGATATCGTTTAAAGATGATGGTACGTA
TAAAACGCGTGCGGAAGTAAAATTTGAAGGTGATACGCTAGTAAATCGTATAGTACTAAAAGGT
ATAGATTTTAAAGAAGATGGTAATATACTAGGTCATAAACTAGAATATAATTTTAATTCGCATA
AAGTATATATAACGGCGGATAAACAAAAAAATGGTATAAAAGCGAATTTTAAAACGCGTCATAA
TGTAGAAGATGGTGGTGTACAACTAGCGGATCATTATCAACAAAATACGCCGATAGGTGATGGT
CCGGTACTACTACCGGATAATCATTATCTATCGCATCAATCGAAACTATCGAAAGATCCGAATG
AAAAACGTGATCATATGGTACTAAAAGAACGTGTAACGGCGGCGGGTATAACGCATGATATGGA
TGAACTATATAAATAA
 **SEQ ID NO: 20:** >GreenLantern_HS_OP 
ATGGTGAGCAAGGGCGAGGAGCTGTTCACCGGCGTGGTGCCCATCCTGGTGGAGCTGGACGGCG
ACGTGAACGGCCACAAGTTCAGCGTGAGAGGCGAGGGCGAGGGCGACGCCACCAACGGCAAGCT
GACCCTGAAGTTCATCTGCACCACCGGCAAGCTGCCCGTGCCCTGGCCCACCCTGGTGACCACC
CTGGGCTACGGCGTGGCCTGCTTCGCCAGATACCCCGACCACATGAAGCAGCACGACTTCTTCA
AGAGCGCCATGCCCGAGGGCTACGTGCAGGAGAGAACCATCAGCTTCAAGGACGACGGCACCTA
CAAGACCAGAGCCGAGGTGAAGTTCGAGGGCGACACCCTGGTGAACAGAATCGTGCTGAAGGGC
ATCGACTTCAAGGAGGACGGCAACATCCTGGGCCACAAGCTGGAGTACAACTTCAACAGCCACA
AGGTGTACATCACCGCCGACAAGCAGAAGAACGGCATCAAGGCCAACTTCAAGACCAGACACAA
CGTGGAGGACGGCGGCGTGCAGCTGGCCGACCACTACCAGCAGAACACCCCCATCGGCGACGGC
CCCGTGCTGCTGCCCGACAACCACTACCTGAGCCACCAGAGCAAGCTGAGCAAGGACCCCAACG
AGAAGAGAGACCACATGGTGCTGAAGGAGAGAGTGACCGCCGCCGGCATCACCCACGACATGGA
CGAGCTGTACAAGTAA
 **SEQ ID NO: 21**: >GreenLantern_VV_OP_IDT 
ATGGTGAGTAAGGGTGAGGAGCTTTTTACTGGTGTGGTCCCAATCCTAGTAGAGCTTGACGGTG
ATGTGAATGGTCATAAGTTTTCTGTGCGAGGTGAGGGAGAAGGTGATGCGACGAATGGTAAGCT
AACTCTAAAGTTCATATGCACTACGGGTAAGTTGCCCGTACCATGGCCCACACTTGTGACGACC
TTGGGTTACGGTGTCGCATGTTTCGCCCGATATCCGGACCATATGAAACAACATGATTTCTTCA
AATCTGCAATGCCAGAAGGATACGTTCAAGAAAGAACGATCTCGTTCAAAGACGATGGTACTTA
CAAAACAAGAGCAGAGGTTAAGTTTGAGGGAGATACGTTAGTGAATCGAATCGTATTAAAGGGT
ATAGACTTCAAAGAGGACGGAAACATATTAGGTCACAAACTTGAGTACAACTTCAACTCTCATA
AAGTCTACATCACCGCAGATAAACAAAAAAACGGAATTAAGGCAAATTTTAAAACAAGACATAA
CGTCGAAGATGGTGGTGTGCAACTTGCAGATCACTATCAACAGAACACTCCAATAGGAGACGGA
CCCGTATTGTTGCCAGATAATCACTATTTGTCTCACCAGTCGAAGCTATCCAAGGACCCCAATG
AGAAGCGTGACCATATGGTGTTGAAAGAGCGAGTCACTGCTGCTGGAATAACCCATGATATGGA
TGAGCTTTACAAGTAA
 **SEQ ID NO: 22**: >GreenLantern_HS_OP_IDT 
ATGGTATCCAAAGGCGAGGAGTTGTTCACAGGGGTGGTCCCTATACTGGTCGAACTGGACGGGG
ATGTAAATGGACACAAATTCAGTGTGAGAGGAGAAGGGGAGGGCGACGCGACCAACGGAAAGCT
CACACTCAAATTCATCTGTACAACCGGCAAATTGCCTGTTCCGTGGCCCACGCTTGTCACGACC
CTCGGATACGGTGTAGCATGTTTTGCAAGATATCCTGACCACATGAAACAACACGACTTCTTCA
AATCTGCCATGCCGGAGGGGTATGTCCAGGAGCGCACAATCAGCTTCAAGGACGATGGGACATA
TAAAACTAGAGCCGAAGTCAAGTTTGAGGGTGACACCCTCGTAAACCGGATCGTTCTGAAAGGC
ATTGATTTTAAGGAGGACGGCAATATCTTGGGTCATAAGTTGGAGTACAATTTTAACTCTCACA
AAGTGTACATTACTGCTGACAAACAGAAGAACGGGATTAAGGCGAACTTTAAGACAAGGCACAA
CGTCGAGGACGGCGGAGTGCAGCTCGCGGACCACTATCAACAAAACACTCCAATAGGCGATGGT
CCCGTCCTTCTTCCTGATAATCATTACCTTAGCCATCAAAGTAAACTTTCTAAGGACCCAAATG
AGAAGAGAGATCATATGGTGCTGAAGGAAAGGGTTACCGCCGCGGGAATTACGCACGACATGGA
TGAGCTGTATAAATAA
 **SEQ ID NO: 23:** Sequence of MVA transfer plasmid, including Sdg gene sequence (in bold). 
GGGCGAATTGGGCCCGACGTCGCATGCGATAAAGTTTCGAAACAGCAAAAAAAATGTAAATATT
GTATCTCTAATCGTGGAGATGATGATAGTTTAAGCATTAATCTATTTATTCCGACTATTAACAA
GTCTATATATATTATTATCGGTTTACGGATGAAAAATTTTTGGAAGCCTAAATTCGAAATAGAA
 
TAATGTTTTTATATTATACATGTTCTAAAAGAATAATCGATACAGTTTAAGTGAAAGCTAGAGA
GGGGTTTTTAAATGGTCATCGGTTTAGTCATATTCGTGTCTGTGGCGGCCGCCATCGTCGGTGT
GTTGTCTAACGTATTGGACATGTTTATGTACGTAGAAGAAAATAATGAAGAGGATGCTAGAATC
AAGGAGGAGCAAGAACTACTGTTGCTATATTGATACATAATTGAAAATCTACCAACTTAAATAC
ACCGCCTATAAATTTACAATGAAACACAGATTGTATTCTGAAGGATTGAGTATTAGTAATGATT
TAAACTCGATAATCGGTCAACAATCTACAATGGATACGGATATAGAAATAGACGAAGATGACAT
CATGGAACTTCTTAATATATTGACTGAGTTAGGTTGTGATGTCGACTTTGATGAAAATTTTAGC
GATATAGCCGATGATATTCTAGAATCGTTGATAGAACAGGATGTATAAGTTTTTATGTTAACTA
AATGTGGCCATTTGCACCGGTACCTGCGGGAGCAAAATGTAGGCTGGTAGAAACACTACCAGAA
AATATGGATTTTAGATCCGATCATTTAACAACATTTGAATGTTTTAACGAAATTATCACTCTAG
CTAAGAAATATATATACATAGCATCTTTTTGTTGTAATCCTCTGAGTACGACTAGGGGAGCGCT
TATTTTTGATAAACTAAAAGAGGCATCTGAAAAAGGGATTAAAATAATAGTTTTGCTAGATGAA
CGAGGGAAAAGAAATCTGGGAGAGCTACAAAGTCACTGCCCGGATATAAATTTTATAACCGTTA
ATATAGATAAAAAAAATAATGTGGGACTACTACTCGGTTGTTTTTGGGTGTCAGATAATGAAAG
ATGTTATGTAGGAAACGCGTCATTTACTGGAGGATCTATACATACGATTAAAACGTTAGGTGTA
TATTCTGATTATCCCCCGCTGGCCACAGATCTTCGTAGAAGATTTGATACTTTTAAAGCCTTTA
ATAGCGCAAAAAATTCATGGTTGAATTTATGCTCTGCGGCTTGTTGTCTGCCAGTTAGCACTGC
GTATCATATTAAGAATCCTATAGGTGGAGTGTTCTTTACTGATTCTCCGGAACACCTATTGGGA
TATTCTAGAGATCTAGACACTGATGTAGTTATTGATAAACTCAAGTCGGCTAAGACTAGTATAG
ATATTGAACATTTGGCCATAGTTCCCACTACACGTGTCGACGGTAATAGCTACTATTGGCCCGA
CATTTACAACTCCATTATAGAAGCAGCCATTAATAGAGGAGTTAAGATCAGACTTCTAGTTGGT
AATTGGGATAAGAACGACGTATATTCTATGGCAACCGCCAGAAGTCTAGACGCGTTGTGTGTTC
AAAATGATCTATCTGTGAAGGTTTTCACTATTCAGAATAATACAAAATTGTTGATAGTCGACGA
CGAATATGTTCATATCACTTCGGCAAATTTCGACGGAACCCATTACCAAAATCACGGATTCGTC
AGTTTTAATAGTATAGATAAACAGCTTGTAAGCGAGGCTAAAAAAATATTTGAGAGAGATTGGG
TGAGCTCACATTCAAAGAGTCTTAAGATATAACTCGAGAAAAATTGAAATTTTATTTTTTTTTT
TTTGGAATATAAATGTTTGTATTTTTAGTATTATTACCATTAGTATCTTCTCAATGTGTAAATT
TAACTACTAGAACTCAATTGCCACCAGCTTATACTAATTCTTTTACTAGAGGAGTATATTATCC
AGATAAAGTATTTAGATCTTCTGTATTACATTCTACTCAAGATTTATTTTTACCATTTTTTTCT
AATGTAACTTGGTTTCATGCTATACATGTATCTGGAACTAATGGAACTAAAAGATTTGATAATC
CAGTATTACCATTTAATGATGGAGTATATTTTGCTTCTACTGAAAAATCTAATATAATAAGAGG
ATGGATATTTGGAACTACTTTAGATTCTAAAACTCAATCTTTATTAATAGTAAATAATGCTACT
AATGTAGTAATAAAAGTATGTGAATTTCAATTTTGTAATGATCCATTTTTAGGAGTATATTATC
ATAAAAATAATAAATCTTGGATGGAATCTGAATTTAGAGTATATTCTTCTGCTAATAATTGTAC
TTTTGAATATGTATCTCAACCATTTTTAATGGATTTAGAAGGAAAACAAGGAAATTTTAAAAAT
TTAAGAGAATTTGTATTTAAAAATATAGATGGATATTTTAAAATATATTCTAAACATACTCCAA
TAAATTTAGTAAGAGATTTACCACAAGGATTTTCTGCTTTAGAACCATTAGTAGATTTACCAAT
AGGAATAAATATAACTAGATTTCAAACTTTATTAGCTTTACATAGATCTTATTTAACTCCAGGA
GATTCTTCTTCTGGATGGACTGCTGGAGCTGCTGCTTATTATGTAGGATATTTACAACCAAGAA
CTTTTTTATTAAAATATAATGAAAATGGAACTATAACTGATGCTGTAGATTGTGCTTTAGATCC
ATTATCTGAAACTAAATGTACTTTAAAATCTTTTACTGTAGAAAAAGGAATATATCAAACTTCT
AATTTTAGAGTACAACCAACTGAATCTATAGTAAGATTTCCAAATATAACTAATTTATGTCCAT
TTGGAGAAGTATTTAATGCTACTAGATTTGCTTCTGTATATGCTTGGAATAGAAAAAGAATATC
TAATTGTGTAGCTGATTATTCTGTATTATATAATTCTGCTTCTTTTTCTACTTTTAAATGTTAT
GGAGTATCTCCAACTAAATTAAATGATTTATGTTTTACTAATGTATATGCTGATTCTTTTGTAA
TAAGAGGAGATGAAGTAAGACAAATAGCTCCAGGACAAACTGGAAAAATAGCTGATTATAATTA
TAAATTACCAGATGATTTTACTGGATGTGTAATAGCTTGGAATAGTAATAATTTAGATTCTAAA
GTAGGAGGAAATTATAATTATTTATATAGATTATTTAGAAAATCTAATTTAAAACCATTTGAAA
GAGATATATCTACTGAAATATATCAAGCTGGATCTACTCCATGTAATGGAGTAGAAGGATTTAA
 
TTGTTATTTTCCATTACAATCTTATGGATTTCAACCAACTAATGGAGTAGGATATCAACCATAT
AGAGTAGTAGTATTATCTTTTGAATTATTACATGCTCCAGCTACTGTATGTGGACCAAAAAAAT
CTACTAATTTAGTAAAAAATAAATGTGTAAATTTTAATTTTAATGGATTAACTGGAACTGGAGT
ATTAACTGAATCTAATAAAAAATTTTTACCATTTCAACAATTTGGAAGAGATATAGCTGATACT
ACTGATGCTGTAAGAGATCCACAAACTTTAGAAATATTAGATATAACTCCATGTTCTTTTGGAG
GAGTATCTGTAATAACTCCAGGAACTAATACTTCTAATCAAGTAGCTGTATTATATCAAGGAGT
AAATTGTACTGAAGTACCAGTAGCTATACATGCTGATCAATTAACTCCAACTTGGAGAGTATAT
TCTACTGGATCTAATGTATTTCAAACTAGAGCTGGATGTTTAATAGGAGCTGAACATGTAAATA
ATTCTTATGAATGTGATATACCAATAGGAGCTGGAATATGTGCTTCTTATCAAACTCAAACTAA
TTCTCCAAGAAGAGCTAGATCTGTAGCTAGCCAATCTATAATAGCTTATACTATGTCTTTAGGA
GCTGAAAATTCTGTAGCTTATTCTAATAATTCTATAGCTATACCAACTAATTTTACTATATCTG
TAACTACTGAAATATTACCAGTATCTATGACTAAAACTTCTGTAGATTGTACTATGTATATATG
TGGAGATTCTACTGAATGTTCTAATTTATTATTACAATATGGATCTTTTTGTACTCAATTAAAT
AGAGCTTTAACTGGAATAGCTGTAGAACAAGATAAAAATACTCAAGAAGTATTTGCTCAAGTAA
AACAAATATATAAAACTCCACCAATAAAAGATTTTGGAGGATTTAATTTTTCTCAAATATTACC
AGATCCATCTAAACCATCTAAAAGATCTTTTATAGAAGATTTATTATTTAATAAAGTAACTTTA
GCTGATGCTGGATTTATAAAACAATATGGAGATTGTTTAGGAGATATAGCTGCTAGAGATTTAA
TATGTGCTCAAAAATTTAATGGATTAACTGTATTACCACCATTATTAACTGATGAAATGATAGC
TCAATATACTTCTGCTTTATTAGCTGGAACTATAACTTCTGGATGGACTTTTGGAGCTGGAGCT
GCTTTACAAATACCATTTGCTATGCAAATGGCTTATAGATTTAATGGAATAGGAGTAACTCAAA
ATGTATTATATGAAAATCAAAAATTAATAGCTAATCAATTTAATTCTGCTATAGGAAAAATACA
AGATTCTTTATCTTCTACTGCTTCTGCTTTAGGAAAATTACAAGATGTAGTAAATCAAAATGCT
CAAGCATTAAATACTTTAGTAAAACAATTATCTTCTAATTTTGGAGCTATATCTTCTGTATTAA
ATGATATATTATCTAGATTAGATAAAGTAGAAGCTGAAGTACAAATAGATAGATTAATAACTGG
AAGATTACAATCTTTACAAACTTATGTAACTCAACAATTAATAAGAGCTGCTGAAATAAGAGCT
TCTGCTAATTTAGCTGCTACTAAAATGTCTGAATGTGTATTAGGACAATCTAAAAGAGTAGATT
TTTGTGGAAAAGGATATCATTTAATGTCTTTTCCACAATCTGCTCCACATGGAGTAGTATTTTT
ACATGTAACTTATGTACCAGCTCAAGAAAAAAATTTTACTACTGCTCCAGCTATATGTCATGAT
GGAAAAGCTCATTTTCCAAGAGAAGGAGTATTTGTATCTAATGGAACTCATTGGTTTGTAACTC
AAAGAAATTTTTATGAACCACAAATAATAACTACTGATAATACTTTTGTATCTGGAAATTGTGA
TGTAGTAATAGGAATAGTAAATAATACTGTATATGATCCATTACAACCAGAATTAGATTCTTTT
AAAGAAGAATTAGATAAATATTTTAAAAATCATACTTCTCCAGATGTAGATTTAGGAGATATAT
CTGGAATAAATGCTTCTGTAGTAAATATACAAAAAGAAATAGATAGATTAAATGAAGTAGCTAA
AAATTTAAATGAATCTTTAATAGATTTACAAGAATTAGGAAAATATGAACAATATATAAAATGG
CCATGGTATATATGGTTAGGATTTATAGCTGGATTAATAGCTATAGTAATGGTAACTATAATGT
TATGTTGTATGACTTCTTGTTGTTCTTGTTTAAAAGGATGTTGTTCTTGTGGATCTTGTTGTAA
ATTTGATGAAGATGATTCTGAACCAGTATTAAAAGGAGTAAAATTACATTATACTTAATTAATA
TCAAGCTTTTTTTTATTGGATCCGGAGAGCTCGGGTATCTAGCCACAGTAAATCGTTAAAAATT
TAAAAAATAGAAACGTATAGAACGCCATCATGTTAAACAGGATACAAACCTTGATGAAAACAGC
TAACAATTATGAAACTATTGAGATATTGCGTAACTATTTAAGACTGTATATCATTTTGGCACGA
AATGAAGAAGGTCATGGTATACTAATATACGATGATAACATAGATAGTGTTATGTCGATGATGA
ATATTACAATATTAGAAGTTATAGGATTGACGACTCATTGCACAAAATTAAGATCATCGCCTCC
AATTCCTATGTCTAGATTGTTTATGGACGAAATAGATCATGAGTCATATTATTCTCCAAAAACT
TCAGATTATCCGTTGATCGATATTATACGAAAGCGTTCTCACGAACAGGGAGATATAGCACTGG
CTTTAGAACGATACGGTATTGAGAATACAGATTCCATATCAGAAATTAATGAATGGTTGTCGTC
AAAAGGTTTAGCATGTTATAGATTTGTAAAATTTAACGATTATAGGAAACAGATGTATCGTAAG
TTCTCTAGGTGTACTATAGTTGACAGTATGATAATAGGGCATATAGGTCATCATTATATTTGGA
TTAAAAATTTAGAAACATATACGCGTCCCGAAATTGATGTGTTACCGTTTGATATTAAATACAT
ATCTAGAGATGCATAGCTTGAGTATTCTATAGTGTCACCTAAATAGCTTGGCGTAATCATGGTC
 
ATAGCTGTTTCCTGTGTGAAATTGTTATCCGCTCACAATTCCACACAACATACGAGCCGGAAGC
ATAAAGTGTAAAGCCTGGGGTGCCTAATGAGTGAGCTAACTCACATTAATTGCGTTGCGCTCAC
TGCCCGCTTTCCAGTCGGGAAACCTGTCGTGCCAGCTGCATTAATGAATCGGCCAACGCGCGGG
GAGAGGCGGTTTGCGTATTGGGCGCTCTTCCGCTTCCTCGCTCACTGACTCGCTGCGCTCGGTC
GTTCGGCTGCGGCGAGCGGTATCAGCTCACTCAAAGGCGGTAATACGGTTATCCACAGAATCAG
GGGATAACGCAGGAAAGAACATGTGAGCAAAAGGCCAGCAAAAGGCCAGGAACCGTAAAAAGGC
CGCGTTGCTGGCGTTTTTCGATAGGCTCCGCCCCCCTGACGAGCATCACAAAAATCGACGCTCA
AGTCAGAGGTGGCGAAACCCGACAGGACTATAAAGATACCAGGCGTTTCCCCCTGGAAGCTCCC
TCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCC
AAGCTGGGCTGTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACTGGTAACAGGAT
TAGCAGAGCGAGGTATGTAGGCGGTGCTACAGAGTTCTTGAAGTGGTGGCCTAACTACGGCTAC
ACTAGAAGGACAGTATTTGGTATCTGCGCTCTGCTGAAGCCAGTTACCTTCGGAAAAAGAGTTG
GTAGCTCTTGATCCGGCAAACAAACCACCGCTGGTAGCGGTGGTTTTTTTGTTTGCAAGCAGCA
GATTACGCGCAGAAAAAAAGGATCTCAAGAAGATCCTTTGATCTTTTCTACGGGGTCTGACGCT
CAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTC
TGACAGTTACCAATGCTTAATCAGTGAGGCACCTATCTCAGCGATCTGTCTATTTCGTTCATCC
ATAGTTGCCTGACTCCCCGTCGTGTAGATAACTACGATACGGGAGGGCTTACCATCTGGCCCCA
GTGCTGCAATGATACCGCGAGACCCACGCTCACCGGCTCCAGATTTATCAGCAATAAACCAGCC
AGCCGGAAGGGCCGAGCGCAGAAGTGGTCCTGCAACTTTATCCGCCTCCATCCAGTCTATTAAT
TGTTGCCGGGAAGCTAGAGTAAGTAGTTCGCCAGTTAATAGTTTGCGCAACGTTGTTGGCATTG
CTACAGGCATCGTGGTGTCACGCTCGTCGTTTGGTATGGCTTCATTCAGCTCCGGTTCCCAACG
ATCAAGGCGAGTTACATGATCCCCCATGTTGTGCAAAAAAGCGGTTAGCTCCTTCGGTCCTCCG
ATCGTTGTCAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCAAGTCATT
CTGAGAATACCGCGCCCGGCGACCGAGTTGCTCTTGCCCGGCGTCAATACGGGATAATAGTGTA
TGACATAGCAGAACTTTAAAAGTGCTCATCATTGGAAAACGTTCTTCGGGGCGAAAACTCTCAA
GGATCTTACCGCTGTTGAGATCCAGTTCGATGTAACCCACTCGTGCACCCAACTGATCTTCAGC
ATCTTTTACTTTCACCAGCGTTTCTGGGTGAGCAAAAACAGGAAGGCAAAATGCCGCAAAAAAG
GGAATAAGGGCGACACGGAAATGTTGAATACTCATACTCTTCCTTTTTCAATATTATTGAAGCA
TTTATCAGGGTTATTGTCTCATGAGCGGATACATATTTGAATGTATTTAGAAAAATAAACAAAT
AGGGGTTCCGCGCACATTTCCCCGAAAAGTGCCACCTGTATGCGGTGTGAAATACCGCACAGAT
GCGTAAGGAGAAAATACCGCATCAGGCGAAATTGTAAACGTTAATATTTTGTTAAAATTCGCGT
TAAATATTTGTTAAATCAGCTCATTTTTTAACCAATAGGCCGAAATCGGCAAAATCCCTTATAA
ATCAAAAGAATAGACCGAGATAGGGTTGAGTGTTGTTCCAGTTTGGAACAAGAGTCCACTATTA
AAGAACGTGGACTCCAACGTCAAAGGGCGAAAAACCGTCTATCAGGGCGATGGCCCACTACGTG
AACCATCACCCAAATCAAGTTTTTTGCGGTCGAGGTGCCGTAAAGCTCTAAATCGGAACCCTAA
AGGGAGCCCCCGATTTAGAGCTTGACGGGGAAAGCCGGCGAACGTGGCGAGAAAGGAAGGGAAG
AAAGCGAAAGGAGCGGGCGCTAGGGCGCTGGCAAGTGTAGCGGTCACGCTGCGCGTAACCACCA
CACCCGCCGCGCTTAATGCGCCGCTACAGGGCGCGTCCATTCGCCATTCAGGCTGCGCAACTGT
TGGGAAGGGCGATCGGTGCGGGCCTCTTCGCTATTACGCCAGCTGGCGAAAGGGGGATGTGCTG
CAAGGCGATTAAGTTGGGTAACGCCAGGGTTTTCCCAGTCACGACGTTGTAAAACGACGGCCAG
TGAATTGTAATACGACTCACTATA
 **SEQ ID NO: 24:** Sequence of MVA transfer plasmid, including Spf gene sequence (in bold). 
GGGCGAATTGGGCCCGACGTCGCATGCGATAAAGTTTCGAAACAGCAAAAAAATGTAAATATT
GTATCTCTAATCGTGGAGATGATGATAGTTTAAGCATTAATCTATTTATTCCGACTATTAACAA
GTCTATATATATTATTATCGGTTTACGGATGAAAAATTTTTGGAAGCCTAAATTCGAAATAGAA
TAATGTTTTTATATTATACATGTTCTAAAAGAATAATCGATACAGTTTAAGTGAAAGCTAGAGA
GGGGTTTTTAAATGGTCATCGGTTTAGTCATATTCGTGTCTGTGGCGGCCGCCATCGTCGGTGT
GTTGTCTAACGTATTGGACATGTTTATGTACGTAGAAGAAAATAATGAAGAGGATGCTAGAATC
AAGGAGGAGCAAGAACTACTGTTGCTATATTGATACATAATTGAAAATCTACCAACTTAAATAC
ACCGCCTATAAATTTACAATGAAACACAGATTGTATTCTGAAGGATTGAGTATTAGTAATGATT
TAAACTCGATAATCGGTCAACAATCTACAATGGATACGGATATAGAAATAGACGAAGATGACAT
CATGGAACTTCTTAATATATTGACTGAGTTAGGTTGTGATGTCGACTTTGATGAAAATTTTAGC
GATATAGCCGATGATATTCTAGAATCGTTGATAGAACAGGATGTATAAGTTTTTATGTTAACTA
AATGTGGCCATTTGCACCGGTACCTGCGGAGCAAAATGTAGGCTGGTAGAAACACTACCAGAA
AATATGGATTTTAGATCCGATCATTTAACAACATTTGAATGTTTTAACGAAATTATCACTCTAG
CTAAGAAATATATATACATAGCATCTTTTTGTTGTAATCCTCTGAGTACGACTAGGGGAGCGCT
TATTTTTGATAAACTAAAAGAGGCATCTGAAAAAGGGATTAAAATAATAGTTTTGCTAGATGAA
CGAGGGAAAAGAAATCTGGGAGAGCTACAAAGTCACTGCCCGGATATAAATTTTATAACCGTTA
ATATAGATAAAAAAAATAATGTGGGACTACTACTCGGTTGTTTTTGGGTGTCAGATAATGAAAG
ATGTTATGTAGGAAACGCGTCATTTACTGGAGGATCTATACATACGATTAAAACGTTAGGTGTA
TATTCTGATTATCCCCCGCTGGCCACAGATCTTCGTAGAAGATTTGATACTTTTAAAGCCTTTA
ATAGCGCAAAAAATTCATGGTTGAATTTATGCTCTGCGGCTTGTTGTCTGCCAGTTAGCACTGC
GTATCATATTAAGAATCCTATAGGTGGAGTGTTCTTTACTGATTCTCCGGAACACCTATTGGGA
TATTCTAGAGATCTAGACACTGATGTAGTTATTGATAAACTCAAGTCGGCTAAGACTAGTATAG
ATATTGAACATTTGGCCATAGTTCCCACTACACGTGTCGACGGTAATAGCTACTATTGGCCCGA
CATTTACAACTCCATTATAGAAGCAGCCATTAATAGAGGAGTTAAGATCAGACTTCTAGTTGGT
AATTGGGATAAGAACGACGTATATTCTATGGCAACCGCCAGAAGTCTAGACGCGTTGTGTGTTC
AAAATGATCTATCTGTGAAGGTTTTCACTATTCAGAATAATACAAAATTGTTGATAGTCGACGA
CGAATATGTTCATATCACTTCGGCAAATTTCGACGGAACCCATTACCAAAATCACGGATTCGTC
AGTTTAATAGTATAGATAAACAGCTTGTAAGCGAGGCTAAAAAAATATTTGAGAGAGATTGGG
TGAGCTCACATTCAAAGAGTCTTAAGATATAACTCGAGAAAAATTGAAATTTTATTTTTTTTT
TTTGGAATATAAATGTTTGTATTTTTAGTATTATTACCATTAGTATCTTCTCAATGTGTAAATT
TAACTACTAGAACTCAATTGCCACCAGCTTATACTAATTCTTTTACTAGAGGAGTATATTATCC
AGATAAAGTATTTAGATCTTCTGTATTACATTCTACTCAAGATTTATTTTTACCATTTTTTTCT
AATGTAACTTGGTTTCATGCTATACATGTATCTGGAACTAATGGAACTAAAAGATTTGATAATC
CAGTATTACCATTTAATGATGGAGTATATTTTGCTTCTACTGAAAAATCTAATATAATAAGAGG
ATGGATATTTGGAACTACTTTAGATTCTAAAACTCAATCTTTATTAATAGTAAATAATGCTACT
AATGTAGTAATAAAAGTATGTGAATTTCAATTTTGTAATGATCCATTTTTAGGAGTATATTATC
ATAAAAATAATAAATCTTGGATGGAATCTGAATTTAGAGTATATTCTTCTGCTAATAATTGTAC
TTTTGAATATGTATCTCAACCATTTTTAATGGATTTAGAAGGAAAACAAGGAAATTTTAAAAAT
TTAAGAGAATTTGTATTTAAAAATATAGATGGATATTTTAAAATATATTCTAAACATACTCCAA
TAAATTTAGTAAGAGATTTACCACAAGGATTTTCTGCTTTAGAACCATTAGTAGATTTACCAAT
AGGAATAAATATAACTAGATTTCAAACTTTATTAGCTTTACATAGATCTTATTTAACTCCAGGA
GATTCTTCTTCTGGATGGACTGCTGGAGCTGCTGCTTATTATGTAGGATATTTACAACCAAGAA
CTTTTTTATTAAAATATAATGAAAATGGAACTATAACTGATGCTGTAGATTGTGCTTTAGATCC
ATTATCTGAAACTAAATGTACTTTAAAATCTTTTACTGTAGAAAAAGGAATATATCAAACTTCT
AATTTTAGAGTACAACCAACTGAATCTATAGTAAGATTTCCAAATATAACTAATTTATGTCCAT
TTGGAGAAGTATTTAATGCTACTAGATTTGCTTCTGTATATGCTTGGAATAGAAAAAGAATATC
TAATTGTGTAGCTGATTATTCTGTATTATATAATTCTGCTTCTTTTTCTACTTTTAAATGTTAT
GGAGTATCTCCAACTAAATTAAATGATTTATGTTTTACTAATGTATATGCTGATTCTTTTGTAA
TAAGAGGAGATGAAGTAAGACAAATAGCTCCAGGACAAACTGGAAAAATAGCTGATTATAATTA
 
TAAATTACCAGATGATTTTACTGGATGTGTAATAGCTTGGAATAGTAATAATTTAGATTCTAAA
GTAGGAGGAAATTATAATTATTTATATAGATTATTTAGAAAATCTAATTTAAAACCATTTGAAA
GAGATATATCTACTGAAATATATCAAGCTGGATCTACTCCATGTAATGGAGTAGAAGGATTTAA
TTGTTATTTTCCATTACAATCTTATGGATTTCAACCAACTAATGGAGTAGGATATCAACCATAT
AGAGTAGTAGTATTATCTTTTGAATTATTACATGCTCCAGCTACTGTATGTGGACCAAAAAAAT
CTACTAATTTAGTAAAAAATAAATGTGTAAATTTTAATTTTAATGGATTAACTGGAACTGGAGT
ATTAACTGAATCTAATAAAAAATTTTTACCATTTCAACAATTTGGAAGAGATATAGCTGATACT
ACTGATGCTGTAAGAGATCCACAAACTTTAGAAATATTAGATATAACTCCATGTTCTTTTGGAG
GAGTATCTGTAATAACTCCAGGAACTAATACTTCTAATCAAGTAGCTGTATTATATCAAGGAGT
AAATTGTACTGAAGTACCAGTAGCTATACATGCTGATCAATTAACTCCAACTTGGAGAGTATAT
TCTACTGGATCTAATGTATTTCAAACTAGAGCTGGATGTTTAATAGGAGCTGAACATGTAAATA
ATTCTTATGAATGTGATATACCAATAGGAGCTGGAATATGTGCTTCTTATCAAACTCAAACTAA
TTCTCCATCTAGAGCCTCTTCTGTAGCTAGCCAATCTATAATAGCTTATACTATGTCTTTAGGA
GCTGAAAATTCTGTAGCTTATTCTAATAATTCTATAGCTATACCAACTAATTTTACTATATCTG
TAACTACTGAAATATTACCAGTATCTATGACTAAAACTTCTGTAGATTGTACTATGTATATATG
TGGAGATTCTACTGAATGTTCTAATTTATTATTACAATATGGATCTTTTTGTACTCAATTAAAT
AGAGCTTTAACTGGAATAGCTGTAGAACAAGATAAAAATACTCAAGAAGTATTTGCTCAAGTAA
AACAAATATATAAAACTCCACCAATAAAAGATTTTGGAGGATTTAATTTTTCTCAAATATTACC
AGATCCATCTAAACCATCTAAAAGATCTTTTATAGAAGATTTATTATTTAATAAAGTAACTTTA
GCTGATGCTGGATTTATAAAACAATATGGAGATTGTTTAGGAGATATAGCTGCTAGAGATTTAA
TATGTGCTCAAAAATTTAATGGATTAACTGTATTACCACCATTATTAACTGATGAAATGATAGC
TCAATATACTTCTGCTTTATTAGCTGGAACTATAACTTCTGGATGGACTTTTGGAGCTGGAGCT
GCTTTACAAATACCATTTGCTATGCAAATGGCTTATAGATTTAATGGAATAGGAGTAACTCAAA
ATGTATTATATGAAAATCAAAAATTAATAGCTAATCAATTTAATTCTGCTATAGGAAAAATACA
AGATTCTTTATCTTCTACTGCTTCTGCTTTAGGAAAATTACAAGATGTAGTAAATCAAAATGCT
CAAGCATTAAATACTTTAGTAAAACAATTATCTTCTAATTTTGGAGCTATATCTTCTGTATTAA
ATGATATATTATCTAGATTAGATCCACCTGAAGCTGAAGTACAAATAGATAGATTAATAACTGG
AAGATTACAATCTTTACAAACTTATGTAACTCAACAATTAATAAGAGCTGCTGAAATAAGAGCT
TCTGCTAATTTAGCTGCTACTAAAATGTCTGAATGTGTATTAGGACAATCTAAAAGAGTAGATT
TTTGTGGAAAAGGATATCATTTAATGTCTTTTCCACAATCTGCTCCACATGGAGTAGTATTTTT
ACATGTAACTTATGTACCAGCTCAAGAAAAAAATTTTACTACTGCTCCAGCTATATGTCATGAT
GGAAAAGCTCATTTTCCAAGAGAAGGAGTATTTGTATCTAATGGAACTCATTGGTTTGTAACTC
AAAGAAATTTTTATGAACCACAAATAATAACTACTGATAATACTTTTGTATCTGGGAATTGTGA
TGTAGTAATAGGAATAGTAAATAATACTGTATATGATCCATTACAACCAGAATTAGATTCTTTT
AAAGAAGAATTAGATAAATATTTTAAAAATCATACTTCTCCAGATGTAGATTTAGGAGATATAT
CTGGAATAAATGCTTCTGTAGTAAATATACAAAAAGAAATAGATAGATTAAATGAAGTAGCTAA
AAATTTAAATGAATCTTTAATAGATTTACAAGAATTAGGAAAATATGAACAATATATAAAATGG
CCATGGTATATATGGTTAGGATTTATAGCTGGATTAATAGCTATAGTAATGGTAACTATAATGT
TATGTTGTATGACTTCTTGTTGTTCTTGTTTAAAAGGATGTTGTTCTTGTGGATCTTGTTGTAA
ATTTGATGAAGATGATTCTGAACCAGTATTAAAAGGAGTAAAATTACATTATACTTAATTAATA
TCAAGCTTTTTTTTATTGGATCCGGAGAGCTCGGGTATCTAGCCACAGTAAATCGTTAAAAATT
TAAAAAATAGAAACGTATAGAACGCCATCATGTTAAACAGGATACAAACCTTGATGAAAACAGC
TAACAATTATGAAACTATTGAGATATTGCGTAACTATTTAAGACTGTATATCATTTTGGCACGA
AATGAAGAAGGTCATGGTATACTAATATACGATGATAACATAGATAGTGTTATGTCGATGATGA
ATATTACAATATTAGAAGTTATAGGATTGACGACTCATTGCACAAAATTAAGATCATCGCCTCC
AATTCCTATGTCTAGATTGTTTATGGACGAAATAGATCATGAGTCATATTATTCTCCAAAAACT
TCAGATTATCCGTTGATCGATATTATACGAAAGCGTTCTCACGAACAGGGAGATATAGCACTGG
CTTTAGAACGATACGGTATTGAGAATACAGATTCCATATCAGAAATTAATGAATGGTTGTCGTC
AAAAGGTTTAGCATGTTATAGATTTGTAAAATTTAACGATTATAGGAAACAGATGTATCGTAAG
 
TTCTCTAGGTGTACTATAGTTGACAGTATGATAATAGGGCATATAGGTCATCATTATATTTGGA
TTAAAAATTTAGAAACATATACGCGTCCCGAAATTGATGTGTTACCGTTTGATATTAAATACAT
ATCTAGAGATGCATAGCTTGAGTATTCTATAGTGTCACCTAAATAGCTTGGCGTAATCATGGTC
ATAGCTGTTTCCTGTGTGAAATTGTTATCCGCTCACAATTCCACACAACATACGAGCCGGAAGC
ATAAAGTGTAAAGCCTGGGGTGCCTAATGAGTGAGCTAACTCACATTAATTGCGTTGCGCTCAC
TGCCCGCTTTCCAGTCGGGAAACCTGTCGTGCCAGCTGCATTAATGAATCGGCCAACGCGCGGG
GAGAGGCGGTTTGCGTATTGGGCGCTCTTCCGCTTCCTCGCTCACTGACTCGCTGCGCTCGGTC
GTTCGGCTGCGGCGAGCGGTATCAGCTCACTCAAAGGCGGTAATACGGTTATCCACAGAATCAG
GGGATAACGCAGGAAAGAACATGTGAGCAAAAGGCCAGCAAAAGGCCAGGAACCGTAAAAAGGC
CGCGTTGCTGGCGTTTTTCGATAGGCTCCGCCCCCCTGACGAGCATCACAAAAATCGACGCTCA
AGTCAGAGGTGGCGAAACCCGACAGGACTATAAAGATACCAGGCGTTTCCCCCTGGAAGCTCCC
TCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCC
AAGCTGGGCTGTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACTGGTAACAGGAT
TAGCAGAGCGAGGTATGTAGGCGGTGCTACAGAGTTCTTGAAGTGGTGGCCTAACTACGGCTAC
ACTAGAAGGACAGTATTTGGTATCTGCGCTCTGCTGAAGCCAGTTACCTTCGGAAAAAGAGTTG
GTAGCTCTTGATCCGGCAAACAAACCACCGCTGGTAGCGGTGGTTTTTTTGTTTGCAAGCAGCA
GATTACGCGCAGAAAAAAAGGATCTCAAGAAGATCCTTTGATCTTTTCTACGGGGTCTGACGCT
CAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTC
TGACAGTTACCAATGCTTAATCAGTGAGGCACCTATCTCAGCGATCTGTCTATTTCGTTCATCC
ATAGTTGCCTGACTCCCCGTCGTGTAGATAACTACGATACGGGAGGGCTTACCATCTGGCCCCA
GTGCTGCAATGATACCGCGAGACCCACGCTCACCGGCTCCAGATTTATCAGCAATAAACCAGCC
AGCCGGAAGGGCCGAGCGCAGAAGTGGTCCTGCAACTTTATCCGCCTCCATCCAGTCTATTAAT
TGTTGCCGGGAAGCTAGAGTAAGTAGTTCGCCAGTTAATAGTTTGCGCAACGTTGTTGGCATTG
CTACAGGCATCGTGGTGTCACGCTCGTCGTTTGGTATGGCTTCATTCAGCTCCGGTTCCCAACG
ATCAAGGCGAGTTACATGATCCCCCATGTTGTGCAAAAAAGCGGTTAGCTCCTTCGGTCCTCCG
ATCGTTGTCAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCAAGTCATT
CTGAGAATACCGCGCCCGGCGACCGAGTTGCTCTTGCCCGGCGTCAATACGGGATAATAGTGTA
TGACATAGCAGAACTTTAAAAGTGCTCATCATTGGAAAACGTTCTTCGGGGCGAAAACTCTCAA
GGATCTTACCGCTGTTGAGATCCAGTTCGATGTAACCCACTCGTGCACCCAACTGATCTTCAGC
ATCTTTTACTTTCACCAGCGTTTCTGGGTGAGCAAAAACAGGAAGGCAAAATGCCGCAAAAAAG
GGAATAAGGGCGACACGGAAATGTTGAATACTCATACTCTTCCTTTTTCAATATTATTGAAGCA
TTTATCAGGGTTATTGTCTCATGAGCGGATACATATTTGAATGTATTTAGAAAAATAAACAAAT
AGGGGTTCCGCGCACATTTCCCCGAAAAGTGCCACCTGTATGCGGTGTGAAATACCGCACAGAT
GCGTAAGGAGAAAATACCGCATCAGGCGAAATTGTAAACGTTAATATTTTGTTAAAATTCGCGT
TAAATATTTGTTAAATCAGCTCATTTTTTAACCAATAGGCCGAAATCGGCAAAATCCCTTATAA
ATCAAAAGAATAGACCGAGATAGGGTTGAGTGTTGTTCCAGTTTGGAACAAGAGTCCACTATTA
AAGAACGTGGACTCCAACGTCAAAGGGCGAAAAACCGTCTATCAGGGCGATGGCCCACTACGTG
AACCATCACCCAAATCAAGTTTTTTGCGGTCGAGGTGCCGTAAAGCTCTAAATCGGAACCCTAA
AGGGAGCCCCCGATTTAGAGCTTGACGGGGAAAGCCGGCGAACGTGGCGAGAAAGGAAGGGAAG
AAAGCGAAAGGAGCGGGCGCTAGGGCGCTGGCAAGTGTAGCGGTCACGCTGCGCGTAACCACCA
CACCCGCCGCGCTTAATGCGCCGCTACAGGGCGCGTCCATTCGCCATTCAGGCTGCGCAACTGT
TGGGAAGGGCGATCGGTGCGGGCCTCTTCGCTATTACGCCAGCTGGCGAAAGGGGGATGTGCTG
CAAGGCGATTAAGTTGGGTAACGCCAGGGTTTTCCCAGTCACGACGTTGTAAAACGACGGCCAG
TGAATTGTAATACGACTCACTATA
 **SEQ ID NO: 25:** Sequence of MVA transfer plasmid, including Spfx gene sequence (in bold). 
GGGCGAATTGGGCCCGACGTCGCATGCGATAAAGTTTCGAAACAGCAAAAAAAATGTAAATATT
GTATCTCTAATCGTGGAGATGATGATAGTTTAAGCATTAATCTATTTATTCCGACTATTAACAA
GTCTATATATATTATTATCGGTTTACGGATGAAAAATTTTTGGAAGCCTAAATTCGAAATAGAA
TAATGTTTTTATATTATACATGTTCTAAAAGAATAATCGATACAGTTTAAGTGAAAGCTAGAGA
GGGGTTTTTAAATGGTCATCGGTTTAGTCATATTCGTGTCTGTGGCGGCCGCCATCGTCGGTGT
GTTGTCTAACGTATTGGACATGTTTATGTACGTAGAAGAAAATAATGAAGAGGATGCTAGAATC
AAGGAGGAGCAAGAACTACTGTTGCTATATTGATACATAATTGAAAATCTACCAACTTAAATAC
ACCGCCTATAAATTTACAATGAAACACAGATTGTATTCTGAAGGATTGAGTATTAGTAATGATT
TAAACTCGATAATCGGTCAACAATCTACAATGGATACGGATATAGAAATAGACGAAGATGACAT
CATGGAACTTCTTAATATATTGACTGAGTTAGGTTGTGATGTCGACTTTGATGAAAATTTTAGC
GATATAGCCGATGATATTCTAGAATCGTTGATAGAACAGGATGTATAAGTTTTTATGTTAACTA
AATGTGGCCATTTGCACCGGTACCTGCGGGAGCAAAATGTAGGCTGGTAGAAACACTACCAGAA
AATATGGATTTTAGATCCGATCATTTAACAACATTTGAATGTTTTAACGAAATTATCACTCTAG
CTAAGAAATATATATACATAGCATCTTTTTGTTGTAATCCTCTGAGTACGACTAGGGGAGCGCT
TATTTTTGATAAACTAAAAGAGGCATCTGAAAAAGGGATTAAAATAATAGTTTTGCTAGATGAA
CGAGGGAAAAGAAATCTGGGAGAGCTACAAAGTCACTGCCCGGATATAAATTTTATAACCGTTA
ATATAGATAAAAAAAATAATGTGGGACTACTACTCGGTTGTTTTTGGGTGTCAGATAATGAAAG
ATGTTATGTAGGAAACGCGTCATTTACTGGAGGATCTATACATACGATTAAAACGTTAGGTGTA
TATTCTGATTATCCCCCGCTGGCCACAGATCTTCGTAGAAGATTTGATACTTTTAAAGCCTTTA
ATAGCGCAAAAAATTCATGGTTGAATTTATGCTCTGCGGCTTGTTGTCTGCCAGTTAGCACTGC
GTATCATATTAAGAATCCTATAGGTGGAGTGTTCTTTACTGATTCTCCGGAACACCTATTGGGA
TATTCTAGAGATCTAGACACTGATGTAGTTATTGATAAACTCAAGTCGGCTAAGACTAGTATAG
ATATTGAACATTTGGCCATAGTTCCCACTACACGTGTCGACGGTAATAGCTACTATTGGCCCGA
CATTTACAACTCCATTATAGAAGCAGCCATTAATAGAGGAGTTAAGATCAGACTTCTAGTTGGT
AATTGGGATAAGAACGACGTATATTCTATGGCAACCGCCAGAAGTCTAGACGCGTTGTGTGTTC
AAAATGATCTATCTGTGAAGGTTTTCACTATTCAGAATAATACAAAATTGTTGATAGTCGACGA
CGAATATGTTCATATCACTTCGGCAAATTTCGACGGAACCCATTACCAAAATCACGGATTCGTC
AGTTTTAATAGTATAGATAAACAGCTTGTAAGCGAGGCTAAAAAAATATTTGAGAGAGATTGGG
TGAGCTCACATTCAAAGAGTCTTAAGATATAACTCGAGAAAAATTGAAATTTTATTTTTTTTTT
TTTGGAATATAAATGTTTGTATTTTTAGTATTATTACCATTAGTATCTTCTCAATGTGTAAATT
TAACTACTAGAACTCAATTGCCCCCGGCCTATACTAATTCGTTTACTCGTGGTGTTTACTATCC
TGATAAAGTGTTTCGATCGTCTGTGCTTCATTCGACTCAAGATCTATTTTTGCCCTTCTTTAGT
AATGTTACATGGTTCCATGCTATACATGTATCGGGTACAAACGGTACGAAGCGTTTCGACAACC
CAGTCTTACCATTCAATGACGGAGTTTATTTTGCCAGTACCGAGAAATCAAATATCATCAGAGG
ATGGATTTTTGGTACTACTTTGGATAGTAAGACTCAGTCTCTATTAATCGTCAATAACGCAACG
AACGTTGTAATCAAAGTATGCGAGTTTCAATTCTGCAACGATCCATTTCTAGGTGTTTACTACC
ACAAGAACAATAAGTCGTGGATGGAAAGTGAGTTCCGTGTGTACTCTTCTGCCAATAACTGCAC
TTTCGAATATGTTTCACAGCCGTTTTTAATGGACTTAGAGGGTAAACAAGGTAATTTCAAAAAC
TTGAGAGAATTTGTCTTCAAAAATATTGATGGTTATTTCAAAATATATTCAAAGCATACGCCCA
TTAATCTTGTTCGAGACCTACCGCAAGGTTTTTCCGCATTAGAACCCTTAGTTGATCTACCCAT
TGGAATCAACATTACAAGATTCCAGACATTGTTAGCTTTACACCGTTCGTATCTTACACCCGGT
GATTCGTCATCAGGATGGACCGCTGGAGCTGCTGCCTATTATGTCGGTTACTTGCAACCGCGTA
CATTCCTATTGAAGTACAATGAAAATGGAACTATTACAGACGCCGTGGACTGTGCCTTGGATCC
TTTATCTGAAACTAAGTGTACATTAAAATCTTTTACAGTAGAAAAAGGTATTTACCAAACGTCC
AACTTCCGTGTCCAACCCACGGAATCGATAGTTAGATTTCCGAATATTACGAACCTATGCCCAT
 
TTGGAGAAGTCTTTAACGCCACGCGTTTTGCCTCAGTTTACGCTTGGAATCGTAAGCGTATTAG
TAACTGCGTAGCGGATTATTCCGTACTATACAATTCCGCGTCCTTTTCGACTTTCAAATGTTAC
GGAGTGTCGCCCACTAAACTTAACGATCTTTGTTTCACCAACGTGTACGCAGACTCATTTGTAA
TCAGAGGAGACGAAGTGCGACAAATTGCGCCTGGTCAGACCGGAAAAATCGCAGATTACAATTA
TAAACTTCCGGACGATTTCACTGGTTGTGTCATTGCATGGAATTCCAACAACCTAGACAGTAAA
GTAGGAGGTAACTATAATTACCTATATAGATTATTCCGAAAATCAAATTTAAAACCATTTGAAC
GTGACATCAGTACAGAGATATACCAAGCGGGATCTACGCCGTGCAACGGAGTAGAGGGATTTAA
TTGCTATTTCCCGTTACAATCGTACGGATTCCAACCCACGAACGGAGTAGGTTACCAACCGTAC
CGTGTCGTGGTCCTTAGTTTTGAGTTGTTGCACGCACCTGCCACCGTGTGCGGTCCCAAAAAGT
CAACAAACTTAGTCAAAAACAAATGTGTCAACTTCAATTTTAATGGACTAACAGGAACCGGAGT
CTTGACTGAATCAAACAAAAAATTCCTTCCTTTCCAACAATTCGGTCGTGATATTGCGGATACG
ACAGATGCGGTACGAGACCCGCAAACACTTGAGATATTGGATATAACCCCTTGCTCATTCGGTG
GTGTTTCGGTAATCACCCCTGGAACAAACACGAGTAACCAGGTAGCCGTACTTTACCAGGGTGT
GAACTGTACAGAGGTGCCAGTCGCGATACATGCGGATCAGCTAACGCCAACGTGGAGAGTATAT
TCTACCGGAAGTAATGTGTTCCAAACCAGAGCCGGATGTCTTATCGGTGCAGAACATGTAAACA
ATAGTTACGAGTGTGATATCCCCATTGGAGCGGGTATTTGCGCAAGTTATCAAACCCAAACAAA
CTCTCCCTCTAGAGCCTCTAGTGTTGCCTCTCAGAGTATCATTGCTTACACGATGTCGTTGGGA
GCAGAAAATTCTGTTGCATATTCTAACAATTCGATAGCAATTCCAACCAATTTCACGATATCCG
TAACCACCGAGATCTTGCCAGTTAGTATGACTAAAACATCGGTTGACTGCACAATGTATATATG
CGGTGATTCTACTGAGTGTTCAAACTTGCTTCTTCAGTATGGTTCGTTTTGTACTCAGCTAAAT
CGAGCCTTGACGGGAATAGCCGTTGAACAAGACAAAAATACGCAGGAGGTGTTTGCTCAAGTCA
AACAAATTTACAAGACCCCACCTATCAAGGATTTCGGTGGTTTTAACTTTTCCCAAATATTACC
AGACCCATCAAAGCCAAGTAAGAGATCCTTCATAGAGGACCTATTGTTTAATAAGGTAACGTTG
GCGGACGCGGGTTTTATTAAGCAATACGGTGACTGCTTAGGTGATATAGCTGCCAGAGACTTAA
TCTGCGCTCAGAAATTCAACGGATTAACCGTTTTACCACCATTATTGACGGACGAGATGATCGC
GCAGTATACCTCGGCTTTGCTAGCTGGTACGATTACATCCGGTTGGACTTTCGGTGCCGGTGCA
GCCCTACAGATACCATTTGCGATGCAAATGGCCTATCGTTTTAATGGAATCGGTGTCACCCAGA
ACGTGCTTTACGAGAACCAGAAGTTAATTGCTAATCAATTCAACTCTGCAATCGGAAAGATTCA
AGACTCATTATCTAGTACAGCCTCCGCCTTAGGAAAGTTACAGGACGTTGTGAACCAGAATGCT
CAGGCGTTAAATACCTTGGTCAAACAACTATCCTCAAATTTCGGTGCGATATCTTCAGTCTTGA
ATGATATCCTAAGTCGACTAGATCCACCTGAGGCTGAAGTACAGATTGACCGACTTATCACAGG
TCGTCTACAAAGTTTGCAGACGTATGTGACCCAACAGCTAATAAGAGCGGCTGAGATACGTGCT
TCTGCCAATTTAGCAGCTACAAAAATGTCAGAGTGTGTGCTAGGACAATCGAAACGAGTTGACT
TCTGTGGAAAGGGTTACCACTTGATGTCATTCCCCCAAAGTGCGCCTCATGGTGTGGTTTTCTT
GCATGTAACGTACGTACCCGCGCAAGAAAAGAATTTTACTACCGCCCCGGCCATTTGTCATGAC
GGAAAAGCTCATTTTCCGCGTGAAGGTGTATTCGTATCCAACGGTACGCACTGGTTTGTGACCC
AAAGAAACTTTTATGAACCGCAGATTATAACGACCGACAATACGTTCGTGTCTGGAAATTGCGA
CGTTGTAATTGGAATCGTAAACAATACAGTTTACGACCCATTGCAACCCGAGCTAGATAGTTTT
AAGGAAGAACTTGACAAGTATTTTAAGAACCACACGTCACCGGACGTCGACTTGGGAGATATTT
CGGGAATTAACGCTAGTGTTGTGAATATCCAGAAAGAAATAGATCGACTAAATGAAGTGGCCAA
GAACCTTAACGAGTCCCTAATTGACTTACAGGAATTGGGTAAGTATGAACAATACATAAAATGG
CCATGGTATATATGGTTAGGATTTATAGCTGGATTAATAGCTATAGTAATGGTTACTATCATGT
TATGCTGCATGACCTCCTGCTGTTCATGTTTAAAAGGATGTTGCTCCTGCGGTTCGTGTTGTAA
GTTCGATGAAGATGACTCCGAACCTGTCTTGAAGGGAGTGAAATTGCATTACACTTAATTAATA
TCAAGCTTTAACCATGGAGGCCTAAATAAATAATTTTTATGGATCCGGAGAGCTCGGGTATCTA
GCCACAGTAAATCGTTAAAAATTTAAAAAATAGAAACGTATAGAACGCCATCATGTTAAACAGG
ATACAAACCTTGATGAAAACAGCTAACAATTATGAAACTATTGAGATATTGCGTAACTATTTAA
GACTGTATATCATTTTGGCACGAAATGAAGAAGGTCATGGTATACTAATATACGATGATAACAT
AGATAGTGTTATGTCGATGATGAATATTACAATATTAGAAGTTATAGGATTGACGACTCATTGC
 
ACAAAATTAAGATCATCGCCTCCAATTCCTATGTCTAGATTGTTTATGGACGAAATAGATCATG
AGTCATATTATTCTCCAAAAACTTCAGATTATCCGTTGATCGATATTATACGAAAGCGTTCTCA
CGAACAGGGAGATATAGCACTGGCTTTAGAACGATACGGTATTGAGAATACAGATTCCATATCA
GAAATTAATGAATGGTTGTCGTCAAAAGGTTTAGCATGTTATAGATTTGTAAAATTTAACGATT
ATAGGAAACAGATGTATCGTAAGTTCTCTAGGTGTACTATAGTTGACAGTATGATAATAGGGCA
TATAGGTCATCATTATATTTGGATTAAAAATTTAGAAACATATACGCGTCCCGAAATTGATGTG
TTACCGTTTGATATTAAATACATATCTAGAGATGCATAGCTTGAGTATTCTATAGTGTCACCTA
AATAGCTTGGCGTAATCATGGTCATAGCTGTTTCCTGTGTGAAATTGTTATCCGCTCACAATTC
CACACAACATACGAGCCGGAAGCATAAAGTGTAAAGCCTGGGGTGCCTAATGAGTGAGCTAACT
CACATTAATTGCGTTGCGCTCACTGCCCGCTTTCCAGTCGGGAAACCTGTCGTGCCAGCTGCAT
TAATGAATCGGCCAACGCGCGGGGAGAGGCGGTTTGCGTATTGGGCGCTCTTCCGCTTCCTCGC
TCACTGACTCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAAGGCCAGCAA
AAGGCCAGGAACCGTAAAAAGGCCGCGTTGCTGGCGTTTTTCGATAGGCTCCGCCCCCCTGACG
AGCATCACAAAAATCGACGCTCAAGTCAGAGGTGGCGAAACCCGACAGGACTATAAAGATACCA
GGCGTTTCCCCCTGGAAGCTCCCTCGTGCGCTCTCCTGTTCCGACCCTGCCGCTTACCGGATAC
CTGTCCGCCTTTCTCCCTTCGGGAAGCGTGGCGCTTTCTCATAGCTCACGCTGTAGGTATCTCA
GTTCGGTGTAGGTCGTTCGCTCCAAGCTGGGCTGTGTGCACGAACCCCCCGTTCAGCCCGACCG
CTGCGCCTTATCCGGTAACTATCGTCTTGAGTCCAACCCGGTAAGACACGACTTATCGCCACTG
GCAGCAGCCACTGGTAACAGGATTAGCAGAGCGAGGTATGTAGGCGGTGCTACAGAGTTCTTGA
AGTGGTGGCCTAACTACGGCTACACTAGAAGGACAGTATTTGGTATCTGCGCTCTGCTGAAGCC
AGTTACCTTCGGAAAAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCAAGAAGATCCTTTGA
TCTTTTCTACGGGGTCTGACGCTCAGTGGAACGAAAACTCACGTTAAGGGATTTTGGTCATGAG
ATTATCAAAAAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCACCTATCTCAG
CGATCTGTCTATTTCGTTCATCCATAGTTGCCTGACTCCCCGTCGTGTAGATAACTACGATACG
GGAGGGCTTACCATCTGGCCCCAGTGCTGCAATGATACCGCGAGACCCACGCTCACCGGCTCCA
GATTTATCAGCAATAAACCAGCCAGCCGGAAGGGCCGAGCGCAGAAGTGGTCCTGCAACTTTAT
CCGCCTCCATCCAGTCTATTAATTGTTGCCGGGAAGCTAGAGTAAGTAGTTCGCCAGTTAATAG
TTTGCGCAACGTTGTTGGCATTGCTACAGGCATCGTGGTGTCACGCTCGTCGTTTGGTATGGCT
TCATTCAGCTCCGGTTCCCAACGATCAAGGCGAGTTACATGATCCCCCATGTTGTGCAAAAAAG
CGGTTAGCTCCTTCGGTCCTCCGATCGTTGTCAGAAGTAAGTTGGCCGCAGTGTTATCACTCAT
GGTTATGGCAGCACTGCATAATTCTCTTACTGTCATGCCATCCGTAAGATGCTTTTCTGTGACT
GGTGAGTACTCAACCAAGTCATTCTGAGAATACCGCGCCCGGCGACCGAGTTGCTCTTGCCCGG
CGTCAATACGGGATAATAGTGTATGACATAGCAGAACTTTAAAAGTGCTCATCATTGGAAAACG
TTCTTCGGGGCGAAAACTCTCAAGGATCTTACCGCTGTTGAGATCCAGTTCGATGTAACCCACT
CGTGCACCCAACTGATCTTCAGCATCTTTTACTTTCACCAGCGTTTCTGGGTGAGCAAAAACAG
GAAGGCAAAATGCCGCAAAAAAGGGAATAAGGGCGACACGGAAATGTTGAATACTCATACTCTT
CCTTTTTCAATATTATTGAAGCATTTATCAGGGTTATTGTCTCATGAGCGGATACATATTTGAA
TGTATTTAGAAAAATAAACAAATAGGGGTTCCGCGCACATTTCCCCGAAAAGTGCCACCTGTAT
GCGGTGTGAAATACCGCACAGATGCGTAAGGAGAAAATACCGCATCAGGCGAAATTGTAAACGT
TAATATTTTGTTAAAATTCGCGTTAAATATTTGTTAAATCAGCTCATTTTTTAACCAATAGGCC
GAAATCGGCAAAATCCCTTATAAATCAAAAGAATAGACCGAGATAGGGTTGAGTGTTGTTCCAG
TTTGGAACAAGAGTCCACTATTAAAGAACGTGGACTCCAACGTCAAAGGGCGAAAAACCGTCTA
TCAGGGCGATGGCCCACTACGTGAACCATCACCCAAATCAAGTTTTTTGCGGTCGAGGTGCCGT
AAAGCTCTAAATCGGAACCCTAAAGGGAGCCCCCGATTTAGAGCTTGACGGGGAAAGCCGGCGA
ACGTGGCGAGAAAGGAAGGGAAGAAAGCGAAAGGAGCGGGCGCTAGGGCGCTGGCAAGTGTAGC
GGTCACGCTGCGCGTAACCACCACACCCGCCGCGCTTAATGCGCCGCTACAGGGCGCGTCCATT
 
CGCCATTCAGGCTGCGCAACTGTTGGGAAGGGCGATCGGTGCGGGCCTCTTCGCTATTACGCCA
GCTGGCGAAAGGGGGATGTGCTGCAAGGCGATTAAGTTGGGTAACGCCAGGGTTTTCCCAGTCA
CGACGTTGTAAAACGACGGCCAGTGAATTGTAATACGACTCACTATA
 **SEQ ID NO: 26:** Sequence of MVA transfer plasmid, including Spf-o (omicron) gene sequence (in bold). 
GGGCGAATTGGGCCCGACGTCGCATGCGATAAAGTTTCGAAACAGCAAAAAAAATGTAAATATT
GTATCTCTAATCGTGGAGATGATGATAGTTTAAGCATTAATCTATTTATTCCGACTATTAACAA
GTCTATATATATTATTATCGGTTTACGGATGAAAAATTTTTGGAAGCCTAAATTCGAAATAGAA
TAATGTTTTTATATTATACATGTTCTAAAAGAATAATCGATACAGTTTAAGTGAAAGCTAGAGA
GGGGTTTTTAAATGGTCATCGGTTTAGTCATATTCGTGTCTGTGGCGGCCGCCATCGTCGGTGT
GTTGTCTAACGTATTGGACATGTTTATGTACGTAGAAGAAAATAATGAAGAGGATGCTAGAATC
AAGGAGGAGCAAGAACTACTGTTGCTATATTGATACATAATTGAAAATCTACCAACTTAAATAC
ACCGCCTATAAATTTACAATGAAACACAGATTGTATTCTGAAGGATTGAGTATTAGTAATGATT
TAAACTCGATAATCGGTCAACAATCTACAATGGATACGGATATAGAAATAGACGAAGATGACAT
CATGGAACTTCTTAATATATTGACTGAGTTAGGTTGTGATGTCGACTTTGATGAAAATTTTAGC
GATATAGCCGATGATATTCTAGAATCGTTGATAGAACAGGATGTATAAGTTTTTATGTTAACTA
AATGTGGCCATTTGCACCGGTACCTGCGGGAGCAAAATGTAGGCTGGTAGAAACACTACCAGAA
AATATGGATTTTAGATCCGATCATTTAACAACATTTGAATGTTTTAACGAAATTATCACTCTAG
CTAAGAAATATATATACATAGCATCTTTTTGTTGTAATCCTCTGAGTACGACTAGGGGAGCGCT
TATTTTTGATAAACTAAAAGAGGCATCTGAAAAAGGGATTAAAATAATAGTTTTGCTAGATGAA
CGAGGGAAAAGAAATCTGGGAGAGCTACAAAGTCACTGCCCGGATATAAATTTTATAACCGTTA
ATATAGATAAAAAAAATAATGTGGGACTACTACTCGGTTGTTTTTGGGTGTCAGATAATGAAAG
ATGTTATGTAGGAAACGCGTCATTTACTGGAGGATCTATACATACGATTAAAACGTTAGGTGTA
TATTCTGATTATCCCCCGCTGGCCACAGATCTTCGTAGAAGATTTGATACTTTTAAAGCCTTTA
ATAGCGCAAAAAATTCATGGTTGAATTTATGCTCTGCGGCTTGTTGTCTGCCAGTTAGCACTGC
GTATCATATTAAGAATCCTATAGGTGGAGTGTTCTTTACTGATTCTCCGGAACACCTATTGGGA
TATTCTAGAGATCTAGACACTGATGTAGTTATTGATAAACTCAAGTCGGCTAAGACTAGTATAG
ATATTGAACATTTGGCCATAGTTCCCACTACACGTGTCGACGGTAATAGCTACTATTGGCCCGA
CATTTACAACTCCATTATAGAAGCAGCCATTAATAGAGGAGTTAAGATCAGACTTCTAGTTGGT
AATTGGGATAAGAACGACGTATATTCTATGGCAACCGCCAGAAGTCTAGACGCGTTGTGTGTTC
AAAATGATCTATCTGTGAAGGTTTTCACTATTCAGAATAATACAAAATTGTTGATAGTCGACGA
CGAATATGTTCATATCACTTCGGCAAATTTCGACGGAACCCATTACCAAAATCACGGATTCGTC
AGTTTTAATAGTATAGATAAACAGCTTGTAAGCGAGGCTAAAAAAATATTTGAGAGAGATTGGG
TGAGCTCACATTCAAAGAGTCTTAAGATATAACTCGAGAAAAATTGAAATTTTATTTTTTTTTT
TTTGGAATATAAATGTTTGTATTTTTAGTATTATTACCATTAGTATCTTCTCAATGTGTAAATT
TAATAACTAGAACTCAATCTTATACTAATTCTTTTACTAGAGGAGTATATTATCCAGATAAAGT
ATTTAGATCTTCTGTATTACATTCTACTCAAGATTTATTTTTACCATTCTTCTCTAATGTAACT
TGGTTTCATGCTATACATGTATCTGGAACTAATGGAACTAAAAGATTTGATAATCCAGTATTAC
CATTTAATGATGGAGTATATTTTGCTTCTACTGAAAAATCTAATATAATAAGAGGATGGATATT
TGGAACTACTTTAGATTCTAAAACTCAATCTTTATTAATAGTAAATAATGCTACTAATGTAGTA
ATAAAAGTATGTGAATTTCAATTTTGTAATGATCCATTTTTAGATGTATATTATCATAAAAATA
ATAAATCTTGGATGGAATCTGAATTTAGAGTATATTCTTCTGCTAATAATTGTACTTTTGAATA
TGTATCTCAACCATTTTTAATGGATTTAGAAGGAAAACAAGGAAATTTTAAAAATTTAAGAGAA
TTTGTATTTAAAAATATAGATGGATATTTTAAAATATATTCTAAACATACTCCAATAAATTTAG
GAAGAGATTTACCACAAGGATTTTCTGCTTTAGAACCATTAGTAGATTTACCAATAGGAATAAA
TATAACTAGATTTCAAACTTTATTAGCTTTACATAGATCTTATTTAACTCCAGGAGATTCTTCT
TCTGGATGGACTGCTGGAGCTGCTGCTTATTATGTAGGATATTTACAACCAAGAACTTTCTTAT
TAAAATATAATGAAAATGGAACTATAACTGATGCTGTAGATTGTGCTTTAGATCCATTATCTGA
AACTAAATGTACTTTAAAATCTTTTACTGTAGAAAAAGGAATATATCAAACTTCTAATTTTAGA
 
GTACAACCAACTGAATCTATAGTAAGATTTCCAAATATAACTAATTTATGTCCATTTGATGAAG
TATTTAATGCTACTAGATTTGCTTCTGTATATGCTTGGAATAGAAAAAGAATATCTAATTGTGT
AGCTGATTATTCTGTATTATATAATTTTGCTCCATTTTTTGCTTTTAAATGTTATGGAGTATCT
CCAACTAAATTAAATGATTTATGTTTTACTAATGTATATGCTGATTCTTTTGTAATAAGAGGAA
ATGAAGTATCTCAAATAGCTCCAGGACAAACTGGAAATATAGCTGATTATAATTATAAATTACC
AGATGATTTTACTGGATGTGTAATAGCTTGGAATTCTAATAAATTAGATTCTAAAGTAGGAGGA
AATTATAATTATTTATATAGATTATTTAGAAAATCTAATTTAAAACCATTTGAAAGAGATATAT
CTACTGAAATATATCAAGCTGGAAATAAACCATGTAATGGAGTAGCTGGATTTAATTGTTATTT
TCCATTAAGATCTTATGGATTTAGACCAACTTATGGAGTAGGACATCAACCATATAGAGTAGTA
GTATTATCTTTTGAATTATTACATGCTCCAGCTACTGTATGTGGACCAAAAAAATCTACTAATT
TAGTAAAAAATAAATGTGTAAATTTTAATTTTAATGGATTAACTGGAACTGGAGTATTAACTGA
ATCTAATAAAAAATTTTTACCATTTCAACAATTTGGAAGAGATATAGCTGATACTACTGATGCT
GTAAGAGATCCACAAACTTTAGAAATATTAGATATAACTCCATGTTCTTTTGGAGGAGTATCTG
TAATAACTCCAGGAACTAATACTTCTAATCAAGTAGCTGTATTATATCAAGGAGTAAATTGTAC
TGAAGTACCAGTAGCTATACATGCTGATCAATTAACTCCAACTTGGAGAGTATATTCTACTGGA
TCTAATGTATTTCAAACTAGAGCTGGATGTTTAATAGGAGCTGAATATGTAAATAATTCTTATG
AATGTGATATACCAATAGGAGCTGGAATATGTGCTTCTTATCAAACTCAAACTAAATCTCATAG
TAGAGCTAGTTCTGTAGCTTCTCAATCTATAATAGCTTATACTATGTCTTTAGGAGCTGAAAAT
TCTGTAGCTTATTCTAATAATTCTATAGCTATACCAACTAATTTTACTATATCTGTAACTACTG
AAATATTACCAGTATCTATGACTAAAACTTCTGTAGATTGTACTATGTATATATGTGGAGATTC
TACTGAATGTTCTAATTTATTATTACAATATGGATCTTTCTGTACTCAATTAAAAAGAGCTTTA
ACTGGAATAGCTGTAGAACAAGATAAAAATACTCAAGAAGTATTTGCTCAAGTAAAACAAATAT
ATAAAACTCCACCAATAAAATATTTTGGAGGATTTAATTTCTCTCAAATATTACCAGATCCATC
TAAACCATCTAAAAGATCTTTTATAGAAGATTTATTATTTAATAAAGTAACTTTAGCTGATGCT
GGATTTATAAAACAATATGGAGATTGTTTAGGAGATATAGCTGCTAGAGATTTAATATGTGCTC
AAAAATTTAATGGATTAACTGTATTACCACCATTATTAACTGATGAAATGATAGCTCAATATAC
TTCTGCTTTATTAGCTGGAACTATAACTTCTGGATGGACTTTTGGAGCTGGAGCTGCTTTACAA
ATACCATTTGCTATGCAAATGGCTTATAGATTTAATGGAATAGGAGTAACTCAAAATGTATTAT
ATGAAAATCAAAAATTAATAGCTAATCAATTTAATTCTGCTATAGGAAAAATACAAGATTCTTT
ATCTTCTACTGCTTCTGCTTTAGGAAAATTACAAGATGTAGTAAATCATAATGCTCAAGCTTTA
AATACTTTAGTAAAACAATTATCTTCTAAATTTGGAGCTATATCTTCTGTATTAAATGATATAT
TATCTAGATTAGATCCACCAGAAGCTGAAGTACAAATAGATAGATTAATAACTGGAAGATTACA
ATCTTTACAAACTTATGTAACTCAACAATTAATAAGAGCTGCTGAAATAAGAGCTTCTGCTAAT
TTAGCTGCTACTAAAATGTCTGAATGTGTATTAGGACAATCTAAAAGAGTAGATTTCTGTGGAA
AAGGATATCATTTAATGTCTTTTCCACAATCTGCTCCACATGGAGTAGTATTTTTACATGTAAC
TTATGTACCAGCTCAAGAAAAAAATTTTACTACTGCTCCAGCTATATGTCATGATGGAAAAGCT
CATTTTCCAAGAGAAGGAGTATTTGTATCTAATGGAACTCATTGGTTTGTAACTCAAAGAAATT
TCTATGAACCACAAATAATAACTACTGATAATACTTTTGTATCTGGAAATTGTGATGTAGTAAT
AGGAATAGTAAATAATACTGTATATGATCCATTACAACCAGAATTAGATTCTTTTAAAGAAGAA
TTAGATAAATATTTTAAAAATCATACTTCTCCAGATGTAGATTTAGGAGATATATCTGGAATAA
ATGCTTCTGTAGTAAATATACAAAAAGAAATAGATAGATTAAATGAAGTAGCTAAAAATTTAAA
TGAATCTTTAATAGATTTACAAGAATTAGGAAAATATGAACAATATATAAAATGGCCATGGTAT
ATATGGTTAGGATTTATAGCTGGATTAATAGCTATAGTAATGGTAACTATAATGTTATGTTGTA
TGACTTCTTGTTGTTCTTGTTTAAAAGGATGTTGTTCTTGTGGATCTTGTTGTAAATTTGATGA
AGATGATTCTGAACCAGTATTAAAAGGAGTAAAATTACATTATACTTAATTAATTAATATCAAG
CTTTTTTTTATTGGATCCGGAGAGCTCGGGTATCTAGCCACAGTAAATCGTTAAAAATTTAAAA
AATAGAAACGTATAGAACGCCATCATGTTAAACAGGATACAAACCTTGATGAAAACAGCTAACA
ATTATGAAACTATTGAGATATTGCGTAACTATTTAAGACTGTATATCATTTTGGCACGAAATGA
AGAAGGTCATGGTATACTAATATACGATGATAACATAGATAGTGTTATGTCGATGATGAATATT
ACAATATTAGAAGTTATAGGATTGACGACTCATTGCACAAAATTAAGATCATCGCCTCCAATTC
CTATGTCTAGATTGTTTATGGACGAAATAGATCATGAGTCATATTATTCTCCAAAAACTTCAGA
TTATCCGTTGATCGATATTATACGAAAGCGTTCTCACGAACAGGGAGATATAGCACTGGCTTTA
GAACGATACGGTATTGAGAATACAGATTCCATATCAGAAATTAATGAATGGTTGTCGTCAAAAG
 
GTTTAGCATGTTATAGATTTGTAAAATTTAACGATTATAGGAAACAGATGTATCGTAAGTTCTC
TAGGTGTACTATAGTTGACAGTATGATAATAGGGCATATAGGTCATCATTATATTTGGATTAAA
AATTTAGAAACATATACGCGTCCCGAAATTGATGTGTTACCGTTTGATATTAAATACATATCTA
GAGATGCATAGCTTGAGTATTCTATAGTGTCACCTAAATAGCTTGGCGTAATCATGGTCATAGC
TGTTTCCTGTGTGAAATTGTTATCCGCTCACAATTCCACACAACATACGAGCCGGAAGCATAAA
GTGTAAAGCCTGGGGTGCCTAATGAGTGAGCTAACTCACATTAATTGCGTTGCGCTCACTGCCC
GCTTTCCAGTCGGGAAACCTGTCGTGCCAGCTGCATTAATGAATCGGCCAACGCGCGGGGAGAG
GCGGTTTGCGTATTGGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAATCAGGGGAT
AACGCAGGAAAGAACATGTGAGCAAAAGGCCAGCAAAAGGCCAGGAACCGTAAAAAGGCCGCGT
TGCTGGCGTTTTTCGATAGGCTCCGCCCCCCTGACGAGCATCACAAAAATCGACGCTCAAGTCA
GAGGTGGCGAAACCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCCCTTCGGGAAGCG
TGGCGCTTTCTCATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGATTAGCA
GAGCGAGGTATGTAGGCGGTGCTACAGAGTTCTTGAAGTGGTGGCCTAACTACGGCTACACTAG
AAGGACAGTATTTGGTATCTGCGCTCTGCTGAAGCCAGTTACCTTCGGAAAAAGAGTTGGTAGC
TCTTGATCCGGCAAACAAACCACCGCTGGTAGCGGTGGTTTTTTTGTTTGCAAGCAGCAGATTA
CGCGCAGAAAAAAAGGATCTCAAGAAGATCCTTTGATCTTTTCTACGGGGTCTGACGCTCAGTG
GAACGAAAACTCACGTTAAGGGATTTTGGTCATGAGATTATCAAAAAGGATCTTCACCTAGATC
CTTTTAAATTAAAAATGAAGTTTTAAATCAATCTAAAGTATATATGAGTAAACTTGGTCTGACA
GTTACCAATGCTTAATCAGTGAGGCACCTATCTCAGCGATCTGTCTATTTCGTTCATCCATAGT
TGCCTGACTCCCCGTCGTGTAGATAACTACGATACGGGAGGGCTTACCATCTGGCCCCAGTGCT
GCAATGATACCGCGAGACCCACGCTCACCGGCTCCAGATTTATCAGCAATAAACCAGCCAGCCG
GAAGGGCCGAGCGCAGAAGTGGTCCTGCAACTTTATCCGCCTCCATCCAGTCTATTAATTGTTG
CCGGGAAGCTAGAGTAAGTAGTTCGCCAGTTAATAGTTTGCGCAACGTTGTTGGCATTGCTACA
GGCATCGTGGTGTCACGCTCGTCGTTTGGTATGGCTTCATTCAGCTCCGGTTCCCAACGATCAA
GGCGAGTTACATGATCCCCCATGTTGTGCAAAAAAGCGGTTAGCTCCTTCGGTCCTCCGATCGT
TGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAG
AATACCGCGCCCGGCGACCGAGTTGCTCTTGCCCGGCGTCAATACGGGATAATAGTGTATGACA
TAGCAGAACTTTAAAAGTGCTCATCATTGGAAAACGTTCTTCGGGGCGAAAACTCTCAAGGATC
TTACCGCTGTTGAGATCCAGTTCGATGTAACCCACTCGTGCACCCAACTGATCTTCAGCATCTT
TTACTTTCACCAGCGTTTCTGGGTGAGCAAAAACAGGAAGGCAAAATGCCGCAAAAAAGGGAAT
AAGGGCGACACGGAAATGTTGAATACTCATACTCTTCCTTTTTCAATATTATTGAAGCATTTAT
CAGGGTTATTGTCTCATGAGCGGATACATATTTGAATGTATTTAGAAAAATAAACAAATAGGGG
TTCCGCGCACATTTCCCCGAAAAGTGCCACCTGTATGCGGTGTGAAATACCGCACAGATGCGTA
AGGAGAAAATACCGCATCAGGCGAAATTGTAAACGTTAATATTTTGTTAAAATTCGCGTTAAAT
ATTTGTTAAATCAGCTCATTTTTTAACCAATAGGCCGAAATCGGCAAAATCCCTTATAAATCAA
AAGAATAGACCGAGATAGGGTTGAGTGTTGTTCCAGTTTGGAACAAGAGTCCACTATTAAAGAA
CGTGGACTCCAACGTCAAAGGGCGAAAAACCGTCTATCAGGGCGATGGCCCACTACGTGAACCA
TCACCCAAATCAAGTTTTTTGCGGTCGAGGTGCCGTAAAGCTCTAAATCGGAACCCTAAAGGGA
GCCCCCGATTTAGAGCTTGACGGGGAAAGCCGGCGAACGTGGCGAGAAAGGAAGGGAAGAAAGC
GAAAGGAGCGGGCGCTAGGGCGCTGGCAAGTGTAGCGGTCACGCTGCGCGTAACCACCACACCC
GCCGCGCTTAATGCGCCGCTACAGGGCGCGTCCATTCGCCATTCAGGCTGCGCAACTGTTGGGA
AGGGCGATCGGTGCGGGCCTCTTCGCTATTACGCCAGCTGGCGAAAGGGGGATGTGCTGCAAGG
CGATTAAGTTGGGTAACGCCAGGGTTTTCCCAGTCACGACGTTGTAAAACGACGGCCAGTGAAT
TGTAATACGACTCACTATA
 **SEQ ID NO: 27 Nucleic acid sequence of S protein from MVA-Spf-o (Omicron)** 
ATGTTTGTATTTTTAGTATTATTACCATTAGTATCTTCTCAATGTGTAAATTTAATAACTAGAA
CTCAATCTTATACTAATTCTTTTACTAGAGGAGTATATTATCCAGATAAAGTATTTAGATCTTC
TGTATTACATTCTACTCAAGATTTATTTTTACCATTCTTCTCTAATGTAACTTGGTTTCATGCT
ATACATGTATCTGGAACTAATGGAACTAAAAGATTTGATAATCCAGTATTACCATTTAATGATG
GAGTATATTTTGCTTCTACTGAAAAATCTAATATAATAAGAGGATGGATATTTGGAACTACTTT
AGATTCTAAAACTCAATCTTTATTAATAGTAAATAATGCTACTAATGTAGTAATAAAAGTATGT
GAATTTCAATTTTGTAATGATCCATTTTTAGATGTATATTATCATAAAAATAATAAATCTTGGA
TGGAATCTGAATTTAGAGTATATTCTTCTGCTAATAATTGTACTTTTGAATATGTATCTCAACC
ATTTTTAATGGATTTAGAAGGAAAACAAGGAAATTTTAAAAATTTAAGAGAATTTGTATTTAAA
AATATAGATGGATATTTTAAAATATATTCTAAACATACTCCAATAAATTTAGGAAGAGATTTAC
CACAAGGATTTTCTGCTTTAGAACCATTAGTAGATTTACCAATAGGAATAAATATAACTAGATT
TCAAACTTTATTAGCTTTACATAGATCTTATTTAACTCCAGGAGATTCTTCTTCTGGATGGACT
GCTGGAGCTGCTGCTTATTATGTAGGATATTTACAACCAAGAACTTTCTTATTAAAATATAATG
AAAATGGAACTATAACTGATGCTGTAGATTGTGCTTTAGATCCATTATCTGAAACTAAATGTAC
TTTAAAATCTTTTACTGTAGAAAAAGGAATATATCAAACTTCTAATTTTAGAGTACAACCAACT
GAATCTATAGTAAGATTTCCAAATATAACTAATTTATGTCCATTTGATGAAGTATTTAATGCTA
CTAGATTTGCTTCTGTATATGCTTGGAATAGAAAAAGAATATCTAATTGTGTAGCTGATTATTC
TGTATTATATAATTTTGCTCCATTTTTTGCTTTTAAATGTTATGGAGTATCTCCAACTAAATTA
AATGATTTATGTTTTACTAATGTATATGCTGATTCTTTTGTAATAAGAGGAAATGAAGTATCTC
AAATAGCTCCAGGACAAACTGGAAATATAGCTGATTATAATTATAAATTACCAGATGATTTTAC
TGGATGTGTAATAGCTTGGAATTCTAATAAATTAGATTCTAAAGTAGGAGGAAATTATAATTAT
TTATATAGATTATTTAGAAAATCTAATTTAAAACCATTTGAAAGAGATATATCTACTGAAATAT
ATCAAGCTGGAAATAAACCATGTAATGGAGTAGCTGGATTTAATTGTTATTTTCCATTAAGATC
TTATGGATTTAGACCAACTTATGGAGTAGGACATCAACCATATAGAGTAGTAGTATTATCTTTT
GAATTATTACATGCTCCAGCTACTGTATGTGGACCAAAAAAATCTACTAATTTAGTAAAAAATA
AATGTGTAAATTTTAATTTTAATGGATTAACTGGAACTGGAGTATTAACTGAATCTAATAAAAA
ATTTTTACCATTTCAACAATTTGGAAGAGATATAGCTGATACTACTGATGCTGTAAGAGATCCA
CAAACTTTAGAAATATTAGATATAACTCCATGTTCTTTTGGAGGAGTATCTGTAATAACTCCAG
GAACTAATACTTCTAATCAAGTAGCTGTATTATATCAAGGAGTAAATTGTACTGAAGTACCAGT
AGCTATACATGCTGATCAATTAACTCCAACTTGGAGAGTATATTCTACTGGATCTAATGTATTT
CAAACTAGAGCTGGATGTTTAATAGGAGCTGAATATGTAAATAATTCTTATGAATGTGATATAC
CAATAGGAGCTGGAATATGTGCTTCTTATCAAACTCAAACTAAATCTCATAGTAGAGCTAGTTC
TGTAGCTTCTCAATCTATAATAGCTTATACTATGTCTTTAGGAGCTGAAAATTCTGTAGCTTAT
TCTAATAATTCTATAGCTATACCAACTAATTTTACTATATCTGTAACTACTGAAATATTACCAG
TATCTATGACTAAAACTTCTGTAGATTGTACTATGTATATATGTGGAGATTCTACTGAATGTTC
TAATTTATTATTACAATATGGATCTTTCTGTACTCAATTAAAAAGAGCTTTAACTGGAATAGCT
GTAGAACAAGATAAAAATACTCAAGAAGTATTTGCTCAAGTAAAACAAATATATAAAACTCCAC
CAATAAAATATTTTGGAGGATTTAATTTCTCTCAAATATTACCAGATCCATCTAAACCATCTAA
AAGATCTTTTATAGAAGATTTATTATTTAATAAAGTAACTTTAGCTGATGCTGGATTTATAAAA
CAATATGGAGATTGTTTAGGAGATATAGCTGCTAGAGATTTAATATGTGCTCAAAAATTTAATG
GATTAACTGTATTACCACCATTATTAACTGATGAAATGATAGCTCAATATACTTCTGCTTTATT
AGCTGGAACTATAACTTCTGGATGGACTTTTGGAGCTGGAGCTGCTTTACAAATACCATTTGCT
ATGCAAATGGCTTATAGATTTAATGGAATAGGAGTAACTCAAAATGTATTATATGAAAATCAAA
AATTAATAGCTAATCAATTTAATTCTGCTATAGGAAAAATACAAGATTCTTTATCTTCTACTGC
TTCTGCTTTAGGAAAATTACAAGATGTAGTAAATCATAATGCTCAAGCTTTAAATACTTTAGTA
AAACAATTATCTTCTAAATTTGGAGCTATATCTTCTGTATTAAATGATATATTATCTAGATTAG
ATCCACCAGAAGCTGAAGTACAAATAGATAGATTAATAACTGGAAGATTACAATCTTTACAAAC
TTATGTAACTCAACAATTAATAAGAGCTGCTGAAATAAGAGCTTCTGCTAATTTAGCTGCTACT
AAAATGTCTGAATGTGTATTAGGACAATCTAAAAGAGTAGATTTCTGTGGAAAAGGATATCATT
TAATGTCTTTTCCACAATCTGCTCCACATGGAGTAGTATTTTTACATGTAACTTATGTACCAGC
TCAAGAAAAAAATTTTACTACTGCTCCAGCTATATGTCATGATGGAAAAGCTCATTTTCCAAGA
 
GAAGGAGTATTTGTATCTAATGGAACTCATTGGTTTGTAACTCAAAGAAATTTCTATGAACCAC
AAATAATAACTACTGATAATACTTTTGTATCTGGAAATTGTGATGTAGTAATAGGAATAGTAAA
TAATACTGTATATGATCCATTACAACCAGAATTAGATTCTTTTAAAGAAGAATTAGATAAATAT
TTTAAAAATCATACTTCTCCAGATGTAGATTTAGGAGATATATCTGGAATAAATGCTTCTGTAG
TAAATATACAAAAAGAAATAGATAGATTAAATGAAGTAGCTAAAAATTTAAATGAATCTTTAAT
AGATTTACAAGAATTAGGAAAATATGAACAATATATAAAATGGCCATGGTATATATGGTTAGGA
TTTATAGCTGGATTAATAGCTATAGTAATGGTAACTATAATGTTATGTTGTATGACTTCTTGTT
GTTCTTGTTTAAAAGGATGTTGTTCTTGTGGATCTTGTTGTAAATTTGATGAAGATGATTCTGA
ACCAGTATTAAAAGGAGTAAAATTACATTATACTTAA
 **SEQ ID NO: 28 Amino acid sequence of S protein from MVA-Spf-o (Omicron)**

MFVFLVLLPLVSSQCVNLITRTQSYTNSFTRGVYYPDKVFRSSVLHSTQD 50
LFLPFFSNVTWFHAIHVSGTNGTKRFDNPVLPFNDGVYFASTEKSNIIRG 100
WIFGTTLDSKTQSLLIVNNATNVVIKVCEFQFCNDPFLDVYYHKNNKSWM 150
ESEFRVYSSANNCTFEYVSQPFLMDLEGKQGNFKNLREFVFKNIDGYFKI 200
YSKHTPINLGRDLPQGFSALEPLVDLPIGINITRFQTLLALHRSYLTPGD 250
SSSGWTAGAAAYYVGYLQPRTFLLKYNENGTITDAVDCALDPLSETKCTL 300
KSFTVEKGIYQTSNFRVQPTESIVRFPNITNLCPFDEVFNATRFASVYAW 350
NRKRISNCVADYSVLYNFAPFFAFKCYGVSPTKLNDLCFTNVYADSFVIR 400
GNEVSQIAPGQTGNIADYNYKLPDDFTGCVIAWNSNKLDSKVGGNYNYLY 450
RLFRKSNLKPFERDISTEIYQAGNKPCNGVAGFNCYFPLRSYGFRPTYGV 500
GHQPYRVVVLSFELLHAPATVCGPKKSTNLVKNKCVNFNFNGLTGTGVLT 550
ESNKKFLPFQQFGRDIADTTDAVRDPQTLEILDITPCSFGGVSVITPGTN 600
TSNQVAVLYQGVNCTEVPVAIHADQLTPTWRVYSTGSNVFQTRAGCLIGA 650
EYVNNSYECDIPIGAGICASYQTQTKSHSRASSVASQSIIAYTMSLGAEN 700
SVAYSNNSIAIPTNFTISVTTEILPVSMTKTSVDCTMYICGDSTECSNLL 750
LQYGSFCTQLKRALTGIAVEQDKNTQEVFAQVKQIYKTPPIKYFGGFNFS 800
QILPDPSKPSKRSFIEDLLFNKVTLADAGFIKQYGDCLGDIAARDLICAQ 850
KFNGLTVLPPLLTDEMIAQYTSALLAGTITSGWTFGAGAALQIPFAMQMA 900
YRFNGIGVTQNVLYENQKLIANQFNSAIGKIQDSLSSTASALGKLQDVVN 950
HNAQALNTLVKQLSSKFGAISSVLNDILSRLDPPEAEVQIDRLITGRLQS 1000
LQTYVTQQLIRAAEIRASANLAATKMSECVLGQSKRVDFCGKGYHLMSFP 1050
QSAPHGVVFLHVTYVPAQEKNFTTAPAICHDGKAHFPREGVFVSNGTHWF 1100
VTQRNFYEPQIITTDNTFVSGNCDVVIGIVNNTVYDPLQPELDSFKEELD 1150
KYFKNHTSPDVDLGDISGINASVVNIQKEIDRLNEVAKNLNESLIDLQEL 1200
GKYEQYIKWPWYIWLGFIAGLIAIVMVTIMLCCMTSCCSCLKGCCSCGSC 1250
CKFDEDDSEPVLKGVKLHYT*

### Examples

### Example 1

### Material and Methods

### Cells and viruses

BHK-21 cells (ATCC CCL10) were grown in BHK-21 Glasgow minimal essential medium (Gibco BRL) containing 5% FBS, 3 g/ml tryptose phosphate broth, 0.01 M HEPES, antibiotics, and glutamine. Virus infections were performed with 2% FBS for all cell types. BSC-1 cells (ATCC CCL-26) were grown in Eagle's Minimal Essential Medium (EMEM) supplemented with 0.1 mg/ml penicillin, 0.1 mg/ml streptomycin, 2 mM L-glutamine (Lonza) and 5% foetal bovine serum (FBS).

MVA-ΔF13L is an MVA virus that has replaced the F13L coding sequence with a red fluorescent protein (dsRed) gene (Sanchez-Puig, M and Blasco, R. Isolation of vaccinia MVA recombinants using the viral F13L gene as the selective markerBioTechniques 39:665-674 (November 2005) doi 10.2144/000112012).

### Design and generation of MVAs

Isolation of MVA recombinants was performed using a plaque selection procedure using the F13L gene as a selectable marker as described (Sanchez-Puig, J.M. and Blasco, R. (2005). Isolation of vaccinia MVA recombinants using the viral F13L gene as the selective marker (Biotechniques 39, 665-670; Sanchez-Puig, et al.. (2012) Isolation of recombinant MVA using F13L selection marker. Methods Mol Biol. 2012;890:93-111).

Complete SARS-CoV-2 S gene was synthesized and cloned into the transfer vector pMVA by Genscript. The pMVA transfer plasmid contains the F13L gene of MVA, recombination flanks and a vaccinia virus early/late promoter to direct the transcription of the foreign gene.

Plasmid pMVA-S was used to generate different S gene versions by site-directed mutagenesis. Mutations were introduced into the S gene sequence using the Q5 Site Directed Mutagenesis kit (New England Biolabs) following manufacturer's instructions.

Mutations K986P and V987P were introduced into pMVA-S by using oligonucleotides S4800-Q5-F: TAGATTAGATccacctGAAGCTGAAGTACAAATAGATAGATTAATAAC S4800-Q5-R: GATAATATATCATTTAATACAGAAGATATAG,
to generate plasmid pMVA-S-PP .

Mutations R682S and R685S were introduced into plasmid pMVA-S-PP by using oligonucleotides:
S3910-Q5-F: gcctctTCTGTAGCTAGCCAATCTATAATAG
S3910-Q5-R: tctagaTGGAGAATTAGTTTGAGTTTGATAAG,
generating plasmids pMVA-S-SS-PP.

Mutation D614G was introduced into plasmid pMVA-S and pMVA-S-SS-PP by using oligonucleotides
S3690-Q5-F TTATATCAAGgaGTAAATTGTACTGAAGTAC
S3690-Q5-R TACAGCTACTTGATTAGAAG
to generate pMVA-Sdg and pMVA-S-DG-SS-PP (also termed pMVA-Spf), respectively.

Plasmid pMVA-Spfx was obtained by GenScript by synthesis of an S version sequence optimized using a codon optimization algorithm and cloned into plasmid pMVA.

Recombinant viruses MVA-S, MVA-Sdg and MVA-Spf were generated by infecting BHK-21 cells with MVAΔF13L at a MOI of 0.05 and transfecting them with the corresponding transfer plasmids. Cell cultures were harvested at 48 hpi and MVAs were selected and isolated by plaque isolation procedure as described in Wyatt, L. S., Earl, P. L. & Moss, B. Generation of Recombinant Vaccinia Viruses. Curr Protoc Mol Biol 117, 16 17 11-16 17 18, https://doi.org/10.1002/cpmb.32 (2017).

Recombinant viruses MVA-S, MVA-Sdg and MVA-Spf were generated by infecting BHK-21 cells with MVAΔF13L at MOI of 0.05 and transfecting them with the different transfer plasmids. Cell cultures were harvested at 48-72 hpi and MVAs were selected and cloned by plaque isolation assay.

### Western blot

Cells were seeded in 6-well plates and the whole cell lysates (WCL) were prepared in denaturant buffer (80 mM Tris-HCI, pH 6.8, 2 % sodium dodecyl sulfate [SDS], 10 % glycerol, 0.01 % bromophenol blue solution and 0.71 M 2-mercaptoethanol). After SDS-polyacrylamide gel electrophoresis (PAGE), proteins were transferred to PVDF membranes and incubated 1 h at RT with primary antibody diluted in PBS containing 0.05 % Tween-20 and 1 % nonfat dry milk. Primary antibodies were Rabbit polyclonal anti-RBD antibody (GeneTex, ref GTX135709) diluted 1:500; Mouse monoclonal anti-S antibody (GeneTex, ref GTX632604) diluted 1:10000; Rat monoclonal anti-F13 (Schmelz M, et al. Assembly of vaccinia virus: the second wrapping cisterna is derived from the trans Golgi network. J Virol. 1994 Jan;68(1):130-47. doi: 10.1128/JVI.68.1.130-147.1994. PMID: 8254722; PMCID: PMC236272) diluted 1:100. After extensive washing with PBS-0.05 % Tween-20, membranes were incubated with HRP-conjugated secondary antibodies: polyclonal goat anti-rabbit IgG (Dako P0448) diluted 1:3000, polyclonal goat anti-mouse (GeneTex GTX213111) diluted 1:2000 and polyclonal rabbit anti-rat (Dako P0450) diluted 1:3000. After removal of unbound antibody, membranes were incubated for 1 min with a 1:1 mix of solution A (2.5 mM luminol [Sigma], 0.4 mM p-coumaric acid [Sigma], 100 mM Tris-HCI, pH 8.5) and solution B (0.018 % H2O2, 100 mM Tris-HCI, pH 8.5) to finally record the luminiscence using a Molecular Imager Chemi Doc-XRS (Bio-Rad).

### Immunofluorescence assay

Cells were seeded in coverslips, washed with PBS and fixed by ice-cold 4 % paraformaldehyde for 12 min. Those samples that were subsequently permeabilized were treated with PBS containing 0.1 % Triton X-100 for 15 min at room-temperature. Subsequently, all preparations were treated with PBS containing 0.1 M glycine for 5 min and incubated with primary antibodies in PBS-20 % FBS for 30 min, followed by incubation with secondary antibodies diluted 1:300 in PBS-20 % FBS. Antibodies used were: Rabbit polyclonal anti-RBD antibody (GeneTex, ref GTX135709) diluted 1:500; Rat monoclonal anti-B5 (Schmelz M, et al. Assembly of vaccinia virus: the second wrapping cisterna is derived from the trans Golgi network. J Virol. 1994 Jan;68(1):130-47. doi: 10.1128/JVI.68.1.130-147.1994. PMID: 8254722; PMCID: PMC236272.) diluted 1:100; anti-mouse IgG-Alexa Fluor 488, anti-Rat IgG-Alexa Fluor 594, (Invitrogen) diluted 1:300.

### Mouse Experiments

All mouse experiments were approved by the Ethical Review Committee at the INIA-CISA and Comunidad de Madrid (Permit number: PROEX 037/15), Spain, in strict accordance with EU guidelines 2010/63/UE about protection of animals used for experimentation and other scientific purposes, and Spanish Animal Welfare Act 32/2007, and the Institutional Animal Care and Use Committees (IACUC) of Institutional Biosafety Committee of Yale University (IBSCYU), US. All the animals were housed under specific pathogen-free conditions and allowed to acclimatize in the facilities provided to the biosafety level 3 (BSL3) animal facilities at the Animal Health Research Center (INIA-CISA) of Madrid and at the Yale animal facilities (BSL2 and 3), supported by Yale Animal Resources Center (YARC). All IVIS imaging, blood draw and virus inoculation experiments were done under anaesthesia using regulated flow of isoflurane:oxygen mix to minimize pain and discomfort to the animals. C57BL/6 (B6) wild type and C57BL/6 (B6), hACE2 transgenic B6 mice (heterozygous) were obtained from Jackson Laboratory. 8-10 week-old male and female mice were used for all the experiments. The heterozygous mice were crossed and genotyped to select heterozygous mice for experiments and confirmed by Automated Genotyping (TransnetYX).

### Immunization and SARS-CoV-2 infection

Groups of BALBc mice (n=5) were intraperitoneal, intramuscular or subcutaneously immunized at 0 and 21 days with 10⁷ PFU of MVA-Sdg, MVA-Spf or MVA-wt (non-immunized). Intraperitoneally immunized mice were bled one and two weeks post-prime, and two weeks post-boost, to determine IgM and IgG against SARS-CoV-2 S protein and the presence of neutralizing SARS-CoV-2 antibodies. Intramuscular and subcutaneously immunized mice were bled two weeks post-boost to determine neutralizing antibodies against SARS-CoV-2.

Groups of C57BL/6 mice (n=X) were intramuscularly immunized at 0 and 21 days with 10⁷ PFU of MVA-Sdg, MVA-Spf or MVA-wt (non-immunized). All animals were euthanized at day 15 post-boost, and their blood and spleens were harvested to analyze specific cellular and humoral immune responses.

Groups of 8 to 10 week old male and female hACE2 mice (n = 4 for histopathology and viral burden; n=5 for survival analysis) were intramuscularly immunized at 0 and 21 days with 10⁷ PFU of MVA-Sdg, MVA-Spf or MVA-wt (non-immunized). Two weeks after second immunization, all animals were bled to determine antibodies against SARS-CoV-2 S protein and were intranasally challenged with 1 × 10⁵ PFU in 30 µl volume of sterile PBS, under anaesthesia (0.5 - 5 % isoflurane delivered using precision Drager vaporizer with oxygen flow rate of 1 L/min). Body weight was measured and recorded daily. The starting body weight was set to 100 %. For survival experiments, mice were monitored every 24 h. Lethargic and moribund mice or mice that had lost more than 20% of their body weight were euthanized and considered to have succumbed to infection for Log rank survival plots.

### SARS-CoV-2 Spike ELISA (enzyme-linked immunosorbent assay)

Indirect enzyme-linked immunosorbent assays (ELISA) were used to determine specific anti S antibody levels in sera from immunized mice. 96w MaxiSorp plates (Nunc, USA) were coated with 150 ng/well of purified recombinant RDB protein expressed in expi293 cells, overnight at 4°C. Coated wells were subsequently blocked with blocking buffer (phosphate-buffered saline [PBS] containing 0.1% Tween20 and 3% dried milk) for 1 hour at room temperature (RT). Wells were then washed three times with washing buffer (PBST). Mice sera collected before challenge was diluted in PBST 1% dried milk and plates were incubated for 1h at RT. After three washes, wells were incubated with an anti-mouse IgG-HRP secondary antibody (cat. number P0447, Dako) at a 1:2000 dilution (1h, RT) and the reaction was developed with 50 µL of substrate solution TMB (Sigma) and stopped by adding the same volume of 3 N H2SO4. Results were expressed as ODs measured at 450 nm.

### Microneutralization assay

A microneutralization assay for SARS-CoV-2 serology was performed as previously described (Amanat et al., A serological assay to detect SARS-CoV-2 seroconversion in humans 2020 Nat Med 26, 1033-1036 (2020). https://doi.org/10.1038/s41591-020-0913-5). Experiments were conducted with 1200 PFU of the SARS-CoV-2 USA-WA1/2020 Nluc virus strain per well. One day prior to infection, 2×10⁴ Vero E6 cells were seeded per well of a 96 well flat bottom plate and incubated overnight at 37°C under 5% CO2 to permit cell adherence. Three fold dilutions of the heat inactivated sera (1h 56C) were performed in a separate 96 well culture plate using DMEM supplemented with penicillin (100 U/mL), streptomycin (100 mg/mL), HEPES, L-Glutamine (0.3 mg/mL), 0.12% sodium bicarbonate, 10% FBS (all from Thermo Fisher Scientific), in a Biosafety Level 3 laboratory. The 1200 PFU SARS-CoV-2 USA-WA1/2020 live virus was prepared in DMEM containing 10% FBS and combined with an equivalent volume of respective antibody dilutions for one hour at room temperature. After this incubation, all media was removed from the 96 well plate seeded with Vero E6 cells and virus:antibody mixtures were added to each respective well at a volume corresponding to 600 PFU per well and incubated for one hour further at 37°C. Both virus only and media only conditions were included in this assay. All virus:plasma supernatants were removed from wells without disrupting the Vero E6 monolayer. Each antibody concentration (100 µL) was added to its respective Vero E6-seeded well in addition to an equivalent volume of DMEM containing 10% FBS and was then incubated for 72 hours. Media was then discarded and replaced with 10% formaldehyde for 24 hours for fixation. Formaldehyde was removed from wells and 50 µL of crystal violet solution were added per well for visualization. PRNT50 titer was calculated as the reciprocal (log10) of the highest dilution of serum that neutralized 50% of the control virus input.

### Bioluminescence Imaging (BLI) of SARS-CoV-2 infection

All standard operating procedures and protocols for IVIS imaging of SARS-CoV-2 infected animals under ABSL-3 conditions were approved by IACUC, IBSCYU and YARC. All the imaging (which was not certified by peer review) is the author/funder. All rights reserved. No reuse allowed without permission. The copyright holder for this preprint was carried out using IVIS Spectrum^{®} (PerkinElmer) 985 in XIC-3 animal isolation chamber (PerkinElmer) that provided biological isolation of anesthetized mice or individual organs during the imaging procedure. All mice were anesthetized via isoflurane inhalation (3 - 5 % isoflurane, oxygen flow rate of 1.5 L/min) prior and during BLI using the XGI-8 Gas Anesthesia System. Prior to imaging, 100 µL of nanoluciferase substrate, furimazine (NanoGloTM, Promega, Madison, WI) diluted 1:40 in endotoxin-free PBS was retroorbitally administered to mice under anesthesia. The mice were then placed into XIC-3 animal isolation chamber (PerkinElmer) pre-saturated with isothesia and oxygen mix. The mice were imaged in both dorsal and ventral position at indicated day post infection. The animals were then imaged again after euthanasia and necropsy by spreading additional 200 µL of substrate on to exposed intact organs. Infected areas of interest identified by carrying out whole-body imaging after necropsy were isolated, washed in PBS to remove residual blood and placed onto a clear plastic plate. Additional droplets of furimazine in PBS (1:40) were added to organs and soaked in substrate for 1-2 min before BLI. Images were acquired and analyzed with the manufacturer's Living Image v4.7.3 in vivo software package. Image acquisition exposures were set to auto, with imaging parameter preferences set in order of exposure time, binning, and f/stop, respectively. Images were acquired with luminescent f/stop of 2, photographic f/stop of 8. Binning was set to medium. Comparative images were compiled and batch-processed using the image browser with collective luminescent scales. Photon flux was measured as luminescent radiance (p/sec/cm2/sr). During luminescent threshold selection for image display, luminescent signals were regarded as background when minimum threshold levels resulted in displayed radiance above non-tissue-containing or known uninfected regions. To determine the pattern of virus spread, the image sequences were acquired during the indicated days following administration of SARS-CoV-2 (i.n).

### Measurement of viral burden and analysis of signature inflammatory cytokines mRNA

Organs analyzed (lung and brain, from infected or uninfected mice) were collected, weighted, and homogenized in 0.8 mL of complete RPMI media containing penicillin-streptomycin and homogenized in 2 mL tube containing 1.5 mm Zirconium beads with BeadBug 6 homogenizer (Benchmark Scientific, TEquipment Inc). Virus titers were measured by quantitative real-time PCR (qRT-PCR) and plaque assay. RNA transcripts for inflammatory cytokines were also measured by qRT-PCR. First, total RNA was extracted from 100 µL of the homogenized tissues using TRIzol reagent (Invitrogen) and purified using NucleoSpin RNA columns (Macherey-Nagel), reverse transcribed with iScript advanced cDNA kit (Bio-Rad Cat#1725036) and qRT-PCR analysis was performed using SYBR Green Real-time PCR assay for determining copies of SARS-CoV-2 N gene RNA, following manufacturer conditions. Second, serially diluted clarified tissue homogenates were used to infect Vero-E6 cell culture monolayer. The titers per gram of tissue were quantified using standard plaque forming assay. Briefly, the 4×105 Vero-E6 cells were seeded on 12-well plate. 24 h later, the cells were infected with 100 µL of the serially diluted clarified tissue homogenates. After 1 hour, the cells were overlayed with 1ml of pre-warmed 0.6% Avicel (RC-5811059 FMC BioPolymer) made in complete DMEM medium. Plaques were resolved at 72 h post infection by fixing in 10 % formaldehyde o/n at RT followed by staining for 20 min with 1% crystal violet solution (Sigma-Aldrich). Plates were rinsed in water to visualize plaques.

### Results

### General codon optimization methods versus the codon optimization method of the present invention

We have tested different codon optimization strategies by evaluating the effect of different gene versions on the expression of a model protein (the GFP variant called GreenLantern, see Campbell BC, et al. A bright monomeric fluorescent protein with rapid expression and cell filling properties for neuronal imaging. Proc Natl Acad Sci U S A. 2020 Dec 1;117(48):30710-30721. doi: 10.1073/pnas.2000942117). We prepared GFP variants that were codon optimized following the codon optimization method of the present invention or deoptimized with respect to the vaccinia WR or the human codon usage table 1.

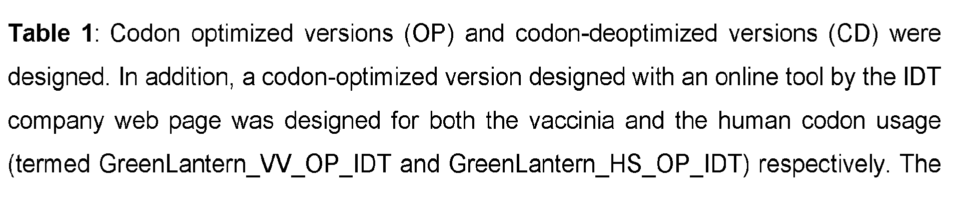

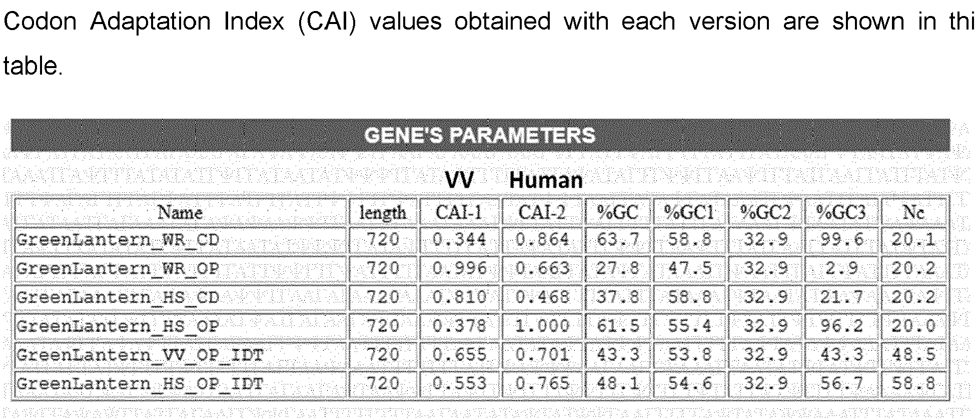

In this context, codon optimization means choosing the most frequently used synonymous codon for each amino acid, and codon deoptimization means choosing the least frequently used synonymous codon for each amino acid. The only exception allowed to this rule is that early transcription termination signals within the gene were removed to prevent premature mRNA termination. Also, GFP gene versions were defined by using an online tool in the IDT company web page (https://eu.idtdna.com/pages/tools/codon-optimization-tool), where optimized versions for the Vaccinia or human codon usage were defined. The values for codon adaptation index for each of the gene versions are shown in Fig 1. Codon optimized versions of the GFP model gene were introduced into the Vaccinia genome as a bi-cistronic construct in which a second gene (TagRFP, coding for a red fluorescent protein) serves as an internal control. Expression of the gene indicated that codon optimization to the human codon usage results in an increased early gene expression. Conversely, late expression is higher in the codon optimized version to the vaccinia codon usage according to the codon optimization method of the present invention. The codon deoptimization to the human codon usage has a similar positive influence, consistent with a high codon adaptation index to Vaccinia virus.

### Evaluation of codon usage influence on protein expression.

Different versions of the model gene GFP were inserted in the VACV genome and evaluated in protein expression. Figure 1 shows the Codon Adaptation Index of the different constructs with respect to Vaccinia virus and the human codon usage tables. Codon usage tables for Vaccinia Virus WR are included below in Table 2. Those GFP versions were placed in a bi-cistronic construct where a red fluorescent protein (TagRFP) was included downstream of an Internal Ribosomal Entry Site (IRES). Red fluorescence served as an internal control for the accurate determination of translation efficiency of the GFP open reading frame.

Protein expression was measured as fluorescence in infected cultures, using conditions either for early gene expression (24 hours post-infection in the presence of cytosine arabinoside) or for late gene expression (24 hours post-infection in the absence of drugs). Figure 2 shows the GFP fluorescence levels relative to the RFP fluorescence in both early and late conditions. Results show that higher levels of expression in the early conditions were obtained with gene versions optimized for the human codon usage than with the VACV codon optimized version (WR OP). Conversely, codon optimization for VACV according to the method of the present invention (or de-optimization to human codon usage) produced a higher level of expression in the late conditions.

**Table 2. Codon Usage tables used (From the Codon usage database https://www.kazusa.or.jp/codon/)**

| |
|---|
| Vaccinia WR codon usage table |
| Vaccinia virus WR [gbvrl]: 218 CDS's (57854 codons) fields: [triplet] [frequency: per thousand] ([number]) |
| UUU 33.0( 1912) UCU 23.8( 1375) UAU 36.5( 2114) UGU 15.8( 916) UUC 13.2( 762) UCC 9.4( 545) UAC 15.1( 873) UGC 4.9( 283) UUA 25.8( 1494) UCA 14.8( 857) UAA 2.3( 135) UGA 0.9( 50) UUG 18.5( 1068) UCG 8.3( 479) UAG 0.6( 33) UGG 7.3( 422) CUU 11.5( 668) CCU 10.9( 628) CAU 14.7( 852) CGU 6.4( 371) CUC 6.0( 349) CCC 4.3( 246) CAC 6.0( 350) CGC 2.5( 142) CUA 18.1( 1045) CCA 12.3( 714) CAA 16.0( 924) CGA 5.9( 343) CUG 6.8( 395) CCG 6.2( 360) CAG 6.3( 363) CGG 1.6( 92) AUU 37.9( 2193) ACU 22.3( 1292) AAU 48.0( 2779) AGU 14.6( 846) AUC 18.5( 1073) ACC 8.5( 489) AAC 20.4( 1180) AGC 5.8( 333) AUA 38.2( 2208) ACA 21.5( 1244) AAA 50.8( 2941) AGA 22.8( 1318) AUG 28.1( 1628) ACG 8.7( 506) AAG 22.9( 1324) AGG 4.0( 233) GUU 20.6( 1191) GCU 13.1( 756) GAU 51.1( 2955) GGU 13.0( 750) GUC 8.1( 471) GCC 6.6( 384) GAC 16.2( 939) GGC 3.6( 206) GUA 23.1( 1335) GCA 11.4( 657) GAA 39.7( 2299) GGA 20.8( 1202) GUG 9.4( 545) GCG 7.1( 413) GAG 14.5( 840) GGG 2.8( 164) |
| Coding GC 34.03% 1st letter GC 39.67% 2nd letter GC 32.18% 3rd letter GC 30.23% |
| Homo sapiens codon usage table |
| Homo sapiens [gbpri]: 93487 CDS's (40662582 codons) fields: [triplet] [frequency: per thousand] ([number]) |
| UUU 17.6(714298)  UCU 15.2(618711)  UAU 12.2(495699)  UGU 10.6(430311) UUC 20.3(824692)  UCC 17.7(718892)  UAC 15.3(622407)  UGC 12.6(513028) UUA  7.7(311881)  UCA 12.2(496448)  UAA  1.0( 40285)  UGA  1.6( 63237) UUG 12.9(525688)  UCG  4.4(179419)  UAG  0.8( 32109)  UGG 13.2(535595) CUU 13.2(536515)  CCU 17.5(713233)  CAU 10.9(441711)  CGU  4.5(184609) CUC 19.6(796638)  CCC 19.8(804620)  CAC 15.1(613713)  CGC 10.4(423516) CUA  7.2(290751)  CCA 16.9(688038)  CAA 12.3(501911)  CGA  6.2(250760) CUG 39.6(1611801)  CCG  6.9(281570)  CAG 34.2(1391973)  CGG 11.4(464485) AUU 16.0(650473)  ACU 13.1(533609)  AAU 17.0(689701)  AGU 12.1(493429) AUC 20.8(846466)  ACC 18.9(768147)  AAC 19.1(776603)  AGC 19.5(791383) AUA  7.5(304565)  ACA 15.1(614523)  AAA 24.4(993621)  AGA 12.2(494682) AUG 22.0(896005)  ACG  6.1(246105)  AAG 31.9(1295568)  AGG 12.0(486463) GUU 11.0(448607)  GCU 18.4(750096)  GAU 21.8(885429)  GGU 10.8(437126) GUC 14.5(588138)  GCC 27.7(1127679)  GAC 25.1(1020595)  GGC 22.2(903565) GUA  7.1(287712)  GCA 15.8(643471)  GAA 29.0(1177632)  GGA 16.5(669873) GUG 28.1(1143534)  GCG  7.4(299495)  GAG 39.6(1609975)  GGG 16.5(669768) |
| Coding GC 52.27% 1st letter GC 55.72% 2nd letter GC 42.54% 3rd letter GC 58.55% |

### Generation of vaccine candidates MVA-Sdg and MVA-Spf.

Isolation of recombinant MVA vaccinia viruses was carried out by inserting genes into the viral genome by homologous recombination occurring between the viral genome and transfected plasmid DNA. Insertion occurs at an intergenic site between the F13L and F12L genes and does not lead to inactivation of any virus gene. Thus, the insertion preserves the genetic makeup of the original vector. The inserted sequence includes the combined synthetic early/late Poxviral promoter and open reading frames encoding different versions of the protein S gene. The sequences were synthesized, cloned into plasmids and verified by sequencing. Subsequently, the plasmids were transfected into cells infected with the MVA virus to allow recombination, and finally the virus recombinants were obtained by plaque isolation and amplified in cell cultures.

### SARS-CoV-2 S gene inserted:

- **S: SARS-CoV-2 S gene.** Sequence corresponds to S protein obtained from reference virus isolate (SARS-CoV-2 Wuhan-Hu-1 NC_045512.2). Gene sequence was optimized for Vaccinia codon usage using the codon optimization described in the present invention.
- **Sdg: SARS-CoV-2 S gene with D614G mutation.** This mutation appeared early in circulating SARS-CoV-2 and became predominant in the circulating virus since then. The nucleotide sequence of the S protein in this virus was codon optimized for Vaccinia virus using the codon optimization method of the present invention.
- **Spf and Spfx (pre-fusion):** mutated form of Coronavirus SARS-CoV-2 S in which some mutations that prevent the conformational change in the protein were introduced. The mutations consist in two amino acid substitutions in the cleavage site (R682S and R685S mutations - Wrobel et al 2020 SARS-CoV-2 and bat RaTG13 spike glycoprotein structures inform on virus evolution and furin-cleavage effects. Nat Struct Mol Biol. 2020 Jul 9. doi: 10.1038/s41594-020-0468-7) and two amino acid substitutions that prevent the protein conformational change that precedes membrane fusion (K986P V987P mutations, defined by Pallesen et al (2017) Immunogenicity and structures of a rationally designed prefusion MERS-CoV spike antigen Proc Natl Acad Sci USA 2017;114(35):E7348-E7357. doi: 10.1073/pnas.1707304114). These changes result in a form of the S protein which is stabilized in the pre-fusion conformation and are inactive in inducing membrane fusion. Two vectors, MVA-Spf and MVA-Spfx, which vary in codon optimization method were generated: while Spf nucleotide sequence is codon optimized for VACV virus using the codon optimization method of the present invention, Spfx sequence was codon optimized for VACV using IDT webpage tool.
- **Spf-** δ **(delta).** Sequence was derived from variant sequence hCoV-19/Spain/GA-VITHAS-51592630/2021. Due to the differences in the protein with respect to the Wuhan Spike protein of reference, mutations to stabilize this protein are numbered as R680S, R683S, K984P and V985P, but they are equivalent to R682S, R685S, K986P and V987P mutations when taking the Spike protein of the SARS-CoV-2 Wuhan-Hu-1 (GenBank accession No QHD43416.1 or Uniprot ID: P0DTC2) as reference. The sequence was codon optimized for VACV virus following the codon optimization method of the present invention. For more detail of the mutations included in the S protein of this virus, please see Example 2.
- **Spf-o (omicron).** Sequence was derived from variant sequence hCoV-19/Spain/GA-CHUVI-21822434/2022. Due to the differences in the protein with respect to the Wuhan Spike protein of reference, mutations to stabilize this protein were R679S, R682S, K983P and V984P, but they are equivalent to R682S, R685S, K986P and V987P mutations when taking the Spike protein of the SARS-CoV-2 Wuhan-Hu-1 (GenBank accession No QHD43416.1 or Uniprot ID: P0DTC2) as reference. The sequence was codon optimized for VACV virus following the codon optimization method of the present invention. For more detail of the mutations included in the S protein of this virus, please see Example 3.

Figure 20 shows a multiple alignment of the S protein of reference (Spike protein of the SARS-CoV-2 Wuhan-Hu-1m GenBank accession No QHD43416.1 or Uniprot ID: P0DTC2) with the S proteins included in the viruses MVA-Spf, MVA-Spf- δ, MVA-Spf-omicron and MVA-Sdg. It can be observed that the mutations R682S, R685S, K986P and V987P are in equivalent positions in the S protein from the viruses MVA-Spf, MVA-Spf-delta, MVA-Spf-omicron.

### Expression of the Spike from MVA recombinants.

Once the recombinant viruses were isolated, we verified the expression of the proteins in cells infected with MVA recombinants by Western Blot and immunofluorescence. These tests have been carried out on BHK cell cultures, in which the MVA strain replicates efficiently and produces high virus titers.

Cleavable S versions (S and Sdg) produced several forms that are recognized by anti-RBD and anti-S2 antibodies (Fig 4). The larger forms (S) are compatible with the presence of the complete protein and different glycosylation states. A second, somewhat smaller form was detected which was compatible with the size of the products generated after cleavage in the region of the fusion peptide (NTD-RBD and HH-T-C following the nomenclature of the scheme in Fig 3). In the case of the pre-fusion stabilized forms (Sfp and Spfx), this second band was not observed, indicating that the mutations introduced effectively prevent the proteolytic cleavage between the S1 and S2 domains.

### Subcellular localization.

SARS-CoV-2 proteins expressed by the recombinant MVA viruses were localized in infected cells by indirect immunofluorescence (Fig 5). Using non-permeabilizing conditions, cell surface expression of all full versions of protein S (S, Sdg and Spf) was detected. As a control for infection and permeabilization, we used antibody to Vaccinia virus B5 protein, which stains viral particles adhered to the surface of the cells. Immunofluorescence assays carried out under permeabilizing conditions demonstrated, in addition to the surface mark mentioned above, an intracellular pattern compatible with endoplasmic reticulum (reticular mark throughout cytoplasm) and Golgi apparatus (juxtanuclear staining).

### Membrane fusion activity.

Mature protein S mediates virus entry by promoting fusion between the virus membrane and the cell membrane. Thanks to this functionality, it is possible to detect the integrity of the protein expressed by recombinant MVAs by means of a cell fusion assay. In order to analyze whether the different versions of the S protein were expressed correctly, BSC-1 cells (African green monkey kidney cell line) were infected at high multiplicity with the recombinant MVAs for 18h and observed under the microscope. Although BSC-1 cells are not totally permissive for MVA, infection blockage occurs at the end of the infective cycle, and therefore recombinants are expected to express all their proteins even though they are unable to produce infective progeny virus. Correct expression of S was confirmed by the observation that MVA-Sdg virus induced a massive fusion of the cells in the monolayer (Fig 6). Cell-cell fusion was S-dependent, since could not be detected in cells infected with the parental MVA. Significantly, the expression of the pre-fusion version by the recombinant MVA-Spf did not induce cell fusion, confirming that the introduced mutations destroy the protein-mediated membrane fusion activity, as expected.

### Immunogenicity in Balb/C mice.

To study the induction of immunity by the recombinants we carried out a first experiment in BALB/c mice. Groups of 5 animals were inoculated with the vaccine candidates by the intraperitoneal route (I.P.). Two doses were administered 21 days apart with 10⁷ PFU of purified virus in 200 µl per mouse (Fig 7).

The appearance of the antibody response was followed by ELISA (Fig 8). One week after the first immunization, the serum IgM levels of the animals were analyzed. At this early time, significant levels of IgM specific to the RBD domain of protein S were detected in the serum of mice immunized with the MVA-Spf construct, but were not significant in animals inoculated with MVA-Sdg. Two weeks after the first immunization, significant levels of RBD domain-specific IgG were detected in mice immunized with either the MVA-Sdg or MVA-Spf construct. Antibody levels were higher with the latter, indicating a better induction of immunity with the pre-fusion stabilized version.

After a second immunization with the different recombinant MVAs, IgGs specific to the RBD domain were detected in the sera from animals immunized with recombinant MVA expressing both versions (MVA-Spf and MVA-Sdg). To measure the magnitude of the response, IgG levels in the sera were analyzed by RBD domain-specific ELISA. Serial dilutions of these sera indicated that MVA-Spf immunized mice had a higher level of RBD-specific IgG than MVA-Sdg immunized animals (Fig 8).

Also, the level of neutralizing antibodies was determined by assays using SARS-CoV-2 infective virus, or vesicular stomatitis virus (VSV) particles pseudotyped with S glycoprotein. With both methods, the measured neutralization titers were significantly higher in mice inoculated with MVA-Spf than in mice inoculated with the unmodified S version, MVA-Sdg (Fig. 9).

In the preceding experiment, inoculation of the vaccine candidates was carried out by the intraperitoneal route. However, we tested if the intramuscular (IM) or subcutaneous (SC) routes were also efficient in immunization, since those are the preferred options for MVA based vaccines. Therefore, we compared these two inoculation routes by assessing the ability of the MVA-Spf vaccine candidate to elicit a good immune response. Both anti-RBD responses and neutralization titers indicated a good response in both cases (Fig 10). IM inoculation produced a slightly better response and was chosen as the preferred way of administration.

### Protection assay In K18-Hace2 Transgenic Mouse

Protection from SARS-CoV-2 induced disease was demonstrated in a transgenic mouse model. K18-hACE2 transgenic mice express human ACE2, the receptor used by severe acute respiratory syndrome coronavirus (https://www.jax.org/strain/034860). The protocol included two inoculations of the vaccine candidate 21 days apart and a later challenge by intranasal route with SARS-CoV-2 (Fig 11). Body weight loss and clinical signs indicated protective activity of MVA-Sdg and Spf recombinants (Fig 12). Also, the replication of the virus in the animals could be prevented, as revealed by the amount of viral RNA and the virus titers in lungs and brain (Fig 13), Notably, the efficacy of the MVA-Spf vaccine candidate was higher than that of MVA-Sdg, since virus RNA was significantly lower in animals vaccinated with the former. Viral RNA was undetectable in the brain tissue of those animals.

In an independent experiment, we studied the induction of protection in vaccinated mice. We used the same experimental design as before, using a SARS-CoV-2 strain expressing nanoluciferase. This virus allows the monitoring of virus dissemination and also the progression of disease (Ullah I, et al 2021 Live imaging of SARS-CoV-2 infection in mice reveals neutralizing antibodies require Fc function for optimal efficacy. bioRxiv [Preprint]. 2021 Mar 22:2021.03.22.436337. doi: 10.1101/2021.03.22.436337). Vacciniation of K18-hACE2 transgenic mice with MVA-Spf resulted in complete protection from a lethal challenge (n=5) (Fig 14). However, survival was only partial (3 out of 5 mice) in animals vaccinated with MVA-Sdg. These results correlated well with virus spread in the animal (Fig 15).

### Hamster Protection Model

A second model of protection was the Syrian Hamster, which has been well characterized for SARS-CoV-2 infection (Sia et al. Pathogenesis and transmission of SARS-CoV-2 in golden hamsters. Nature 583, 834-838 (2020). https://doi.org/10.1038/s41586-020-2342-5; Imai et al. Syrian hamsters as a small animal model for SARS-CoV-2 infection and countermeasure development. Proceedings of the National Academy of Sciences Jul 2020, 117 (28) 16587-16595; DOI: 10.1073/pnas.2009799117). Animals inoculated twice with the MVA recombinants MVA-Sdg and MVA-Spf developed neutralizing antibodies to SARS-CoV-2. Virus neutralization titers were significantly higher in the animals vaccinated with MVA-Spf than in the animals vaccinated with MVA-Sdg (Fig 16).

Protection from disease was studied by pathological study of the lungs. After euthanizing the animals in two groups (days 2 and 4 after challenge), lung pathology and viral titers were measured. Fig 17 shows the results obtained, which were indicative of a good degree of protection in vaccinated animals, which was almost complete in the case of animals vaccinated with the MVA-Spf vaccine candidate. Finally, SARS-CoV-2 viral titers were obtained in lungs and nasal turbinates from the two groups of hamsters (day 2 and 4 post-challenge) (Fig 18). In nasal turbinates, animals showed a relative reduction of viral titers after vaccination with MVA-Sdg at day 4. Animals vaccinated with MVA-Spf had decreased viral titers already at day 2, and undetectable levels of virus by day 4, indicating an effective degree of protection. Similar results were obtained in the lungs, where levels of virus were undetectable by day 4 in animals vaccinated with MVA-Spf. These results correlated with lung pathology and clearly demonstrated the ability of this vaccine candidate to elicit efficient protection.

### Example 2 Vaccine candidate MVA-Spf to the Delta variant sequence of SARS-COV-2

We have recently generated an MVA-Spf vaccine candidate with the Delta (δ) version of the SARS-CoV-2 Spike glycoprotein. The sequence inserted is derived from virus hCoV-19/Spain/GA-VITHAS-5 1592630/2021 (variant sequence B.1.617+).

### Data from GISAID database:

| Virus detail | |
|---|---|
| **Virus name:** | hCoV-19/Spain/GA-VITHAS-51592630/2021 |
| **Accession ID:** | EPI_ISL_1824684 |
| **Type:** | betacoronavirus |
| **Clade** | G |
| **Pango Lineage** | B.1.617.2 (version: 2021-04-23) |
| **AA Substitutions** | Spike D614G. Spike D950N, Spike E156G, Spike F157del, Spike L452R, Spike P681R, Spike R158del, Spike T19R, Spike T95I, Spike T478K, M I82T, N D63G, N 0377Y, N G215C N R203M, N T296I, NS3 S26L, NS7a T120I, NS7a V82A, NS7b T40I, NSP3 A488S, NSP3 A1033D, NSP3 P1228L, NSP3 P1469S, NSP4 T4921, NSP4 V167L, NSP6 T77A, NSP12 G671S, NSP12 P323L, NSP13 P77L, NSP14 A394V |
| **Variant** | G/452R.V3 (B,1,617+) |
| **Passage details/history:** | Original |

| **Sample information** | |
|---|---|
| **Collection date:** | 2021-04-29 |
| **Location:** | Europe / Spain / Galicia / Vigo |
| **Host:** | Human |
| **Additional location information:** | |
| **Gender:** | Male |
| **Patient age:** | 24 |
| **Patient status:** | Live |
| **Specimen** source: | Nasopharyngeal swab |
| **Additional host information:** | |
| **Sampling strategy:** | |
| **Outbreak:** | |
| **Last vaccinated:** | |
| **Treatment:** | |
| **Sequencing technology:** | Illumina iSeq |
| **Assembly method:** | |
| **Coverage:** | |
| **Comment:** | ⓘ Gap of 13 nucleotides when compared to the reference sequence. |

We have isolated this MVA-Spf-δ by its interest given the growing percentage of cases derived from this variant, and also as a test for the speed at which we are able to isolate a new vaccine variant.

Total time to isolation of the MVA recombinant was only 17 days from the time we received the plasmid DNA in the laboratory to the amplification of a small virus stock. This is much faster than other options (like insertion in the TK locus or in the Deletion III insertion point) because our selection system (Sanchez-Puig JM, Blasco R. Isolation of vaccinia MVA recombinants using the viral F13L gene as the selective marker. Biotechniques. 2005 Nov;39(5):665-6, 668, 670 passim. doi: 10.2144/000112012. PMID: 16312215; and Sanchez-Puig JM, Lorenzo MM, Blasco R. Isolation of recombinant MVA using F13L selection. Methods Mol Biol. 2012;890:93-111. doi: 10.1007/978-1-61779-876-4_5. PMID: 22688762.) allows for fast selection and isolation of new MVA recombinants.

We have isolated recombinants expressing both the wild-type sequence (MVA-S-δ) and the pre-fusion stabilized version (MVA-Spf-δ). As a first test for these constructs, we have tested their ability to cause cell-cell fusion in BHK-ACE2 cells (Figure 19). These new constructs behaved similarly to the MVA-Spf virus, causing extensive cell-cell fusion by the unmodified S-δ version and no fusion when the four amino acid substitutions leading to the pre-fusion stabilized Spf-δ were introduced.

### Example 3 Vaccine candidate MVA-Spf to the Omicron variant sequence of SARS-COV-2

We have recently generated an MVA-Spf vaccine candidate with the Omicron version of the SARS-CoV-2 Spike glycoprotein. The sequence inserted is derived from virus hCoV-19/Spain/GA-CHUVI-21822434/2022

| Virus detail | |
|---|---|
| **Virus name:** | hCoV-19/Spain/GA-CHUVI-21822434/2022 |
| **Accession ID:** | EPI_ISL_12057271 |
| **Type:** | betacoronavirus |
| **Clade:** | GRA |
| **Pango Lineage:** | BA.2 (Pango v.4.0.5 PLEARN-v1.3), Omicron (BA.2-like) (Scorpio) |
| **AA Substitutions:** | Spike A27S, Spike D405N, Spike D614G, Spike D796Y, Spike E484A, Spike G142D, Spike G339D, Spike H655Y, Spike K417N, Spike L24del Spike N440K, Spike M501Y, Spike N679K, Spike N764K, Spike N969K, Spike P25del, Spike P26del, Spike P681H, Spike Q493R, Spike Q498R, Spike Q954H, Spike R408S, Spike S371F, Spike S373P, Spike S375F, Spike S477N, Spike T19I, Spike T376A, Spike T478K, Spike V213G, Spike Y505H, E T9I, M A63T, M Q19E, N E31del, N G204R, N P13L, N R32del, N R203K, N S33del, N S413R, NS3 T223I, NSP1 S135R, NSP3 D164G, NSP3 G489S, NSP3 T241, NSP4 L264F, NSP4 T3271, NSP4 T492I, NSP5 P132H, NSP6 F108del, NSP6 G107del, NSP6 S106del, NSP12 P323L, NSP13 R392C, NSP14 142V, NSP15 T112I |
| **Variant:** | VOC Omicron GRA (B.1.1.529+BA.*) first detected in Botswana/Hong Kong/South Africa |
| **Passage details/history:** | Original |

| Sample information | |
|---|---|
| **Collection date:** | 2022-03-04 |
| **Location:** | Europe / Spain / Galicia / Vigo |
| **Host:** | Human |
| **Additional location information:** | |
| **Gender:** | Female |
| **Patient age:** | 19 |
| **Patient status:** | Live |
| **Specimen source:** | Nasopharyngeal swab |
| **Additional host information:** | |
| **Sampling strategy:** | |
| **Outbreak:** | |
| **Last vaccinated:** | |
| **Treatment:** | |
| **Sequencing technology:** | Illumina Iseq |
| **Assembly method:** | |
| **Coverage:** | |

## Claims

1. A modified vaccinia virus Ankara (MVA) vector comprising at least one nucleic acid encoding the Spike (S) protein of at least one SARS-CoV-2 variant or linage, wherein said nucleic acid is **characterized by** being inserted in between the open reading frames of the F12L and F13L genes of the MVA genome without substituting, replacing, or interrupting said genes, and wherein the nucleic acid is further characterized for being codon optimized for Vaccinia virus, wherein the method for codon optimization for Vaccinia virus comprises the steps of:
i) choosing for every amino acid of the S protein of at least one SARS-CoV-2 variant or linage the synonymous codon with the highest frequency value according to the table below: 
UUU 33.0( 1912) UCU 23.8( 1375) UAU 36.5( 2114) UGU 15.8( 916)
UUC 13.2( 762) UCC 9.4( 545) UAC 15.1( 873) UGC 4.9( 283)
UUA 25.8( 1494) UCA 14.8( 857) UAA 2.3( 135) UGA 0.9( 50)
UUG 18.5( 1068) UCG 8.3( 479) UAG 0.6( 33) UGG 7.3( 422)

CUU 11.5( 668) CCU 10.9( 628) CAU 14.7( 852) CGU 6.4( 371)
CUC 6.0( 349) CCC 4.3( 246) CAC 6.0( 350) CGC 2.5( 142)
CUA 18.1( 1045) CCA 12.3( 714) CAA 16.0( 924) CGA 5.9( 343)
CUG 6.8( 395) CCG 6.2( 360) CAG 6.3( 363) CGG 1.6( 92)

AUU 37.9( 2193) ACU 22.3( 1292) AAU 48.0( 2779) AGU 14.6( 846)
AUC 18.5( 1073) ACC 8.5( 489) AAC 20.4( 1180) AGC 5.8( 333)
AUA 38.2( 2208) ACA 21.5( 1244) AAA 50.8( 2941) AGA 22.8( 1318)
AUG 28.1( 1628) ACG 8.7( 506) AAG 22.9( 1324) AGG 4.0( 233)

GUU 20.6( 1191) GCU 13.1( 756) GAU 51.1( 2955) GGU 13.0( 750)
GUC 8.1( 471) GCC 6.6( 384) GAC 16.2( 939) GGC 3.6( 206)
GUA 23.1( 1335) GCA 11.4( 657) GAA 39.7( 2299) GGA 20.8( 1202)
GUG 9.4( 545) GCG 7.1( 413) GAG 14.5( 840) GGG 2.8( 164)
 and
ii) reviewing the resulting nucleotide sequence of step i) so that in case the sequence TTTTTNT is present, such TTTTTNT sequence is substituted for a different nucleotide sequence with the proviso that the VACV RNA polymerase does not recognize said different sequence as an early transcription termination signal and that the amino acid sequence of the translated S protein is not modified by the optimization process.

2. The modified vaccinia virus Ankara (MVA) vector according to claim 1, wherein the nucleic acid encoding for the S protein of at least one SARS-CoV-2 variant or linage is further **characterized by** being operably linked to the synthetic early/late Vaccinia virus promoter.

3. The modified vaccinia virus Ankara (MVA) vector according to claims 1 or 2, wherein Spike (S) protein comprises at least the amino acid substitutions R682S, R685S, K986P and V987P, wherein the amino acid numbering is with respect to the Spike protein of the SARS-CoV-2 Wuhan-Hu-1 variant or linage of GenBank accession No QHD43416.1 or Uniprot ID: P0DTC2.

4. The modified vaccinia virus Ankara (MVA) vector according to any one of claims 1 to 3, wherein the variant or linage of SARS-CoV-2 is selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617.1 (Kappa variant) or Linage B.1.1.7 (United Kingdom variant), Linage B.1.617.2 (Delta variant), Linage B.1.1.529 (Omicron variant), or any combination thereof.

5. The modified vaccinia virus Ankara (MVA) vector according to any of claims 1 to 4, wherein the nucleic acid encoding the Spike (S) protein consists of SEQ ID NO: 7.

6. The modified vaccinia virus Ankara (MVA) vector according to any of claims 1 to 4, wherein the nucleic acid encoding the Spike (S) protein consists of SEQ ID NO: 8.

7. The modified vaccinia virus Ankara (MVA) vector according to any of claims 1 to 4, wherein the nucleic acid encoding the Spike (S) protein consists of SEQ ID NO: 27.

8. A **vaccine combination** comprising:
a. a composition comprising an immunologically effective amount of a modified vaccinia virus Ankara (MVA) vector as defined in any claims 1 to 7; and
b. a composition comprising an immunologically effective amount of a modified vaccinia virus Ankara (MVA) vector as defined in any of claims 1 to 7;
wherein one of the compositions is a priming composition and the other composition is a boosting composition.

9. A **vaccine combination** comprising:
a. a composition comprising an immunologically effective amount of a modified vaccinia virus Ankara (MVA) vector as defined in any of claims 1 to 7 and
b. a composition comprising an immunologically effective amount of an immunogen of at least one SARS-CoV-2 variant;
wherein one of the compositions is a priming composition and the other composition is a boosting composition.

10. The vaccine combination according to claim 9, wherein the immunogen derived from at least one SARS-CoV-2 variant or linage is selected from the group consisting of viral vectors, recombinant proteins o peptides, nucleic acids, virus-like particles, nanoparticles, or any combination thereof.

11. The modified vaccinia virus Ankara (MVA) vector according to claims 1 to 7, **for use** in generating an immunogenic and/or protective immune response in a subject in need thereof, preferably in a human subject, wherein said immune response is against at least the Spike (S) protein, or a fragment of said S protein, of at least one SARS-CoV-2 variant, wherein said MVA is used for priming and/or for boosting said immune response, and wherein the MVA is capable of producing said S protein, in the subject to be treated.

12. The vaccine combination according to claims 8 to 10, **for use** in generating an immunogenic and/or protective immune response in a subject in need thereof, preferably in a human subject, wherein said immune response is against at least the Spike (S) protein, or a fragment of said S protein, of at least one SARS-CoV-2 variant, and wherein said combination is capable of producing said S protein, in the subject to be treated.

13. The modified vaccinia virus Ankara (MVA) vector for use according to claim 11, or the vaccine combination for use according to claim 12, wherein the MVA vector/s is/are administered once, twice, three times or four times.

14. The modified vaccinia virus Ankara (MVA) vector for use according to claim 11, or the vaccine combination for use according to claim 12, wherein said modified vaccinia virus Ankara (MVA) vector or vaccine combination is administered via the intramuscular route.

15. A method for codon optimizing a nucleotide sequence for Vaccinia virus (VACV), the method comprising the steps of:
i) choosing for every amino acid of the protein whose nucleotide sequence is to be codon optimized, the most used synonymous codon according to the codon usage table from Vaccinia virus, and
ii) reviewing the resulting nucleotide sequence of step i) so that in case the sequence TTTTTNT is present, such TTTTTNT sequence is substituted for a different nucleotide sequence with the proviso that the VACV RNA polymerase does not recognize said different sequence as an early transcription termination signal and that the amino acid sequence of the translated S protein is not modified by the optimization process.
